# FASCICULE DE BREVET EUROPEEN

(11) **EP 1 066 246 B1**
(45) Date de publication et mention de la délivrance du brevet: **05.11.2003**
(21) Numéro de dépôt: 00900641.2
(22) Date de dépôt: 21.01.2000
(51) Int. Cl.: C07C 233/90, C07D 213/20, C07D 295/14, A61K 7/13

(54) **NOUVEAUX COUPLEURS CATIONIQUES ET LEUR UTILISATION POUR LA TEINTURE D'OXYDATION**
KATIONISCHE KUPPLER DEREN VERWENDUNG ZUM OXIDATIVEN FÄRBEN
NOVEL CATIONIC COUPLERS AND THEIR USE FOR OXIDATION DYEING

(30) Priorité: 21.01.1999 FR 9900639
(43) Date de publication de la demande: 10.01.2001
(73) Titulaire: L'OREAL, 75008 Paris (FR)
(72) Inventeur: VANDENBOSSCHE, Jean-Jacques, F-40400 Tartas (FR); VIDAL, Laurent, F-75013 Paris (FR); SAUNIER, Jean-Baptiste, F-75014 Paris (FR); LAGRANGE, Alain, F-77700 Coupvray (FR)
(74) Mandataire: Miszputen, Laurent
(86) Numéro de dépôt international: FR0000140
(87) Numéro de publication internationale: WO00043356

(56) Documents cités:
- EP-A- 0 345 728
- GB-A- 1 563 674
- US-A- 5 344 463
- US-A- 5 672 180
- US-A- 5 849 042
- PATENT ABSTRACTS OF JAPAN vol. 014, no. 565 (P-1143), 17 décembre 1990 (1990-12-17) -& JP 02 244041 A (FUJI PHOTO FILM CO LTD), 28 septembre 1990 (1990-09-28)

## Description

L'invention concerne de nouveaux composés dérivés du napht-1-ol pouvant être représentés par la formule (I) et comportant au moins un groupement cationique Z de formule (II), leur utilisation à titre de coupleur pour la teinture d'oxydation des fibres kératiniques, les compositions pour la teinture d'oxydation des fibres kératiniques, et en particulier des fibres kératiniques humaines telles que les cheveux les comprenant en association avec au moins une base d'oxydation ; ainsi que les procédés de teinture d'oxydation les mettant en oeuvre.

Il est connu de teindre les fibres kératiniques et en particulier les cheveux humains avec des compositions tinctoriales contenant des précurseurs de colorant d'oxydation, en particulier des paraphénylènediamines, des ortho ou paraaminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols, des naphtols non cationiques ou encore certains composés hétérocycliques tels que par exemples des coupleurs indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Or la demanderesse vient maintenant de découvrir, de façon totalement inattendue et surprenante, que l'utilisation à titre de coupleur de nouveaux composés du type 2-acylaminonapht-1-ol, 2-amidonapht-1-ol et 2-sutfonylaminonapht-1-ol, comportant au moins un groupement cationique Z de formule (II) telle que définie ci-après, et pouvant être représentés par la formule (I) telle que définie ci-après, permet d'obtenir des compositions tinctoriales conduisant à des colorations puissantes, dans les nuances allant du rouge au bleu et présentant de plus une ténacité remarquable tant à la lumière qu'aux intempéries, aux lavages, à la transpiration ou encore à la permanente.

Ces découvertes sont à la base de la présente invention.

L'invention a donc pour premier objet un composé de formule (I) suivante, ainsi que ses sels d'addition avec un acide : 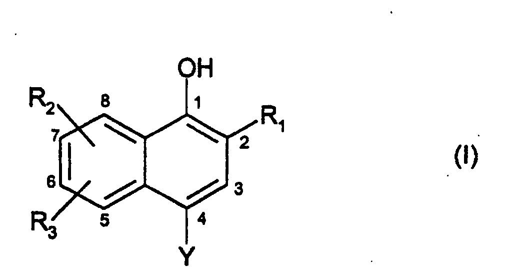 dans laquelle :
- R₁ représente un groupement choisi parmi -NR₄(C=O)R₅, -NR₆SO₂R₇ et -(C=O)NR₆R₈, les radicaux R_{4,} R₅, R_{6,} R₇ et R₈ étant tels que définis ci-après ;
- R₂ et R₃, identiques ou différents, représentent un atome d'hydrogène; un atome d'halogène; un groupement Z tel que défini ci-après; un radical comportant de 1 à 20 atomes de carbone, linéaire ou ramifié (la ou les ramifications pouvant former un ou plusieurs cycles carbonés comportant de 3 à 7 chaînons), pouvant contenir une ou plusieurs liaisons doubles et/ou une ou plusieurs liaisons triples (lesdites liaisons doubles conduisant éventuellement à des groupements aromatiques), dont un ou plusieurs atomes de carbone peuvent être remplacés par un atome d'oxygène, d'azote, ou de soufre ou par un groupement SO₂, et dont les atomes de carbone peuvent, indépendamment les uns des autres, être substitués par un ou plusieurs atomes d'halogène ;
étant entendu que :
- lesdits radicaux R₂ et R₃ ne peuvent être reliés au cycle benzénique de la formule (I) par une liaison -NH-NH-; et que R₂ et R₃ ne comportent pas de liaison peroxyde ni de radicaux diazo, nitro et nitroso ;
- que R₂ et R₃ ne peuvent représenter un radical hydroxyle ou un radical thio ;
- que R₂ et R₃ ne peuvent représenter, simultanément, un radical amino, alkylamino, acylamino ou sulfonylamino ;
- Y représente un atome d'hydrogène ou d'halogène ; un groupement -OR₉, -SR₉, ou -NH-SO₂R₉ dans lesquels R₉ représente un radical alkyle en C₁-C₆, linéaire ou ramifié (la ou les ramifications pouvant alors former un ou plusieurs cycles comportant de 3 à 6 chaînons), substitué ou non substitué par un ou plusieurs radicaux choisis dans le groupe constitué par un atome d'halogène, un radical hydroxy, alcoxy en C₁-C₄, amino et aminoalkyl en C₁-C₄ ; un radical phényle, substitué ou non substitué par un ou deux radicaux choisis dans le groupe constitué par un radical alkyle en C₁-C₄, trifluorométhyle, carboxy, alcoxycarbonyle en C₁-C₄, halogène, hydroxy, alcoxy en C₁-C₄, amino et aminoalkyl en C₁-C₄ ; ou un radical benzyle ;
- R_{4,} R₆ et R₈, identiques ou différents, représentent un atome d'hydrogène; un groupement Z tel que défini ci-après; un radical comportant de 1 à 15 atomes de carbone, linéaire ou ramifié (la ou les ramifications pouvant former un ou plusieurs cycles carbonés comportant de 3 à 7 chaînons), pouvant contenir une ou plusieurs liaisons doubles et/ou une ou plusieurs liaisons triples (lesdites liaisons doubles conduisant éventuellement à des groupements aromatiques), dont un ou plusieurs atomes de carbone peuvent être remplacés par un atome d'oxygène, d'azote, ou de soufre ou par un groupement SO₂, et dont les atomes de carbone peuvent, indépendamment les uns des autres, être substitués par un ou plusieurs atomes d'halogène ;
   étant entendu que ledit groupement SO₂ n'est pas directement lié à l'atome d'azote portant le radical R₄ ou R₆,
   étant entendu que le groupement -(C=O)- n'est pas directement lié à l'atome d'azote portant le radical R₆;
   étant entendu que lesdits radicaux R_{4,} R₆ et R₈ ne comportent pas de liaison peroxyde ni de radicaux diazo, nitro et nitroso;
   étant entendu que les radicaux R_{4,} R₆ et R₈ ne peut représenter un radical hydroxy, un radical thio, un radical amino, un radical alcoxy, un radical alkylthio;
- R₅ et R₇, identiques ou différents, représentent un atome d'hydrogène; un groupement Z tel que défini ci-après; un radical comportant de 1 à 20 atomes de carbone, linéaire ou ramifié (la ou les ramifications pouvant former un ou plusieurs cycles carbonés comportant de 3 à 7 chaînons), pouvant contenir une ou plusieurs liaisons doubles et/ou une ou plusieurs liaisons triples (lesdites liaisons doubles conduisant éventuellement à des groupements aromatiques), dont un ou plusieurs atomes de carbone peuvent être remplacés par un atome d'oxygène, d'azote, ou de soufre ou par un groupement SO₂, et dont les atomes de carbone peuvent, indépendamment les uns des autres, être substitués par un ou plusieurs atomes d'halogène ;
   étant entendu que lesdits radicaux R₅ et R₇ ne comportent pas de liaisons peroxydes ni de radicaux diazo, nitro et nitroso;
   étant entendu que R₅ ne peut pas représenter un radical hydroxyle ou un radical thio ;
   étant entendu que R₇ ne peut pas représenter un radical thio ;
   étant entendu que les radicaux R₄ et R₅ d'une part, et les radicaux R₆ et R₈ d'autre part, peuvent en outre être reliés pour former, indépendamment les uns des autres, un cycle saturé ou insaturé comportant de 5 à 7 chaînons, constitué de carbone, d'azote et/ou d'acyle, chaque chaînon étant substitué ou non substitué par 1 ou 2 radicaux R, identiques ou différents, R étant un radical alkyle en C₁-C₈, linéaire ou ramifié (la ou les ramifications pouvant alors former un ou plusieurs cycles comportant de 3 à 7 chaînons), pouvant contenir une ou plusieurs liaisons doubles et/ou une ou plusieurs liaisons triples (lesdites liaisons doubles conduisant éventuellement à des groupements aromatiques), et dont un ou plusieurs atomes de carbone peuvent être remplacés par un atome d'oxygène, d'azote, ou de soufre ou par un groupement SO₂, et dont les atomes de carbone peuvent, indépendamment les uns des autres, être substitués par un ou plusieurs atomes d'halogène ; ledit cycle ne comportant pas de liaisons peroxydes ni de radicaux diazo, nitro et nitroso;
- Z est un groupement cationique représenté par la formule (II) suivante: 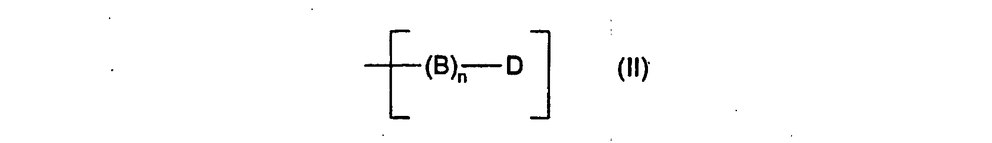
dans laquelle :
- n peut prendre la valeur 0 ou 1.
- B représente un radical alkyle comportant de 1 à 15 atomes de carbone, linéaire ou ramifié (la ou les ramifications pouvant alors former un ou plusieurs cycles comportant de 3 à 7 chaînons), pouvant contenir une ou plusieurs liaisons doubles et/ou une ou plusieurs liaisons triples, lesdites liaisons doubles conduisant éventuellement à des groupements aromatiques, et dont un ou plusieurs atomes de carbone peuvent être remplacés par un atome d'oxygène, d'azote, ou de soufre ou par un radical -SO₂; et dont un ou plusieurs atomes de carbone peuvent, indépendamment les uns des autres, être substitués par un ou plusieurs atomes d'halogène ou par un ou plusieurs groupements Z ; ledit radical B ne comportant pas de liaison peroxyde ni de radicaux diazo, nitro ou nitroso ;
- D est choisi parmi les groupements cationiques de formules (III) et (IV) suivantes : 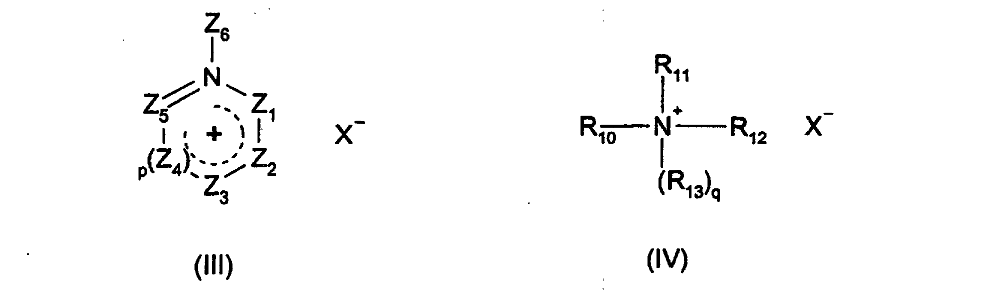
dans lesquelles :
- p et q peuvent, indépendamment l'un de l'autre, prendre la valeur 0 ou 1;
- le radical B est relié au groupement D par l'un quelconque des atomes du radical D ;
- lorsque n = 0 et q = 0, alors le groupement de formule (IV) peut être relié au composé de formule (I) directement par l'atome d'azote de l'ammonium quaternaire;
- Z₁, Z₂, Z₃ et Z₄, indépendamment les uns des autres, représentent un atome d'oxygène ; un atome de soufre ; un atome d'azote substitué ou non substitué par un radical R₁₄ ; un atome de carbone substitué ou non substitué par un ou deux radicaux R₁₄, identiques ou différents ;
- Z₅ représente un atome d'azote ou un atome de carbone substitué ou non substitué par un radical R₁₄ ;
- Z₆ peut prendre les mêmes significations que celles indiquées ci-dessous pour le radical R₁₄ ; étant entendu que Z₆ est différent d'un atome d'hydrogène;
les radicaux Z₁ ou Z₅ peuvent, en outre, former avec Z₆ un cycle saturé ou insaturé comportant de 5 à 7 chaînons, chaque chaînon étant substitué ou non substitué par un ou deux radicaux R₁₄ identiques ou différents ;
- R₁₄ représente un atome d'hydrogène ; un groupement Z ; un radical comportant de 1 à 10 atomes de carbone, linéaire ou ramifié, pouvant contenir une ou plusieurs liaisons doubles et/ou une ou plusieurs liaisons triples, lesdites liaisons doubles pouvant alors éventuellement conduire à des groupes aromatiques, et dont un ou plusieurs atomes de carbone peuvent être remplacés par un atome d'oxygène, d'azote, ou de soufre, ou par un groupe SO₂, et dont un ou plusieurs atomes de carbone peuvent, indépendamment les uns des autres, être substitués par un ou plusieurs atomes d'halogène ; ledit radical ne comportant pas de liaison peroxyde ni de radicaux diazo, nitro et nitroso;
   deux des radicaux adjacents Z₁, Z₂, Z₃, Z₄ et Z₅ pouvant en outre former un cycle comportant de 5 à 7 chaînons, chaque chaînon étant indépendamment représenté par un atome de carbone substitué ou non substitué par un ou deux radicaux R₁₄ identiques ou différents ; un atome d'azote substitué ou non substitué par un radical R₁₄ ; un atome d'oxygène ; ou un atome de soufre ;
- R₁₀, R₁₁, R₁₂ et R₁₃, identiques ou différents, ont les mêmes significations que celles indiquées ci-dessus pour le radical R₁₄ ;
   les radicaux R₁₀, R₁₁, R₁₂ et R₁₃ pouvant également former, deux à deux avec l'atome d'azote quaternaire auquel ils sont rattachés, un ou plusieurs cycles saturés comportant de 5 à 7 chaînons, chaque chaînon étant indépendamment représenté par un atome de carbone substitué ou non substitué par un ou deux radicaux R₁₄ identiques ou différents ; un atome d'azote substitué ou non substitué par un radical R₁₄ ; un atome d'oxygène ; ou un atome de soufre ;
- X ⁻ représente un anion organique ou minéral et est de préférence choisi dans le groupe constitué par un groupement halogénure tel que chlorure, bromure, fluorure, iodure ; un hydroxyde ; un sulfate ; un hydrogénosulfate ; un alkyl(C₁-C₆)sulfate tel que par exemple un méthylsulfate ou un éthylsulfate ; un acétate ; un tartrate ; un oxalate ; un alkyl(C₁-C₆)sulfonate tel que par exemple un méthylsulfonate ; un arylsulfonate substitué ou non substitué par un radical alkyl en C₁-C₄ tel que par exemple un toluylsulfonate ;
   étant entendu qu'au moins un des groupements R₁ à R₃ représente ou contient un groupement Z.

Selon l'invention, lorsque qu'il est indiqué que un ou plusieurs des atomes de carbone du ou des radicaux R₁ à R₈ peuvent être remplacés par un atome d'oxygène, d'azote, ou de soufre ou par un groupement SO₂, et/ou que lesdits radicaux R₁ à R₈ peuvent contenir une ou plusieurs liaisons doubles et/ou une ou plusieurs liaisons triples, cela signifie que l'on peut, à titre d'exemple, faire les transformations suivantes :

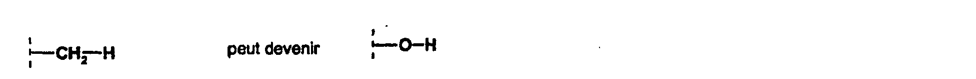

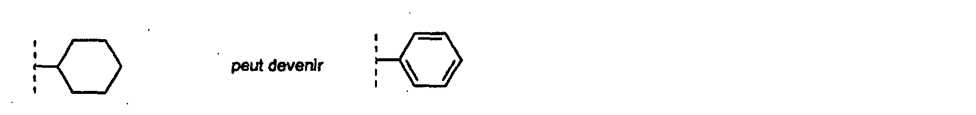

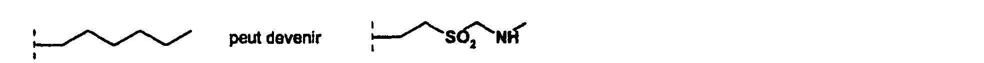

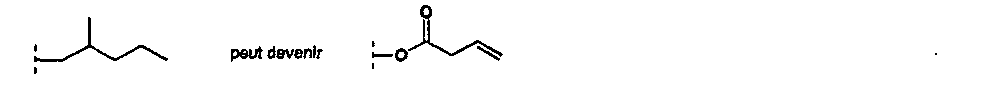

Selon l'invention, R₂ et R₃ représentent de préférence, indépendamment l'un de l'autre, un atome d'hydrogène ou de chlore ; un groupement Z; un radical méthyle ou méthoxy; un radical amino, méthylamino, 2-hydroxyéthylamino ; un groupement -NH(CO)R₁₅ dans lequel R₁₅ représente un des radicaux listés dans le groupe **(G1)** constitué par un radical méthyle, éthyle, propyle, isopropyle, butyle, isobutyle, tert-butyle, pentyle, isopentyle, néopentyle, hexyle, cyclopropyle, cyclobutyle, cyclopentyle, cyclopentylméthyle, 3-cyclopentyl-propyle, cyclohexyle, 2-cyclohexyl-éthyle, norbornane-2-yl, vinyle, 1-méthylvinyle, 2-méthylvinyle, 2,2-diméthylvinyle, allyle, 3-butenyle, phényle, méthylphényle, diméthylphényle, 2,4,6-triméthylphényle, 4-éthylphényle, (trifluorométhyl)phényle, hydroxyphényle, méthoxyphényle, éthoxyphényle, acétoxyphényle, (trifluorométhoxy)phényle, aminophényle, 4-diméthylaminophényle, fluorophényle, difluorophényle, fluoro(trifluorométhyl)phényle, chlorophényle, dichlorophényle, bromophényle, napht-1-yle, napht-2-yle, (2-méthoxy)napht-1-yl, benzyle, 4'-méthoxybenzyle, 2',5'-diméthoxybenzyle, 3',4'-diméthoxybenzyle, 4'-fluorobenzyle, 4'-chlorobenzyle, phénéthyle, 2-phénylvinyle, (1-naphtyl)méthyle, (2-naphtyl)méthyle ; tétrahydrofuran-2-yl, furan-2-yl, 5-méthyl-2-(trifluorométhyl)furan-3-yl, 2-méthyl-5-phénylfuran-3-yl, thiophène-2-yl, (thiophène-2-yl)méthyle, 3-chlorothiophène-2-yl, 2,5-dichlorothiophène-3-yl, benzothiophène-2-yl, 3-chlorobenzothiophène-2-yl, isoxazole-5-yl, 5-méthylisoxazole-3-yl, 3,5-diméthylisoxazole-4-yl, 1,3-diméthylpyrazole-5-yl, 1-éthyl-3-méthylpyrazole-5-yl, 1-tertbutyl-3-méthylpyrazole-5-yl, 3-tertbutyl-1-méthylpyrazole-5-yl, 4-bromo-1-éthyl-3-méthylpyrazole-5-yl, pyridinyl, chloropyridinyl, dichloropyridinyl, 5-(bromo)pyridin-3-yl, piperazin-2-yl, 2-chloro-4-(trifluorométhyl)pyperimidine-5-yl, quinoxal-2-yl, benzofurazan-5-yl ; fluorométhyle, difluorométhyle, trifluorométhyle, 1,1,2,2-tétrafluoroéthyle, pentafluoroéthyle, heptafluoropropyle, 1,1,2,2,3,3,4,4-octafluorobutyle, nonafluorobutyle, chlorométhyle, chloroéthyle, 1,1-diméthyl-2-chloroéthyle, 1,2-dichloroéthyle, 1-chloropropyle, 3-chloropropyle, 4-chlorobutyle, hydroxyméthyle, méthoxyméthyle, phénoxyméthyle, (4-chlorophénoxy)méthyle, benzyloxyméthyle, acétoxyméthyle, 1,2-dihydroxyéthyle, 1-phénoxyéthyle, 1-acétoxyéthyle, 2-(2-carboxyéthoxy)éthyle, 1-phénoxyéthyle, 1-acétoxyéthyle, (méthoxycarbonyl)méthyle, 2-carboxyéthyle, 2-(méthoxycarbonyl)éthyle, 2-carboxycylopropyle, 2-carboxycyclohexane, méthoxy, éthoxy, propoxy, isopropoxy, butoxy, isobutoxy, pentoxy, néopentoxy, hexyloxy, cyclopentyloxy, cyclohexyloxy, vinyloxy, allyloxy, propargyloxy, chlorométhoxy, 1-chloroéthoxy, 2-méthoxyéthoxy, 4-chlorobutoxy, phénoxy, 4-méthyphénoxy, 4-fluorophénoxy, 4-bromophénoxy, 4-chlorophénoxy, 4-méthoxyphénoxy, naphth-2-yloxy, benzyloxy; amino, méthylamino, éthylamino, propylamino, isopropylamino, butylamino, cyclohexylamino, allylamino, 2-chloroéthylamino, 3-chloropropylamino, carboxyméthylamino, phénylamino, fluorophénylamino, (trifluorométhyl)phénylamino, chlorophénylamino, bromophénylamino, 4-acétylphénylamino, méthoxyphénylamino, (trifluorométhoxy)phénylamino, naphth-1-ylamino, benzylamino, phénéthylamino, pyrid-3-ylamino, diméthylamino, 1-pyrolidinyle, 4-morpholinyle; un groupement -NHSO₂R₁₆ dans lequel R₁₆ représente un des radicaux listés dans le groupe **(G2)** constitué par les radicaux méthyle, trifluorométhyle, éthyle, 2-chloroéthyle, propyle, 3-chloropropyle, isopropyle, butyle, thiophène-2-yl, hydroxy, éthoxy, amino, diméthylamino;

Parmi ces radicaux, on préfère plus particulièrement pour R₂ et R₃, indépendamment l'un de l'autre, un atome d'hydrogène ; un groupement -O-E-D', -NH-E-D', -NH(CO)-D', -NH(CO)-E-D', -NH(CO)O-E-D', -NH(CO)NH-E-D' ou -NH(SO₂)-E-D', dans lesquels -E- représente un bras -(CH₂)_{q}-, q étant un nombre entier égal à 1 ou 2, et dans lesquels D' représente les groupements 3-méthylimidazolidinium-1-yl, 3-(2-hydroxyéthyl)-imidazolidinium-1-yl, 1,2,4-triazolinium-1-yl, 1,2,4-triazolinium-4-yl, N-alkyl(C1-C4)pyridinium-2-yl, N-alkyl(C₁-C₄)pyridinium-3-yl, N-alkyl(C₁-C₄)pyridinium-4-yl, N-(2-hydroxyéthyl) pyridinium-2-yl, N-(2-hydroxyéthyl)pyridinium-3-yl, N-(2-hydroxyéthyl)pyridinium-4-yl, pyridinium-1-yl, trialkyl(C1-C4)ammonium-N-yl, 1-méthylpipéridinium-1-yl et 1,4-dimétylpipérazinium-1-yl ; un radical méthyle, méthoxy, amino, ou méthylamino ; ou un groupement -NH(CO)R₁₇ dans lequel R₁₇ représente un des radicaux listés dans le groupe **(G2)** tel que défini ci-dessus ; ou un groupement méthanesulfonylamino, éthanesulfonylamino ou diméthylaminosulfonylamino.

Selon l'invention, R₄ représente de préférence un atome d'hydrogène ou un groupement A₁, A₂, A₃, A₄ ou A₅, éventuellement séparés de l'azote du groupement -NR₄(C=O)R₅ par un groupement -(CO)-.

On entend par groupement A₁ un radical alkyle en C₁-C₈, linéaire ou ramifié, pouvant porter une ou deux doubles liaisons ou une triple liaison, être substitué ou non substitué par un groupement choisi parmi un groupement A₂, un groupement A₄, un groupement A₅, être substitué ou non substitué par un ou deux groupements, identiques ou différents, choisis parmi les groupements N-alkyl(C₁-C₃)amino, N-alkyl(C₁-C₃)-N-alkyl(C₁-C₃)amino, alkoxy(C₁-C₆), oxo, alcoxycarbonyle, acyloxy, amide, acylamino, uréyle, sulfoxy, sulfonyle, sulfonamido, sulfonylamino, bromo, cyano, carboxy, et être substitué ou non substitué par un ou plusieurs groupements hydroxyle, fluoro ou chloro.

On entend par groupement A₂, un groupement aromatique de type phényle ou naphtyle, pouvant être substitué ou non substitué par un à trois groupements, identiques ou différents, choisis parmi les groupements méthyle, trifluorométhyle, éthyle, isopropyle, butyle, pentyle, fluoro, chloro, bromo, méthoxy, trifluorométhoxy, éthoxy, propyloxy, acétyloxy, acétyle et cyano.

On entend par groupement A₃ des groupements hétéroaromatiques choisis parmi les groupements furanyle, thiophényle, pyrrolyle, imidazolyle, thiazolyle, oxazolyle, 1,2,3-triazolyle, 1,2,4-triazolyle, isoxazolyle, isothiazolyle, pyrazolyle, pyrazoltriazolyle, pyrazoloimidazolyle, pyrrolotriazolyle, pyrazolopyrimidyle, pyrazolopyridyle, pyridyle, pyrimidyle, benzoimidazolyle, benzoxazolyle, benzothiazolyle, indolyle, indolidinyle, isoindolyle, indazolyle, benzotriazolyle, quinolinyle, benzoimidazolyle, benzopyrimidyle, lesdits groupements hétéroaromatiques étant substitués ou non substitués par 1 à 3 radicaux choisis parmi les radicaux alkyle en C₁-C₄, linéaire ou ramifié, monohydroxyalkyle en C₁-C₄, polyhydroxyalkyle en C₂-C₄, carboxy, alkoxycarbonyle, halogène, amido, amino et hydroxy.

On entend par groupement A₄, un radical cycloalkyle en C₃-C₇, un radical norbomanyle, portant ou non une double liaison et substitué ou non substitué par 1 ou 2 radicaux choisis parmi les radicaux alkyle en C₁-C₄, linéaire ou ramifié, monohydroxyalkyle en C₁-C₄, polyhydroxyalkyle en C₂-C₄, carboxy, alkoxycarbonyle, halogène, amido, amino et hydroxy.

On entend par groupement A₅ un hétérocycle choisi parmi les cycles dihydrofuranyle, tétrahydrofuranyle, butyrolact-one-yle, dihydrothiophényle, tétrahydrothiophényle, tétrahydrothiophén-one-yle, iminothiolane, dihydropyrrolyle, pyrrolidinyle, pyrrolidin-one-yle, imidazolidin-one-yle, imidazolidinthione-yle, oxazolidinyle, oxazolidin-one-yle, oxazolanethione, thiazolidinyle, isothiazol-one-yle, mercaptothiazolinyle, pyrazolidin-one-yle, iminothiolane, dioxolanyle, pentalactone, dioxanyle, dihydropyridinyle, pipéridinyle, pentalactame, morpholinyle, pyrazoli(di)nyle, pyrimi(di)nyl, pyrazinyle, pipérazinyle et azépinyl.

Parmi ces radicaux, R₄ représente plus particulièrement un atome d'hydrogène; un radical méthyle, éthyle, isopropyle, allyle, phényle, benzyle, fluorobenzyle, hydroxybenzyle, difluorobenzyle, trifluorobenzyle, chlorobenzyle, bromobenzyle, méthoxybenzyle, diméthoxybenzyle, (trifluorométhoxy)benzyle, 3,4-méthylèndioxybenzyle, 6-chloropipéronyle, 4-méthylthiobenzyle, 4-méthylsulfonylbenzyle, 4-acétylaminobenzyle, 4-carboxybenzyle, 1-naphtométhyle, 2-naphtométhyl; ou un groupement 2-hydroxéthyle, 2-méthoxyéthyle ou 2-éthoxyéthyle.

De façon encore plus préférentielle, R₄ représente un atome d'hydrogène ou un radical méthyle.

Selon l'invention, R₅ désigne de préférence un atome d'hydrogène, un groupement amino ; un groupement Z ; un groupement A₁, A₂, A₃, A₄ ou A₅ tels que définis précédemment, éventuellement séparé du carbone (en position 2) de la fonction amide du composé de formule (I) par un groupement -O-, -NH, -NAlkyl(C₁-C₃)-, -(CO)-.

Parmi ces radicaux, on préfère plus particulièrement pour R₅ un groupement Z ou un radical choisi dans le groupe (G1) tel que défini précédemment.

De façon encore plus préférentielle, R₅ représente un groupement Z ; un radical choisi dans le groupe **(G3)** constitué par un radical méthyle, éthyle, propyle, allyle, phényle, tétrahydrofuran-2-yl, furan-2-yl, thiophène-2-yl, pyridinyle, piperazin-2-yl, fluorométhyle, chlorométhyle, 2-chloroéthyle, méthoxyméthyle, acétoxyméthyle, 1,2-dihydroxyéthyle, méthoxycarbonyl, 2-carboxyéthyle, méthoxy, éthoxy, propoxy, allyloxy, 2-chloroéthoxy, 2-méthoxyéthoxy, amino, éthylamino, allylamino, 2-chloroéthylamino, pyridytamino, diméthylamino, 1-pyrolidinyle, 4-morpholinyle.

De façon encore plus préférentielle, R₅ représente un groupement -D', -E-D', -O-E-D' ou -NH-E-D' dans lesquels -E- représente un bras -(CH₂)_{q}-, q étant un nombre entier égal à 1 ou 2, et D' un groupement tel que défini précédemment ; un radical choisi dans le groupe **(G4)** constitué par un radical méthyle, méthoxyméthyle, 2-carboxyéthyle, méthoxy, amino, éthylamino et 1-pyrolidinyle.

Lorsque R₄ et R₅ forment un cycle, ledit cycle est de préférence choisi parmi les groupements 2-pyrrolidinon-1-yl, méthyl-2-pyrrolidinon-1-yl, 5-carboxy-2-pyrrolidinon-1-yl, 5-méthoxycarbonyl-2-pyrrolidinone-1-yl, pyrazolinone-1-yl, succinimide-1-yl, 3,5-dicétopyrazolidin-1-yl, oxindolin-1-yl, maléimide-1yl, isoindole-1,3-dione-2-yl, 2-pipéridinone-1-yl et glutarimide-1-yl.

Selon l'invention, R₆ représente de préférence un atome d'hydrogène ou un groupement A₁, A₂, A₃, A₄ ou A₅ tels que précédemment définis.

On préfère plus particulièrement pour R₆ un atome d'hydrogène; un radical méthyle, éthyle, isopropyle, allyle; phényle, benzyle, fluorobenzyle, hydroxybenzyle, difluorobenzyle, trifluorobenzyle, chlorobenzyle, bromobenzyle, méthoxybenzyle, diméthoxybenzyle, (trifluorométhoxy)benzyle, 3,4-méthylèndioxybenzyle, 6-chloropipéronyle, 4-méthylthiobenzyle, 4-méthylsulfonylbenzyle, 4-acétylaminobenzyle, 4-carboxybenzyle, 1-naphtométhyle, 2-naphtométhyle; un groupement 2-hydroxéthyle, 2-méthoxyéthyle ou 2-éthoxyéthyle.

Encore plus particulièrement, R₆ représente un atome d'hydrogène ou un radical méthyle.

Selon l'invention, R₈ désigne de préférence un groupement Z; un atome d'hydrogène ou un groupement A₁, A₂, A₃, A₄ ou A₅ tels que précédemment définis.

On préfère plus particulièrement pour R₈ un groupement Z; un atome d'hydrogène; un radical méthyle, éthyle, isopropyle, allyle; phényle, benzyle, fluorobenzyle, hydroxybenzyle, difluorobenzyle, trifluorobenzyle, chlorobenzyle, bromobenzyle, méthoxybenzyle, diméthoxybenzyle, (trifluorométhoxy)benzyle, 3,4-méthylèndioxybenzyle, 6-chloropipéronyle, 4-méthylthiobenzyle, 4-méthylsulfonylbenzyle, 4-acétylaminobenzyle, 4-carboxybenzyle, 1-naphtométhyle ou 2-naphtométhyle ; un groupement 2-hydroxéthyle, 2-méthoxyéthyle ou 2-éthoxyéthyle.

De façon encore plus préférentielle, R₈ représente un atome d'hydrogène ou un radical méthyle, un groupement -D', -E-D', dans lesquels -E- représente un bras -(CH₂)_{q}-, q étant un nombre entier égal à 1 ou 2, et D' un groupement tel que défini précédemment.

Lorsque R₆ et R₈ forment un cycle, ledit cycle est de préférence choisi parmi les groupements pyrolidinyl, pipéridinyl, morpholinyl, pyrazolyl, isoxazolyl, imidazolyl, thiazolyl, indolyl, indolidinyl, indazolyl, pyrazolotriazolyl, pyrrolotriazolyl, pyrazoloimidazolyl, pyrazolidinyl, thiomorpholinyl, thiazolyl et pyrazinyl.

Selon l'invention, R₇ désigne de préférence, un groupement amino; un groupement Z ; un groupement A₁, un groupement A₂, un groupement A₃, un groupement A₄ ou un groupement A₅ tels que définis précédemment, éventuellement séparé du soufre du groupe sufonyl du composé de formule (I) par un groupement -NH, ou NAlkyl(C₁-C₃)-.

Parmi ces radicaux, on préfère plus particulièrement pour R₇ un groupement Z, un des radicaux listés dans le groupe **(G4)** constitué par les radicaux méthyle, trifluorométhyle, éthyle, 2-chloroéthyle, propyle, 3-chloropropyle, isopropyle, butyle, thiophène-2-yl, hydroxy, éthoxy, amino et diméthylamino.

De façon encore plus préférentielle, R₇ représente un groupement -D', -E-D' ou -NH-E-D', dans lesquels -E- représente un bras -(CH₂)_{q}-, q étant un nombre entier égal à 1 ou 2, et D' un groupement tel que défini précédemment; un radical méthyle, éthyle ou diméthylamino.

Selon l'invention, Y représente de préférence un radical choisi dans le groupe comprenant : un atome d'hydrogène, de chlore, de fluor, de brome ; les groupements méthoxy, éthoxy, propoxy, benzyloxy, phénoxy ; -OCH₂CH₂OMe; -OCH₂CH₂OMe; -OCH₂CH₂NMe₂; -OCH₂(CO)OH, -OCH₂(CO)OMe, -OCH₂(CO)OEt, -SCH₂CH₂CO₂H et NHSO₂Me.

Parmi ces radicaux, on préfère particulièrement pour Y, les radicaux choisis dans le groupe constitué par un atome d'hydrogène, de chlore, le groupement méthoxy, -OCH₂(CO)OH, -OCH₂(CO)OMe.

Le groupement D peut être choisi parmi notamment les groupements imidazolinium, thiazolinium, oxazolinium, pyrrolinium, 1,2,3-triazolinium, 1,2,4-triazolinium, isoxazolinium, isothiazolinium, imidazolidinium, thiazolidinium, pyrazolinium, pyrazolidinium, oxazolidinium, pyrazoltriazolinium, pyrazoloimidazolinium, pyrrolotriazolinium, pyrazolopyrimidinium, pyrazolopyridinium, pyridinium, pyrimidinium, pyrazinium, triazinium, benzoimidazolinium, benzoxazolinium, benzothiazolinium, indolinium, indolidinium, isoindolinium, indazolinium, benzotriazolinium, quinolinium, tetrahydroquinolinium, benzoimidazolidinium, benzopyrimidinium, tétra-alkyl(C₁-C₄)ammonium, dialkylpipéridinium, dialkylpyrrolidinium, dialkylmorpholinium, dialkylthiomorpholinium, dialkylpipérazinium, azépinium, et 1,4-diazabicyclo[2,2,2]octanium.

De façon encore plus préférentielle, le groupement D peut être choisi parmi les groupements 3-méthylimidazolidinium-1-yl, 3-(2-hydroxyéthyl)imidazolidinium-1-yl, 1,2,4-triazolinium-1-yl, 1,2,4-triazolinium-4-yl, N-alkyl(C₁-C₄)pyridinium-2-yl, N-alkyl(C₁-C₄)pyridinium-3-yl, N-alkyl(C₁-C₄)pyridinium-4-yl, N-(2-hydroxyéthyl) pyridinium-2-yl, N-(2-hydroxyéthyl)pyridinium-3-yl, N-(2-hydroxyéthyl) pyridinium-4-yl, pyridinium-1-yl, trialkyl(C₁-C₄)ammonium-N-yl, 1-méthylpipéridinium-1-yl et 1,4-dimétylpipérazinium-1-yl.

Parmi les composés de formule (I), on préfère particulièrement ceux dans lesquels :
i) - le radical R₁ représente un radical choisi dans le groupe **(G5)** constitué par un radical acétylamino, méthanesulfonylamino, amido, méthylamido, un groupement -NH-C(O)-CH₂-D1, un groupement -NH-SO₂-CH₂-CH₂-D1, un groupement -(CO)-NH-CH₂-CH₂-D1, dans lesquels D1 représente un groupement 3-méthylimidazolidinium-1-yl, triazolinium-1-yl, pyridinium-1-yl ou triméthylammonium-N-yl;
   - le radical R₂ est en position 5 et représente un radical choisi dans le groupe **(G6)** constitué par un radical amino, méthylamino, acétylamino, méthanesulfonylamino, un groupement -NH-[C(O)]ₘ-CH₂-D1 dans lequel m représente 0 ou 1, ou un groupement -NH-SO₂-CH₂-CH₂-D1,
   - le radical R₃ représente un atome d'hydrogène;
   - le radical Y représente un radical choisi dans le groupe **(G7)** constitué par un atome d'hydrogène, un radical chlore et un radical méthoxy ;
   ou
ii) - le radical R₁ représente un radical choisi dans le groupe **(G5)** tel que défini ci-dessus;
   - le radical R₂ est situé en position 8 et représente un radical choisi dans le groupe **(G6)** tel que défini ci-dessus;
   - le radical R₃ représente un atome d'hydrogène;
   - le radical Y représente un radical choisi dans le groupe **(G7)** tel que défini ci-dessus.
   ou
iii) - le radical R₁ représente un radical choisi dans le groupe **(G5)** tel que défini ci-dessus;
   - les radicaux R₂ et R₃ représentent un atome d'hydrogène;
   - le radical Y représente un radical choisi dans le groupe **(G7)** tel que défini ci-dessus.

Parmi les composés de formule (I) conforme à l'invention, on peut tout particulièrement citer :
- le chlorure de 3-[(1-hydroxy-naphthalèn-2-ylcarbamoyl)-méthyl]-1-méthyl-3H-imidazol-1-ium ;
- le dichlorure de 3-[(1-hydroxy-5-(2-(3-méthyl-1H-imidazol-3-ium-1-yl)acétyl)amino-naphthalèn-2-ylcarbamoyl)-méthyl]-1-méthyl-3H-imidazol-1-ium ;
- le dichlorure de 3-[(1-hydroxy-8-(2-(3-méthyl-1H-imidazol-3-ium-1-yl)acétyl)amino-naphthalèn-2-ylcarbamoyl)-méthyl]-1-méthyl-3H-imidazol-1-ium ;
- le chlorure de 3-[(1-hydroxy-5-amino-naphthalèn-2-ylcarbamoyl)-méthyl]-1-méthyl-3H-imidazol-1-ium ;
- le chlorure de 3-[(1-hydroxy-5-acétylamino-naphthalèn-2-ylcarbamoyl)méthyl]-1-méthyl-3H-imidazol-1-ium ;
- le chlorure de 3-[(1-hydroxy-5-méthoxycarbonylamino-naphthalèn-2-ylcarbamoyl)-méthyl]-1-méthyl-3H-imidazol-1-ium ;
- le chlorure de 3-[(1-hydroxy-2-acétylamino-naphthalèn-5-ylcarbamoyl)méthyl]-1-méthyl-3H-imidazol-1-ium ;
- le chlorure de 3-[(1-hydroxy-2-méthoxycarbonylamino-naphthalèn-5-ylcarbamoyl)-méthyl]-1-méthyl-3H-imidazol-1-ium ;
- le chlorure de 3-[(1-hydroxy-5-méthanesulfonylamino-naphthalèn-2-ylcarbamoyl)-méthyl]-1-méthyl-3H-imidazol-1-ium ;
- le chlorure de 3-[(1-hydroxy-8-amino-naphthalèn-2-ylcarbamoyl)-méthyl]-1-méthyl-3H-imidazol-1-ium;
- le chlorure de 3-[(1-hydroxy-8-acétylamino-naphthalèn-2-ylcarbamoyl)méthyl]-1-méthyl-3H-imidazol-1-ium ;
- le chlorure de 3-[(1-hydroxy-8-méthoxycarbonylamino-naphthalèn-2-ylcarbamoyl)-méthyl]-1-méthyl-3H-imidazol-1-ium ;
- le chlorure de 3-[(1-hydroxy-2-acétylamino-naphthalèn-8-ylcarbamoyl)méthyl]-1-méthyl-3H-imidazol-1-ium ;
- le chlorure de 3-[(1-hydroxy-2-méthoxycarbonylamino-naphthalèn-8-ylcarbamoyl)-méthyl]-1-méthyl-3H-imidazol-1-ium ;
- le chlorure de 3-[(1-hydroxy-8-méthanesulfonylamino-naphthalèn-2-yl-carbamoyl)-méthyl]-1-méthyl-3H-imidazol-1-ium ;
- le chlorure de 1-[(1-hydroxy-naphthalèn-2-ylcarbamoyl)-méthyl]-pyridinium ;
- le dichlorure de 1-[(1-hydroxy-5-(2-(pyridinium-1-yl)acétyl)amino-naphthalèn-2-ylcarbamoyl)-méthyl]-pyridinium ;
- le dichlorure de 1-[(1-hydroxy-8-(2-(pyridinium-1-yl)acétyl)amino-naphthalèn-2-ylcarbamoyl)-méthyl]-pyridinium ;
- le chlorure de 1-[(1-hydroxy-5-amino-naphthalèn-2-ylcarbamoyl)-méthyl]-pyridinium ;
- le chlorure de 1-[(1-hydroxy-5-acétylamino-naphthalèn-2-ylcarbamoyl)-méthyl]-pyridinium ;
- le chlorure de 1-[(1-hydroxy-5-méthoxycarbonylamino-naphthalèn-2-ylcarbamoyl)-méthyl]-pyridinium ;
- le chlorure de 1-[(1-hydroxy-2-acétylamino-naphthalèn-5-ylcarbamoyl)-méthyl]-pyridinium ;
- le chlorure de 1-[(1-hydroxy-2-méthoxycarbonylamino-naphthalèn-5-ylcarbamoyl)-méthyl]-pyridinium ;
- le chlorure de 1-[(1-hydroxy-5-méthanesulfonylamino-naphthalèn-2-ylcarbamoyl)-méthyl]-pyridinium ;
- le chlorure de 1-[(1-hydroxy-8-amino-naphthalèn-2-ylcarbamoyl)-méthyl]-pyridinium ;
- le chlorure de 1-[(1-hydroxy-8-acétylamino-naphthalèn-2-ylcarbamoyl)-méthyl]-pyridinium ;
- le chlorure de 1-[(1-hydroxy-8-méthoxycarbonylamino-naphthalèn-2-ylcarbamoyl)-méthyl]-pyridinium ;
- le chlorure de 1-[(1-hydroxy-2-acétylamino-naphthalèn-8-ylcarbamoyl)-méthyl]-pyridinium ;
- le chlorure de 1-[(1-hydroxy-2-méthoxycarbonylamino-naphthalèn-8-ylcarbamoyl)-méthyl]-pyridinium ;
- le chlorure de 1-[(1-hydroxy-8-méthanesulfonylamino-naphthalèn-2-ylcarbamoyl)-méthyl]-pyridinium ;
- le chlorure de 1-[(1-hydroxy-naphthalèn-2-ylcarbamoyl)-méthyl]-1,4-diméthyl-pipérazin-1-ium ;
- le dichlorure de 1-[(1-hydroxy-5-(2-(1,4-diméthyl-pipérazin-1-ium-1-yl)acétyl)-amino-naphthalèn-2-ylcarbamoyl)-méthyl]-1,4-diméthyl-pipérazin-1-ium ;
- le dichlorure de 1-[(1-hydroxy-8-(2-(1,4-diméthyl-pipérazin-1-ium-1-yl)acétyl)-amino-naphthalèn-2-ylcarbamoyl)-méthyl]-1,4-diméthyl-pipérazin-1-ium ;
- le chlorure de 1-[(1-hydroxy-5-amino-naphthalèn-2-ylcarbamoyl)-méthyl]-1,4-diméthyl-pipérazin-1-ium ;
- le chlorure de 1-[(1-hydroxy-5-acétylamino-naphthalèn-2-ylcarbamoyl)-méthyl]-1,4-diméthyl-pipérazin-1-ium ;
- le chlorure de 1-[(1-hydroxy-5-méthoxycarbonylamino-naphthalèn-2-ylcarbamoyl)-méthyl]-1,4-diméthyl-pipérazin-1-ium ;
- le chlorure de 1-[(1-hydroxy-2-acétylamino-naphthalèn-5-ylcarbamoyl)-méthyl]-1,4-diméthyl-pipérazin-1-ium ;
- le chlorure de 1-[(1-hydroxy-2-méthoxycarbonylamino-naphthalèn-5-ylcarbamoyl)-méthyl]-1,4-diméthyl-pipérazin-1-ium ;
- le chlorure de 1-[(1-hydroxy-5-méthanesulfonylamino-naphthalèn-2-ylcarbamoyl)-méthyl]-1,4-diméthyl-pipérazin-1-ium ;
- le chlorure de 1-[(1-hydroxy-8-amino-naphthalèn-2-ylcarbamoyl)-méthyl]-1,4-diméthyl-pipérazin-1-ium ;
- le chlorure de 1-[(1-hydroxy-8-acétylamino-naphthalèn-2-ylcarbamoyl)-méthyl]-1,4-diméthyl-pipérazin-1-ium ;
- le chlorure de 1-[(1-hydroxy-8-méthoxycarbonylamino-naphthalèn-2-ylcarbamoyl)-méthyl]-1,4-diméthyl-pipérazin-1-ium ;
- le chlorure de 1-[(1-hydroxy-2-acétylamino-naphthalèn-8-ylcarbamoyl)-méthyl]-1,4-diméthyl-pipérazin-1-ium ;
- le chlorure de 1-[(1-hydroxy-2-méthoxycarbonylamino-naphthalèn-8-ylcarbamoyl)-méthyl]-1,4-diméthyl-pipérazin-1-ium ;
- le chlorure de 1-[(1-hydroxy-8-méthanesulfonylamino-naphthalèn-2-ylcarbamoyl)-méthyl]-1,4-diméthyl-pipérazin-1-ium ;
et leurs sels d'addition avec un acide

Ces nouveaux composés de formule (I) peuvent être préparés selon dès méthodes bien connues de l'état de la technique et décrites par exemple dans les demandes de brevet ou brevets JP95-303798, JP01186859, JP63258843, DD285095

L'invention a également pour objet ('utilisation des composés de formule (I) à titre de coupleur pour la teinture d'oxydation des fibres kératiniques et en particulier des fibres kératiniques humaines telles que le cheveux.

Un autre objet de l'invention est une composition pour la teinture d'oxydation des fibres kératiniques, et en particulier des fibres kératiniques humaines telles que les cheveux, caractérisée par le fait qu'elle contient, dans un milieu approprié pour la teinture :
- au moins une base d'oxydation, et
- au moins un coupleur choisi parmi les composés de formule (I) telle que ci-dessus définie, ainsi que leurs sels d'addition avec un acide.

Le ou les composés de formule (I) conformes à l'invention et/ou le ou leurs sels d'addition avec un acide représentent de préférence de 0,0005 à 12 % en poids environ du poids total de la composition tinctoriale, et encore plus préférentiellement de 0,005 à 6 % en poids environ de ce poids.

Comme indiqué précédemment, la composition de teinture d'oxydation contenant le ou les composés de formule (I) conforme à l'invention permet d'obtenir des colorations puissantes dans des nuances allant du rouge au bleu et présentant de plus une ténacité remarquable aux différents traitements que peuvent subir les fibres kératiniques. Ces propriétés sont particulièrement remarquables notamment en ce qui concerne la résistance des colorations obtenues vis à vis de l'action de la lumière, des intempéries, des lavages, de l'ondulation permanente et de la transpiration.

La nature de la ou des bases d'oxydation pouvant être utilisées dans la composition tinctoriale conforme à l'invention n'est pas critique. Elles sont de préférence choisies parmi les bases d'oxydation classiquement utilisées en teinture d'oxydation et parmi lesquelles on peut notamment citer les paraphénylènediamines, les bis-phénylalkylènediamines, les para-aminophénols, les ortho-aminophénols et les bases hétérocycliques.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines citées ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques et les dérivés pyrazoliques.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demande de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Selon l'invention, les compositions tinctoriales renfermant une ou plusieurs paraphénylènediamines et/ou une ou plusieurs bases d'oxydation hétérocycliques sont particulièrement préférées.

La ou les bases d'oxydation représentent de préférence de 0,0005 à 12 % en poids environ du poids total de la composition tinctoriale, et encore plus préférentiellement de 0,005 à 6 % en poids environ de ce poids.

La composition tinctoriale conforme à l'invention peut également renfermer, en plus du ou des composés de formule (I) ci-dessus, un ou plusieurs coupleurs additionnels pouvant être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, les dérivés pyridiniques et les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

Lorsqu'ils sont présents ces coupleurs additionnels représentent de préférence de 0,0001 à 10 % en poids environ du poids total de la composition tinctoriale et encore plus préférentiellement de 0,005 à 5 % en poids environ de ce poids.

D'une manière générale, les sels d'addition avec un acide utilisables dans le cadre des compositions tinctoriales de l'invention (composés de formule (I), bases d'oxydation et coupleurs additionnels) sont notamment choisis parmi les chlorhydrates, les bromhydrates, les sulfates, les citrates, les succinates, les tartrates, les lactates et les acétates.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante : 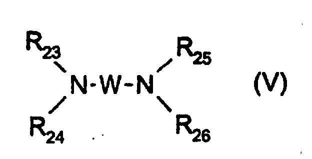 dans laquelle W est un reste propylène substitué ou non substitué par un groupement hydroxyle ou un radical alkyle en C₁-C₆ ; R₂₃, R₂₄, R₂₅ et R₂₆, identiques ou différents, représentent un atome d'hydrogène, un radical alkyle en C₁-C₆ ou hydroxyalkyle en C₁-C₆.

Les compositions de teinture d'oxydation conformes à l'invention peuvent également renfermer au moins un colorant direct, notamment pour modifier les nuances ou les enrichir en reflets.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture d'oxydation des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

Selon une forme de mise en oeuvre préférée du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, et les enzymes telles que les peroxydases, les laccases, les tyrosynases et les oxydo-réductases parmi lesquelles on peut en particulier mentionner les pyranose oxydases, les glucose oxydases, les glycérol oxydases, les lactates oxydases, les pyruvate oxydases, et les uricases.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention.

### EXEMPLES DE PREPARATION

### EXEMPLE DE PREPARATION 1 : Synthèse du chlorure de 3-[(1-hydroxynaphthalèn-2-ylcarbamoyl)-méthyl]-1-méthyl-3H-imidazol-1-ium.

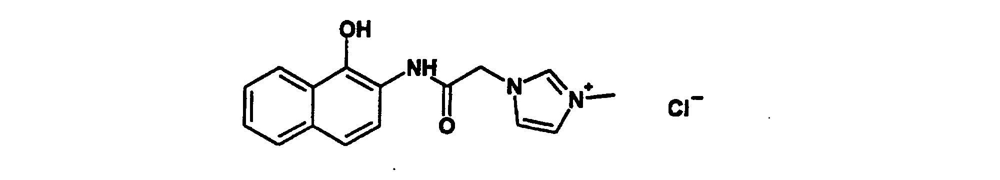

### a) Préparation du 2-chloro-N-(1-hydroxy-naphtalèn-2-yl)-acétamide

A une suspension de 5 g de chlorhydrate de 2-amino-1-naphtol (25,4 mmoles) et de 5,08 g de carbonate de calcium dans 200 ml de dioxane, sous agitation et sous atmosphère inerte, ont été ajoutés goutte à goutte, 2 ml de chlorure de chloroacétyle (25,4 mmoles). Le milieu réactionnel a été agité à 40°C pendant 1 heure 30 minutes, refroidi à 15°C, filtré sur verre fritté et les sels minéraux ont été rincés deux fois à l'acétate d'éthyle. Les phases organiques combinées ont été concentrées jusqu'à apparition d'un insoluble. Le concentrat a été versé sur 250 ml d'eau glacée. Le précipité formé a été essoré, lavé à l'eau et séché sous vide jusqu'à poids constant pour donner 3,58 g de 2-chloro-N-(1-hydroxy-naphtalèn-2-yl)-acétamide sous la forme d'une poudre beige avec un rendement de 60%.

### b) Préparation du chlorure de 3-[(1-hydroxy-naphthalèn-2-ylcarbamoyl)-méthyl]-1-méthyl-3H-imidazol-1-ium

Une solution de 2-chloro-N-(1-hydroxy-naphtalèn-2-yl)-acétamide obtenu ci-dessus à l'étape précédente (2g ; 8,4 mmoles) et de N-méthyl-imidazole (1,4 ml ; 17 mmoles) dans 30 ml de tétrahydrofurane a été chauffée au reflux pendant 5 heures. Le précipité formé a été essoré, lavé deux fois au tétrahydrofurane et séché sous vide jusqu'à poids constant pour donner 1,79 g de chlorure de 3-[(1-hydroxy-naphthalèn-2-ylcarbamoyl)-méthyl]-1-méthyl-3H-imidazol-1-ium sous la forme d'une poudre beige avec un rendement de 67%, et un point de fusion de 130°C.

L'analyse élémentaire calculée pour C₁₆H₁₆N₃O₂Cl était la suivante :

| % | C | H | N | O | Cl |
|---|---|---|---|---|---|
| Trouvé | 60,28 | 5,08 | 12,90 | 10,83 | 11,24 |
| Calculé | 60,48 | 5,08 | 13,22 | 10,07 | 11,16 |

### EXEMPLE DE PREPARATION 2: Synthèse du chlorure de 3-[(5-acétylamino-1-hydroxy-naphthalèn-2-ylcarbamoyl)-méthyl]-1-méthyl-3H-imidazol-1-ium.

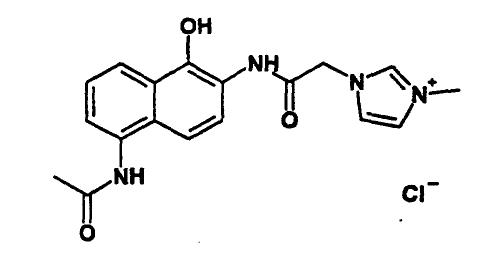

### a) Préparation du N-(5-hydroxy-naphthalèn-1-yl)-acétamide

A une solution de 5-amino-1-napthol (17,6 g ; 0,11 mole) dans 500 ml de dioxane, sous agitation et sous atmosphère inerte, ont été ajoutés 11 g de carbonate de calcium (0,11 mole) et, goutte à goutte, 8 ml de chlorure de chloroacétyle (0,112 mole). Le milieu réactionnel a été chauffé à 80°C pendant 2 heures 30 minutes. La suspension a été refroidie à température ambiante, filtrée sur verre fritté et les sels minéraux ont été rincés deux fois au dioxane. Le mélange des solutions organiques a été concentré jusqu'à l'apparition d'un insoluble, puis versé sur 400 ml d'eau glacée. Le précipité formé a été essoré, lavé une fois à l'eau et une fois au pentane. La poudre brune obtenue a été séchée sous vide jusqu'à poids constant pour donner 16,8 g de N-(5-hydroxynaphthalèn-1-yl)-acétamide avec un rendement de 76%.

### b) Préparation du N-(5-hydroxy-6-nitroso-naphthalèn-1-yl)-acétamide

Une solution marron de N-(5-hydroxy-naphthalèn-1-yl)-acétamide obtenu ci-dessus à l'étape précédente, (25 g, 0,124 mole) et de chlorure de zinc anhydre (25 g, 0,183 mole) dans 125 ml d'éthanol a été chauffée à 50°C. Une solution de nitrite de sodium (8,75 g, 0,127 mole) dans 17,5 ml d'eau a été introduite goutte à goutte dans la suspension. Le milieu réactionnel a été chauffé à 60°C pendant 2 heures, puis refroidi à 20°C et le précipité formé a été essoré, lavé trois fois à l'éthanol, puis versé par petites spatulées sur 50 ml d'acide chlorhydrique (35%) sous agitation à 15°C. Le milieu a été agité pendant 1 heure puis 50 ml d'eau à 2°C ont été ajoutés lentement. Le précipité a été essoré et lavé 4 fois à l'eau. La poudre verte obtenue a été séchée sous vide jusqu'à poids constant pour donner 24,4 g de N-(5-hydroxy-6-nitroso-naphthalèn-1-yl)-acétamide avec un rendement de 86%.

### c) Préparation du N-(5-hydroxy-6-amino-naphthalèn-1-yl)-acétamide

3 g de N-(5-hydroxy-6-nitroso-naphthalèn-1-yl)-acétamide obtenu ci-dessus à l'étape précédente (13 mmoles) ont été dissout dans 30 ml d'une solution aqueuse de soude à 10%. Le milieu réactionnel a été refroidi à 15°C et de l'hydrosulfite de sodium (7 g, 67 mmoles) a été ajouté par petites spatulées en conservant la température à 15-20°C. Le milieu réactionnel a été refroidi à 0°C, et le pH ajusté à pH=7,05 par 5,5 ml d'une solution 6N d'acide chlorhydrique. Le précipité a été essoré, lavé trois fois à l'eau et séché sous vide jusqu'à poids constant pour donner 1,7 g de N-(5-hydroxy-6-amino-naphthalèn-1-yl)-acétamide avec un rendement de 59%.

### d) Préparation du N-(5-acétylamino-1-hydroxy-naphthalèn-2-yl)-2-chloro-acétamide

Dans une suspension de 1,3 g de N-(5-hydroxy-6-amino-naphthalèn-1-yl)acétamide obtenu ci-dessus à l'étape c), (6 mmoles) et de 0,6 g de carbonate de calcium (6 mmoles) dans 50 ml de dioxane, sous agitation et sous atmosphère inerte, a été introduit 0,48 ml de chlorure de chloroacétyle (6 mmoles). Le milieu réactionnel a été agité pendant 3 heures à reflux, puis refroidi à 30°C. Le milieu réactionnel a été dilué par 400 ml d'eau, et extrait trois fois par 200 ml d'acétate d'éthyle. Les phases organiques combinées ont été séchées sur sulfate de sodium et concentrées à sec pour donner 0,67 g de N-(5-acétylamino-1-hydroxy-naphthalèn-2-yl)-2-chloro-acétamide sous la forme d'une poudre rose, avec un rendement de 38%.

### e) Préparation du chlorure de 3-[(1-hydroxy-5-acétylamino-naphthalèn-2-ylcarbamoyl)-méthyl]-1-méthyl-3H-imidazol-1-ium

Dans une solution de N-(5-acétylamino-1-hydroxy-naphthalèn-2-yl)-2-chloro-acétamide obtenu ci-dessus à l'étape d), (0,9 g, 3 mmoles) dans 20 ml de dioxane a été introduit 0,25 ml de N-méthylimidazole (3,1 mmoles). Le milieu réactionnel a été chauffé au reflux pendant 7 heures et 30 minutes. Le précipité formé a été essoré, lavé abondamment au dioxane, et séché sous vide jusqu'à poids constant pour donner 0,82g de chlorure de 3-[(1-hydroxy-5-acétylaminonaphthalèn-2-ylcarbamoyl)-méthyl]-1-méthyl-3H-imidazol-1-ium sous la forme d'une poudre rouge fondant à 168°C et avec un rendement de 73%. L'analyse en spectroscopie de masse cation : m/z = 338 du produit obtenu était conforme à celle du produit obtenu.

### EXEMPLE DE PREPARATION 3 : Synthèse du chlorure de 3-[(1-hydroxy-5-méthanesulfonylamino-naphthalèn-2-ylcarbamoyl)-méthyl]-1-méthyl-3H-imidazol-1-ium.

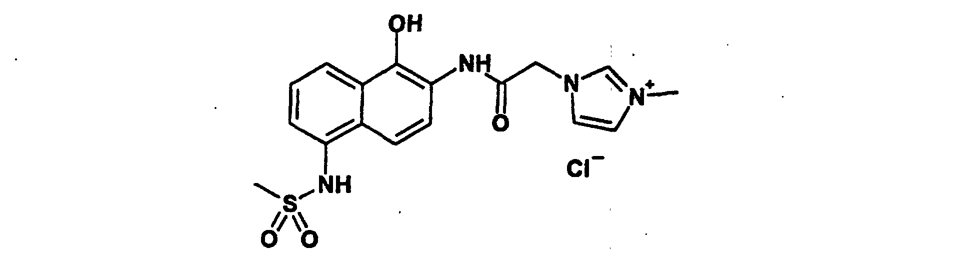

### a) Préparation du N-(5-méthanesulfonylamino-1-hydroxy-naphthalèn-2-yl)-2-chloro-acétamide

Dans une suspension de 1g de N-(5-hydroxy-6-amino-naphthalèn-1-yl)-méthanesulfonamide (4 mmoles, préparé selon le procédé décrit dans le brevet US 4,956,456) et de 0,4 g de carbonate de calcium (4 mmoles) dans 50 ml de dioxane, sous agitation et sous atmosphère inerte, a été introduit 0,32 ml de chlorure de chloroacétyle (4 mmoles). Le milieu réactionnel a été agité pendant 2 heures à 85°C, puis refroidi à 30°C. La suspension a été filtrée sur verre fritté, et les sels minéraux ont été rincés deux fois avec un minimum de dioxane. Les phases organiques combinées ont été versées sur 100 ml d'eau glacée. Le précipité formé a été essoré, lavé deux fois à l'eau et séché sous vide jusqu'à poids constant pour donner 1,12 g de N-(5-méthanesulfonylamino-1-hydroxy-naphthalèn-2-yl)-2-chloro-acétamide sous la forme d'une poudre rose, avec un rendement de 84%.

### b) Préparation du chlorure de 3-[(1-hydroxy-5-méthanesulfonylamino-naphthalèn-2-ylcarbamoyl)-méthyl]-1-méthyl-3H-imidazol-1-ium

Dans une solution de N-(5-méthanesulfonylamino-1-hydroxy-naphthalèn-2-yl)-2-chloro-acétamide obtenu ci-dessus à l'étape précédente (1 g, 3 mmoles) dans 20 ml de tétrahydrofurane a été introduit 0,25 ml de N-méthylimidazole (3,1 mmoles). Le milieu réactionnel a été chauffé au reflux pendant 8 heures. Le précipité formé a été essoré, lavé abondamment au dioxane, et séché sous vide jusqu'à poids constant pour donner 0,86 g de chlorure de 3-[(1-hydroxy-5-méthanesulfonylamino-naphthalèn-2-ylcarbamoyl)-méthyl]-1-méthyl-3H-imidazol-1-ium sous la forme poudre, brune fondant à 198°C, avec un rendement de 70%. L'analyse en spectroscopie de masse cation : m/z = 375 du produit obtenu était conforme à celle du produit attendu.

### EXEMPLES D'APPLICATION

### EXEMPLES DE TEINTURE 1 à 7 EN MILIEU ALCALIN

On a préparé les compositions tinctoriales conformes à l'invention suivantes, (teneurs en grammes) :

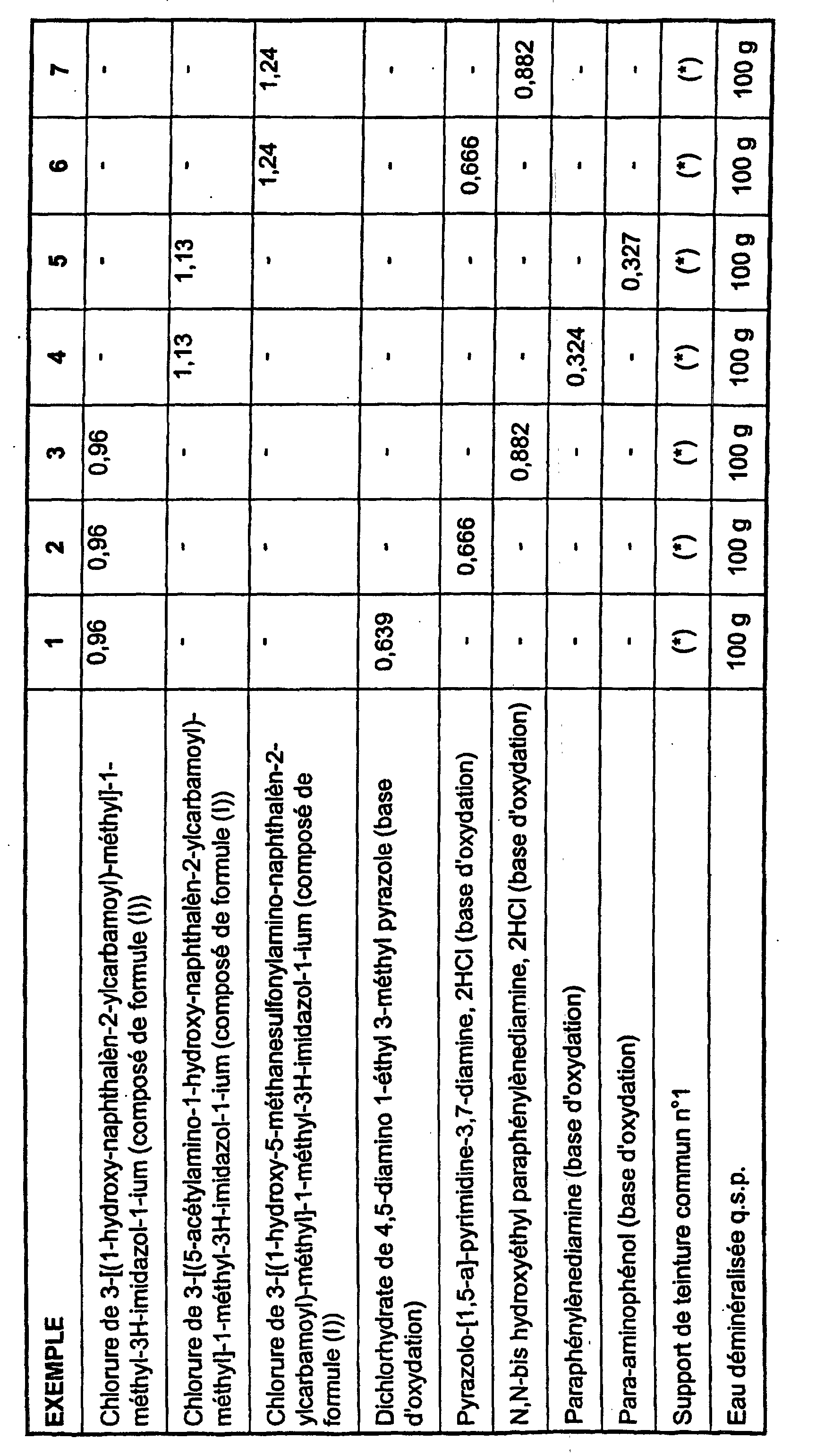

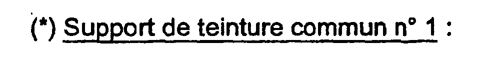

- Alcool benzylique 2,0 g
- Polyéthylène glycol à 6 moles d'oxyde d'éthylène 3,0 g
- Ethanol à 96° 20,0 g
- Alkyl (C₈-C₁₀) polyglucoside en solution aqueuse à 60 % de
   matière active (M.A.), tamponnée par du citrate d'ammonium,
   vendu sous la dénomination ORAMIX CG 110 ® par la
   société SEPPIC 6,0 g
- Ammoniaque à 20% de NH₃ 10,0 g
- Métabisulfite de sodium à 35% de matière active 0,228 g
- Sel pentasodique de l'acide diéthylènetriaminopentacétique 1,1 g

Au moment de l'emploi, on a mélangé poids pour poids chacune des compositions tinctoriales ci-dessus avec une solution de peroxyde d'hydrogène à 20 volumes (6 % en poids) de pH 3.

Le mélange obtenu a été appliqué sur des mèches de cheveux gris naturels à 90 % de blancs pendant 30 minutes. Les mèches ont ensuite été rincées, lavées avec un shampooing standard, rincées à nouveau puis séchées.

Les nuances obtenues figurent dans le tableau ci-après :

| **EXEMPLE** | **pH de teinture** | **Nuance obtenue** |
|---|---|---|
| **1** | 10 ± 0,2 | Châtain clair légèrement violet |
| **2** | 10 ± 0,2 | Châtain clair violine légèrement irisé |
| **3** | 10 ± 0,2 | Bleu |
| **4** | 10 ± 0,2 | Châtain cendré |
| **5** | 10 ± 0,2 | Blond foncé irisé cuivré |
| **6** | 10 ± 0,2 | Châtain violine légèrement irisé |
| **7** | 10 ± 0,2 | Bleu légèrement cendré |

## Revendications

1. Composé de formule (I) suivante, ainsi que ses sels d'addition avec un acide : 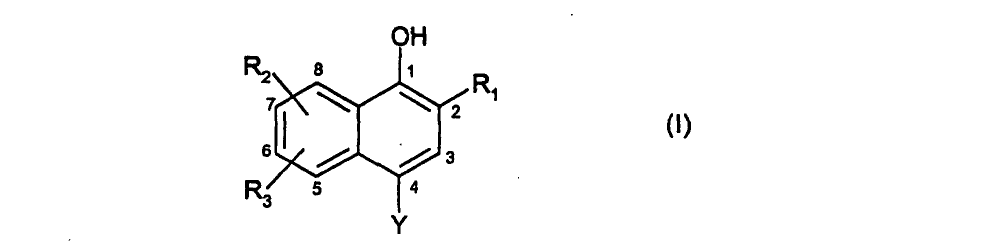 dans laquelle :
- R₁ représente un groupement choisi parmi -NR₄(C=O)R₅, -NR₆SO₂R₇ et -(C=O)NR₆R₈, les radicaux R_{4,} R₅ , R_{6,} R₇ et R₈ étant tels que définis ci-après ;
- R₂ et R₃, identiques ou différents, représentent un atome d'hydrogène; un atome d'halogène; un groupement Z tel que défini ci-après; un radical comportant de 1 à 20 atomes de carbone, linéaire ou ramifié (la ou les ramifications pouvant former un ou plusieurs cycles carbonés comportant de 3 à 7 chaînons), pouvant contenir une ou plusieurs liaisons doubles et/ou une ou plusieurs liaisons triples (lesdites liaisons doubles conduisant éventuellement à des groupements aromatiques), dont un ou plusieurs atomes de carbone peuvent être remplacés par un atome d'oxygène, d'azote, ou de soufre ou par un groupement SO₂, et dont les atomes de carbone peuvent, indépendamment les uns des autres, être substitués par un ou plusieurs atomes d'halogène ;
étant entendu que :
- lesdits radicaux R₂ et R₃ ne peuvent être reliés au cycle benzénique de la formule (I) par une liaison -NH-NH-; et que R₂ et R₃ ne comportent pas de liaison peroxyde ni de radicaux diazo, nitro et nitroso ;
- que R₂ et R₃ ne peuvent représenter un radical hydroxyle ou un radical thio ;
- que R₂ et R₃ ne peuvent représenter, simultanément, un radical amino, alkylamino, acylamino ou sulfonylamino ;
- Y représente un atome d'hydrogène ou d'halogène ; un groupement -OR₉, -SR₉, ou -NH-SO₂R₉ dans lesquels R₉ représente un radical alkyle en C₁-C₆, linéaire ou ramifié (la ou les ramifications pouvant alors former un ou plusieurs cycles comportant de 3 à 6 chaînons), substitué ou non substitué par un ou plusieurs radicaux choisis dans le groupe constitué par un atome d'halogène, un radical hydroxy, alcoxy en C₁-C₄, amino et aminoalkyl en C₁-C₄ ; un radical phényle, substitué ou non substitué par un ou deux radicaux choisis dans le groupe constitué par un radical alkyle en C₁-C₄, trifluorométhyle, carboxy, alcoxycarbonyle en C₁-C₄, halogène, hydroxy, alcoxy en C₁-C₄, amino et aminoalkyl en C₁-C₄ ; ou un radical benzyle ;
- R_{4,} R₆ et R₈, identiques ou différents, représentent un atome d'hydrogène; un groupement Z tel que défini ci-après; un radical comportant de 1 à 15 atomes de carbone, linéaire ou ramifié (la ou les ramifications pouvant former un ou plusieurs cycles carbonés comportant de 3 à 7 chaînons), pouvant contenir une ou plusieurs liaisons doubles et/ou une ou plusieurs liaisons triples (lesdites liaisons doubles conduisant éventuellement à des groupements aromatiques), dont un ou plusieurs atomes de carbone peuvent être remplacés par un atome d'oxygène, d'azote, ou de soufre ou par un groupement SO₂, et dont les atomes de carbone peuvent, indépendamment les uns des autres, être substitués par un ou plusieurs atomes d'halogène ;
étant entendu que ledit groupement SO₂ n'est pas directement lié à l'atome d'azote portant le radical R₄ ou R₆,
étant entendu que le groupement -(C=O)- n'est pas directement lié à l'atome d'azote portant le radical R₆;
étant entendu que lesdits radicaux R_{4,} R₆ et R₈ ne comportent pas de liaison peroxyde ni de radicaux diazo, nitro et nitroso;
étant entendu que les radicaux R_{4,} R₆ et R₈ ne peut représenter un radical hydroxy, un radical thio, un radical amino, un radical alcoxy, un radical alkylthio;
- R₅ et R₇, identiques ou différents, représentent un atome d'hydrogène ; un groupement Z tel que défini ci-après; un radical comportant de 1 à 20 atomes de carbone, linéaire ou ramifié (la ou les ramifications pouvant former un ou plusieurs cycles carbonés comportant de 3 à 7 chaînons), pouvant contenir une
ou plusieurs liaisons doubles et/ou une ou plusieurs liaisons triples (lesdites liaisons doubles conduisant éventuellement à des groupements aromatiques), dont un ou plusieurs atomes de carbone peuvent être remplacés par un atome d'oxygène, d'azote, ou de soufre ou par un groupement SO₂, et dont les atomes de carbone peuvent, indépendamment les uns des autres, être substitués par un ou plusieurs atomes d'halogène ;
étant entendu que lesdits radicaux R₅ et R₇ ne comportent pas de liaisons peroxydes ni de radicaux diazo, nitro et nitroso;
étant entendu que R₅ ne peut pas représenter un radical hydroxyle ou un radical thio ;
étant entendu que R₇ ne peut pas représenter un radical thio ;
étant entendu que les radicaux R₄ et R₅ d'une part, et les radicaux R₆ et R₈ d'autre part, peuvent en outre être reliés pour former, indépendamment les uns des autres, un cycle saturé ou insaturé comportant de 5 à 7 chaînons, constitué de carbone, d'azote et/ou d'acyle, chaque chaînon étant substitué ou non substitué par 1 ou 2 radicaux R, identiques ou différents, R étant un radical alkyle en C₁-C₈, linéaire ou ramifié (la ou les ramifications pouvant alors former un ou plusieurs cycles comportant de 3 à 7 chaînons), pouvant contenir une ou plusieurs liaisons doubles et/ou une ou plusieurs liaisons triples (lesdites liaisons doubles conduisant éventuellement à des groupements aromatiques), et dont un ou plusieurs atomes de carbone peuvent être remplacés par un atome d'oxygène, d'azote, ou de soufre ou par un groupement SO₂, et dont les atomes de carbone peuvent, indépendamment les uns des autres, être substitués par un ou plusieurs atomes d'halogène ; ledit cycle ne comportant pas de liaisons peroxydes ni de radicaux diazo, nitro et nitroso;
- Z est un groupement cationique représenté par la formule (II) suivante: 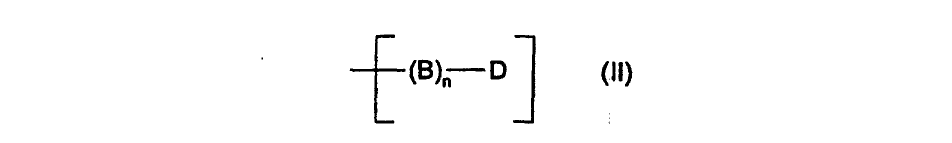
dans laquelle :
- n peut prendre la valeur 0 ou 1.
- B représente un radical alkyle comportant de 1 à 15 atomes de carbone, linéaire ou ramifié (la ou les ramifications pouvant alors former un ou plusieurs cycles comportant de 3 à 7 chaînons), pouvant contenir une ou plusieurs liaisons doubles et/ou une ou plusieurs liaisons triples, lesdites liaisons doubles conduisant éventuellement à des groupements aromatiques, et dont un ou plusieurs atomes de carbone peuvent être remplacés par un atome d'oxygène, d'azote, ou de soufre ou par un radical -SO₂; et dont un ou plusieurs atomes de carbone peuvent, indépendamment les uns des autres, être substitués par un ou plusieurs atomes d'halogène ou par un ou plusieurs groupements Z ; ledit radical B ne comportant pas de liaison peroxyde ni de radicaux diazo, nitro ou nitroso ;
- D est choisi parmi les groupements cationiques de formules (III) et (IV) suivantes : 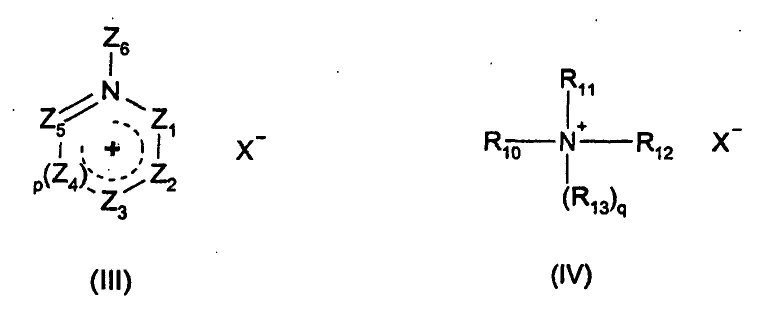
dans lesquelles :
- p et q peuvent, indépendamment l'un de l'autre, prendre la valeur 0 ou 1;
- le radical B est relié au groupement D par l'un quelconque des atomes du radical D ;
- lorsque n = 0 et q = 0, alors le groupement de formule (IV) peut être relié au composé de formule (I) directement par l'atome d'azote de l'ammonium quaternaire;
- Z₁, Z₂, Z₃ et Z₄, indépendamment les uns des autres, représentent un atome d'oxygène ; un atome de soufre ; un atome d'azote substitué ou non substitué par un radical R₁₄ ; un atome de carbone substitué ou non substitué par un ou deux radicaux R₁₄, identiques ou différents ;
- Z₅ représente un atome d'azote ou un atome de carbone substitué ou non substitué par un radical R₁₄ ;
- Z₆ peut prendre les mêmes significations que celles indiquées ci-dessous pour le radical R₁₄ ; étant entendu que Z₆ est différent d'un atome d'hydrogène; les radicaux Z₁ ou Z₅ peuvent, en outre, former avec Z₆ un cycle saturé ou insaturé comportant de 5 à 7 chaînons, chaque chaînon étant substitué ou non substitué par un ou deux radicaux R₁₄ identiques ou différents ;
- R₁₄ représente un atome d'hydrogène ; un groupement Z ; un radical comportant de 1 à 10 atomes de carbone, linéaire ou ramifié, pouvant contenir une ou plusieurs liaisons doubles et/ou une ou plusieurs liaisons triples, lesdites liaisons doubles pouvant alors éventuellement conduire à des groupes aromatiques, et dont un ou plusieurs atomes de carbone peuvent être remplacés par un atome d'oxygène, d'azote, ou de soufre, ou par un groupe SO₂, et dont un ou plusieurs atomes de carbone peuvent, indépendamment les uns des autres, être substitués par un ou plusieurs atomes d'halogène ; ledit radical ne comportant pas de liaison peroxyde ni de radicaux diazo, nitro et nitroso;
deux des radicaux adjacents Z₁, Z₂, Z₃, Z₄ et Z₅ pouvant en outre former un cycle comportant de 5 à 7 chaînons, chaque chaînon étant indépendamment représenté par un atome de carbone substitué ou non substitué par un ou deux radicaux R₁₄ identiques ou différents ; un atome d'azote substitué ou non substitué par un radical R₁₄ ; un atome d'oxygène ; ou un atome de soufre ;
- R₁₀, R₁₁, R₁₂ et R₁₃, identiques ou différents, ont les mêmes significations que celles indiquées ci-dessus pour le radical R₁₄ ;
les radicaux R₁₀, R₁₁, R₁₂ et R₁₃ pouvant également former, deux à deux avec l'atome d'azote quaternaire auquel ils sont rattachés, un ou plusieurs cycles saturés comportant de 5 à 7 chaînons, chaque chaînon étant indépendamment représenté par un atome de carbone substitué ou non substitué par un ou deux radicaux R₁₄ identiques ou différents ; un atome d'azote substitué ou non substitué par un radical R₁₄ ; un atome d'oxygène ; ou un atome de soufre ;
- X⁻ représente un anion organique ou minéral.
étant entendu qu'au moins un des groupements R₁ à R₃ représente ou contient un groupement Z.

2. Composé selon la revendication 1, **caractérisé par le fait que** R₂ et R₃ représentent, indépendamment l'un de l'autre, un atome d'hydrogène ou de chlore ; un groupement Z ; un radical méthyle ou méthoxy; un radical amino, méthylamino, 2-hydroxyéthylamino ; un groupement -NH(CO)R₁₅ dans lequel R₁₅ représente un des radicaux listés dans le groupe **(G1)** constitué par un radical méthyle, éthyle, propyle, isopropyle, butyle, isobutyle, tert-butyle, pentyle, isopentyle, néopentyle, hexyle, cyclopropyle, cyclobutyle, cyclopentyle, cyclopentylméthyle, 3-cyclopentyl-propyle, cyclohexyle, 2-cyclohexyl-éthyle, norbomane-2-yl, vinyle, 1-méthylvinyle, 2-méthylvinyle, 2,2-diméthylvinyle, allyle, 3-butenyle, phényle, méthylphényle, diméthylphényle, 2,4,6-triméthylphényle, 4-éthylphényle, (trifluorométhyl)phényle, hydroxyphényle, méthoxyphényle, éthoxyphényle, acétoxyphényle, (trifluorométhoxy)phényle, aminophényle, 4-diméthylaminophényle, fluorophényle, difluorophényle, fluoro(trifluorométhyl)phényle, chlorophényle, dichlorophényle, bromophényle, napht-1-yle, napht-2-yle, (2-méthoxy)napht-1-yl, benzyle, 4'-méthoxybenzyle, 2',5'-diméthoxybenzyle, 3',4'-diméthoxybenzyle, 4'-fluorobenzyle, 4'-chlorobenzyle, phénéthyle, 2-phénylvinyle, (1-naphtyl)méthyle, (2-naphtyl)méthyle ; tétrahydrofuran-2-yl, furan-2-yl, 5-méthyl-2-(trifluorométhyl)furan-3-yl, 2-méthyl-5-phénylfuran-3-yl, thiophène-2-yl, (thiophène-2-yl)méthyle, 3-chlorothiophène-2-yl, 2,5-dlchlorothiophène-3-yl, benzothiophène-2-yl, 3-chlorobenzothiophène-2-yl, isoxazole-5-yl, 5-méthylisoxazole-3-yl, 3,5-diméthylisoxazole-4-yl, 1,3-diméthylpyrazole-5-yl, 1-éthyl-3-méthylpyrazole-5-yl, 1-tertbutyl-3-méthylpyrazole-5-yl, 3-tertbutyl-1-méthylpyrazole-5-yl, 4-bromo-1-éthyl-3-méthylpyrazole-5-yl, pyridinyl, chloropyridinyl, dichloropyridinyl, 5-(bromo)pyridin-3-yl, piperazin-2-yl, 2-chloro-4-(trifluorométhyl)pyperimidine-5-yl, quinoxal-2-yl, benzofurazan-5-yl; fluorométhyle, difluorométhyle, trifluorométhyle, 1,1,2,2-tétrafluoroéthyle, pentafluoroéthyle, heptafluoropropyle, 1,1,2,2,3,3,4,4-octafluorobutyle, nonafluorobutyle, chlorométhyle, chloroéthyle, 1,1-diméthyl-2-chloroéthyle, 1,2-dichloroéthyle, 1-chloropropyle, 3-chloropropyle, 4-chlorobutyle, hydroxyméthyle, méthoxyméthyle, phénoxyméthyle, (4-chlorophénoxy)méthyle, benzyloxyméthyle, acétoxyméthyle, 1,2-dihydroxyéthyle, 1-phénoxyéthyle, 1-acétoxyéthyle, 2-(2-carboxyéthoxy)éthyle, 1-phénoxyéthyle, 1-acétoxyéthyle, (méthoxycarbonyl)méthyle, 2-carboxyéthyle, 2-(méthoxycarbonyl)éthyle, 2-carboxycylopropyle, 2-carboxycyclohexane, méthoxy, éthoxy, propoxy, isopropoxy, butoxy, isobutoxy, pentoxy, néopentoxy, hexyloxy, cyclopentyloxy, cyclohexyloxy, vinyloxy, allyloxy, propargyloxy, chlorométhoxy, 1-chloroéthoxy, 2-méthoxyéthoxy, 4-chlorobutoxy, phénoxy, 4-méthyphénoxy, 4-fluorophénoxy, 4-bromophénoxy, 4-chlorophénoxy, 4-méthoxyphénoxy, naphth-2-yloxy, benzyloxy ; amino, méthylamino, éthylamino, propylamino, isopropylamino, butylamino, cyclohexylamino, allylamino, 2-chloroéthylamino, 3-chloropropylamino, carboxyméthylamino, phénylamino, fluorophénylamino, (trifluorométhyl)phénylamino, chlorophénylamino, bromophénylamino, 4-acétylphénylamino, méthoxyphénylamino, (trifluorométhoxy)phénylamino, naphth-1-ylamino, benzylamino, phénéthylamino, pyrid-3-ylamino, diméthylamino, 1-pyrolidinyle, et 4-morpholinyle ; ou un groupement -NHSO₂R₁₆ dans lequel R₁₆ représente un des radicaux listés dans le groupe **(G2)** constitué par les radicaux méthyle, trifluorométhyle, éthyle, 2-chloroéthyle, propyle, 3-chloropropyle, isopropyle, butyle, thiophène-2-yl, hydroxy, éthoxy, et amino, diméthylamino.

3. Composé selon la revendication 2, **caractérisé par le fait que** R₂ et R₃, indépendamment l'un de l'autre, un atome d'hydrogène ; un groupement -O-E-D', -NH-E-D', -NH(CO)-D', -NH(CO)-E-D', -NH(CO)O-E-D', -NH(CO)NH-E-D' ou -NH(SO₂)-E-D', dans lesquels -E- représente un bras -(CH₂)_{q}-, q étant un nombre entier égal à 1 ou 2, et dans lesquels D' représente les groupements 3-méthylimidazolidinium-1-yl, 3-(2-hydroxyéthyl)-imidazolidinium-1-yl, 1,2,4-triazolinium-1-yl, 1,2,4-triazolinium-4-yl, N-alkyl(C1-C4)pyridinium-2-yl, N-alkyl(C₁-C₄)pyridinium-3-yl, N-alkyl(C₁-C₄)pyridinium-4-yl, N-(2-hydroxyéthyl) pyridinium-2-yl, N-(2-hydroxyéthyl)pyridinium-3-yl, N-(2-hydroxyéthyl)pyridinium-4-yl, pyridinium-1-yl, trialkyl(C1-C4)ammonium-N-yl, 1-méthylpipéridinium-1-yl ou 1,4-dimétylpipérazinium-1-yl ; un radical méthyle, méthoxy, amino, ou méthylamino ; ou un groupement -NH(CO)R₁₇ dans lequel R₁₇ représente un des radicaux listés dans le groupe **(G2)** tel que défini à la revendication 2 ; ou un groupement méthanesulfonylamino, éthanesulfonylamino ou diméthylaminosulfonylamino.

4. Composé selon l'une quelconque des revendications précédentes, **caractérisé par le fait que** R₄ représente un atome d'hydrogène ou un groupement A₁ constitué par un radical alkyle en C₁-C₈, linéaire ou ramifié, pouvant porter une ou deux doubles liaisons ou une triple liaison, être substitué ou non substitué par un groupement choisi parmi un groupement A₂, un groupement A₄, un groupement A₅ tels que définis ci-après, être substitué ou non substitué par un ou deux groupements, identiques ou différents, choisis parmi les groupements N-alkyl(C₁-C₃)amino, N-alkyl(C₁-C₃)-N-alkyl(C₁-C₃)amino, alkoxy(C₁-C₆), oxo, alcoxycarbonyle, acyloxy, amide, acylamino, uréyle, sulfoxy, sulfonyle, sulfonamido, sulfonylamino, bromo, cyano, carboxy, et être substitué ou non substitué par un ou plusieurs groupements hydroxyle, fluoro ou chloro ; un groupement A₂ constitué par un groupement aromatique de type phényle ou naphtyle, pouvant être substitué ou non substitué par un à trois groupements, identiques ou différents, choisis parmi les groupements méthyle, trifluorométhyle, éthyle, isopropyle, butyle, pentyle, fluoro, chloro, bromo, méthoxy, trifluorométhoxy, éthoxy, propyloxy, acétyloxy, acétyle et cyano ; un groupement A₃ constitué par un groupement hétéroaromatique choisi parmi les groupements furanyle, thiophényle, pyrrolyle, imidazolyle, thiazolyle, oxazolyle, 1,2,3-triazolyle, 1,2,4-triazolyle, isoxazolyle, isothiazolyle, pyrazolyle, pyrazoltriazolyle, pyrazoloimidazolyle, pyrrolotriazolyle, pyrazolopyrimidyle, pyrazolopyridyle, pyridyle, pyrimidyle, benzoimidazolyle, benzoxazolyle, benzothiazolyle, indolyle, indolidinyle, isoindolyle, indazolyle, benzotriazolyle, quinolinyle, benzoimidazolyle, benzopyrimidyle, lesdits groupements hétéroaromatiques étant substitués ou non substitués par 1 à 3 radicaux choisis parmi les radicaux alkyle en C₁-C₄, linéaire ou ramifié, monohydroxyalkyle en C₁-C₄, polyhydroxyalkyle en C₂-C₄, carboxy, alkoxycarbonyle, halogène, amido, amino et hydroxy ; un groupement A₄ constitué par un radical cycloalkyle en C₃-C₇, un radical norbomanyle, portant ou non une double liaison et substitué ou non substitué par 1 ou 2 radicaux choisis parmi les radicaux alkyle en C₁-C₄, linéaire ou ramifié, monohydroxyalkyle en C₁-C₄, polyhydroxyalkyle en C₂-C₄, carboxy, alkoxycarbonyle, halogène, amido, amino et hydroxy ; ou un groupement A₅ constitué par un hétérocycle choisi parmi les cycles dihydrofuranyle, tétrahydrofuranyle, butyrolact-one-yle, dihydrothiophényle, tétrahydrothiophényle, tétrahydrothiophén-one-yle, iminothiolane, dihydropyrrolyle, pyrrolidinyle, pyrrolidin-one-yle, imidazolidin-one-yle, imidazolidinthione-yle, oxazolidinyle, oxazolidin-one-yle, oxazolanethione, thiazolidinyle, isothiazol-one-yle, mercaptothiazolinyle, pyrazolidin-one-yle, iminothiolane, dioxolanyle, pentalactone, dioxanyle, dihydropyridinyle, pipéridinyle, pentalactame, morpholinyle, pyrazoli(di)nyle, pyrimi(di)nyl, pyrazinyle, pipérazinyle et azépinyl ; lesdits groupement A₁, A₂, A₃, A₄ et/ou A₅ éventuellement séparés de l'azote du groupement -NR₄(C=O)R₅ par un groupement -(CO)-.

5. Composé selon la revendication 4, **caractérisé par le fait que** R₄ représente un atome d'hydrogène; un radical méthyle, éthyle, isopropyle, allyle, phényle, benzyle, fluorobenzyle, hydroxybenzyle, difluorobenzyle, trifluorobenzyle, chlorobenzyle, bromobenzyle, méthoxybenzyle, diméthoxybenzyle, (trifluorométhoxy)benzyle, 3,4-méthylèndioxybenzyle, 6-chloropipéronyle, 4-méthylthiobenzyle, 4-méthylsulfonylbenzyle, 4-acétylaminobenzyle, 4-carboxybenzyle, 1-naphtométhyle, 2-naphtométhyl; ou un groupement 2-hydroxéthyle, 2-méthoxyéthyle ou 2-éthoxyéthyle.

6. Composé selon l'une quelconque des revendications précédentes, **caractérisé par le fait que** R₅ désigne un atome d'hydrogène, un groupement amino ; un groupement Z ; un groupement A₁, A₂, A₃, A₄ ou A₅ tels que définis à la revendication 4, éventuellement séparés du carbone (en position 2) de la fonction amide du composé de formule (I) par un groupement -O-, -NH, -NAlkyl(C₁-C₃)-, -(CO)-.

7. Composé selon la revendication 6, **caractérisé par le fait que** R₅ représente un groupement Z; un radical choisi dans le groupe **(G3)** constitué par un radical méthyle, éthyle, propyle, allyle, phényle, tétrahydrofuran-2-yl, furan-2-yl, thiophène-2-yl, pyridinyle, piperazin-2-yl, fluorométhyle, chlorométhyle, 2-chloroéthyle, méthoxyméthyle, acétoxyméthyle, 1,2-dihydroxyéthyle, méthoxycarbonyl, 2-carboxyéthyle, méthoxy, éthoxy, propoxy, allyloxy, 2-chloroéthoxy, 2-méthoxyéthoxy, amino, éthylamino, allylamino, 2-chloroéthylamino, pyridylamino, diméthylamino, 1-pyrolidinyle, et 4-morpholinyle.

8. Composé selon la revendication 7, **caractérisé par le fait que** R₅ représente un groupement -D', -E-D', -O-E-D' ou -NH-E-D' dans lesquels -E- représente un bras -(CH₂)_{q}-, q étant un nombre entier égal à 1 ou 2, et D' un groupement tel que défini à la revendication 3 ; ou un radical choisi dans le groupe **(G4)** constitué par un radical méthyle, méthoxyméthyle, 2-carboxyéthyle, méthoxy, amino, éthylamino et 1-pyrolidinyle.

9. Composé selon l'une quelconque des revendications précédentes, **caractérisé par le fait que** R₄ et R₅ forment un cycle, ledit cycle étant choisi parmi les groupements 2-pyrrolidinon-1-yl, méthyl-2-pyrrolidinon-1-yl, 5-carboxy-2-pyrrolidinon-1-yl, 5-méthoxycarbonyl-2-pyrrolidinone-1-yl, pyrazolinone-1-yl, succinimide-1-yl, 3,5-dicétopyrazolidin-1-yl, oxindolin-1-yl, maléimide-1yl, isoindole-1,3-dione-2-yl, 2-pipéridinone-1-yl et glutarimide-1-yl.

10. Composé selon l'une quelconque des revendications précédentes, **caractérisé par le fait que** R₆ représente un atome d'hydrogène ou un groupement A₁, A₂, A₃, A₄ ou A₅ tels que définis à la revendication 4.

11. Composé selon la revendication 10, **caractérisé par le fait que** R₆ représente un atome d'hydrogène; un radical méthyle, éthyle, isopropyle, allyle; phényle, benzyle, fluorobenzyle, hydroxybenzyle, difluorobenzyle, trifluorobenzyle, chlorobenzyle, bromobenzyle, méthoxybenzyle, diméthoxybenzyle, (trifluorométhoxy)benzyle, 3,4-méthylèndioxybenzyle, 6-chloropipéronyle, 4-méthylthiobenzyle, 4-méthylsulfonylbenzyle, 4-acétylaminobenzyle, 4-carboxybenzyle, 1-naphtométhyle, 2-naphtométhyle ; un groupement 2-hydroxéthyle, 2-méthoxyéthyle ou 2-éthoxyéthyle.

12. Composé selon l'une quelconque des revendications précédentes, **caractérisé par le fait que** R₈ désigne un groupement Z ; un atome d'hydrogène ou un groupement A₁, A₂, A₃, A₄ ou A₅ tels que définis à la revendication 4.

13. Composé selon la revendication 12, **caractérisé par le fait que** R₈ représente un groupement Z; un atome d'hydrogène; un radical méthyle, éthyle, isopropyle, allyle; phényle, benzyle, fluorobenzyle, hydroxybenzyle, difluorobenzyle, trifluorobenzyle, chlorobenzyle, bromobenzyle, méthoxybenzyle, diméthoxybenzyle, (trifluorométhoxy)benzyle, 3,4-méthylèndioxybenzyle, 6-chloropipéronyle, 4-méthylthiobenzyle, 4-méthylsulfonylbenzyle, 4-acétylaminobenzyle, 4-carboxybenzyle, 1-naphtométhyle ou 2-naphtométhyle; un groupement 2-hydroxéthyle, 2-méthoxyéthyle ou 2-éthoxyéthyle.

14. Composé selon la revendication 13, **caractérisé par le fait que** R₈ représente un atome d'hydrogène ou un radical méthyle ;.un groupement -D', -E-D', dans lesquels -E- représente un bras -(CH₂)_{q}-, q étant un nombre entier égal à 1 ou 2, et D' un groupement tel que défini à la revendication 3.

15. Composé selon l'une quelconque des revendications précédentes, **caractérisé par le fait que** R₆ et R₈ forment un cycle choisi parmi les groupements pyrolidinyl, pipéridinyl, morpholinyl, pyrazolyl, isoxazolyl, imidazolyl, thiazolyl, indolyl, indolidinyl, indazolyl, pyrazolotriazolyl, pyrrolotriazolyl, pyrazoloimidazolyl, pyrazolidinyl, thiomorpholinyl, thiazolyl et pyrazinyl.

16. Composé selon l'une quelconque des revendications précédentes, **caractérisé par le fait que** R₇ désigne un groupement amino; un groupement Z ; un groupement A₁, A₂, A₃, A₄ ou A₅ tels que définis à la revendication 4, éventuellement séparés du soufre du groupe sufonyl du composé de formule (I) par un groupement -NH, ou NAlkyl(C₁-C₃)-.

17. Composé selon la revendication 16, **caractérisé par le fait que** R₇ représente un groupement Z, un des radicaux listés dans le groupe **(G4)** constitué par les radicaux méthyle, trifluorométhyle, éthyle, 2-chloroéthyle, propyle, 3-chloropropyle, isopropyle, butyle, thiophène-2-yl, hydroxy, éthoxy, amino et diméthylamino.

18. Composé selon la revendication 17, **caractérisé par le fait que** R₇ représente un groupement -D', -E-D' ou -NH-E-D', dans lesquels -E- représente un bras -(CH₂)_{q}-, q étant un nombre entier égal à 1 ou 2, et D' un groupement tel que défini à la revendication 3 ; ou un radical méthyle, éthyle ou diméthylamino.

19. Composé selon l'une quelconque des revendications précédentes, **caractérisé par le fait que** Y représente de préférence un radical choisi dans le groupe comprenant : un atome d'hydrogène, de chlore, de fluor, de brome ; les groupements méthoxy, éthoxy, propoxy, benzyloxy, phénoxy ; -OCH₂CH₂OMe; -OCH₂CH₂OMe; -OCH₂CH₂NMe₂; -OCH₂(CO)OH, -OCH₂(CO)OMe, -OCH₂(CO)OEt, -SCH₂CH₂CO₂H et NHSO₂Me.

20. Composé selon la revendication 19, **caractérisé par le fait que** Y représente un radical choisi dans le groupe constitué par un atome d'hydrogène, de chlore, le groupement méthoxy, -OCH₂(CO)OH, -OCH₂(CO)OMe.

21. Composé selon l'une quelconque des revendications précédentes, **caractérisé par le fait que** D est choisi parmi les groupements imidazolinium, thiazolinium, oxazolinium, pyrrolinium, 1,2,3-triazolinium, 1,2,4-triazolinium, isoxazolinium, isothiazolinium, imidazolidinium, thiazolidinium, pyrazolinium, pyrazolidinium, oxazolidinium, pyrazoltriazolinium, pyrazoloimidazolinium, pyrrolotriazolinium, pyrazolopyrimidinium, pyrazolopyridinium, pyridinium, pyrimidinium, pyrazinium, triazinium, benzoimidazolinium, benzoxazolinium, benzothiazolinium, indolinium, indolidinium, isoindolinium, indazolinium, benzotriazolinium, quinolinium, tetrahydroquinolinium, benzoimidazolidinium, benzopyrimidinium, tétra-alkyl(C₁-C₄)ammonium, dialkylpipéridinium, dialkylpyrrolidinium, dialkylmorpholinium, dialkylthiomorpholinium, dialkylpipérazinium, azépinium, et 1,4-diazabicyclo[2,2,2]octanium.

22. Composé selon la revendication 21, **caractérisé par le fait que** D est choisi parmi les groupements 3-méthylimidazolidinium-1-yl, 3-(2-hydroxyéthyl)-imidazolidinium-1-yl, 1,2,4-triazolinium-1-yl, 1,2,4-triazolinium-4-yl, N-alkyl(C₁-C₄)pyridinium-2-yl, N-alkyl(C₁-C₄)pyridinium-3-yl, N-alkyl(C₁-C₄)pyridinium-4-yl, N-(2-hydroxyéthyl) pyridinium-2-yl, N-(2-hydroxyéthyl)pyridinium-3-yl, N-(2-hydroxyéthyl) pyridinium-4-yl, pyridinium-1-yl, trialkyl(C₁-C₄)ammonium-N-yl, 1-méthylpipéridinium-1-yl et 1,4-dimétylpipérazinium-1-yl.

23. Composé selon l'une quelconque des revendications précédentes, **caractérisé par le fait qu'**il est choisi parmi ceux dans lesquels :
i) - le radical R₁ représente un radical choisi dans le groupe **(G5)** constitué par un radical acétylamino, méthanesulfonylamino, amido, ou méthylamido, un groupement -NH-C(O)-CH₂-D1, un groupement -NH-SO₂-CH₂-CH₂-D1_{,} un groupement -(CO)-NH-CH₂-CH₂-D1_{,} dans lesquels **D1** représente un groupement 3-méthylimidazolidinium-1-yl, triazolinium-1-yl, pyridinium-1-yl ou triméthylammonium-N-yl;
- le radical R₂ est en position 5 et représente un radical choisi dans le groupe **(G6)** constitué par un radical amino, méthylamino, acétylamino, méthanesulfonylamino, un groupement -NH-[C(O)]ₘ-CH₂-D1 dans lequel m représente 0 ou 1, ou un groupement -NH-SO₂-CH₂-CH₂-D1,
- le radical R₃ représente un atome d'hydrogène;
- le radical Y représente un radical choisi dans le groupe **(G7)** constitué par un atome d'hydrogène, un radical chlore et un radical méthoxy.
ou
ii) - le radical R₁ représente un radical choisi dans le groupe **(G5)** tel que défini ci-dessus;
- le radical R₂ est situé en position 8 et représente un radical choisi dans le groupe **(G6)** tel que défini ci-dessus;
- le radical R₃ représente un atome d'hydrogène;
- le radical Y représente un radical choisi dans le groupe **(G7)** tel que défini ci-dessus.
ou
iii) - le radical R₁ représente un radical choisi dans le groupe **(G5)** tel que défini ci-dessus ;
- les radicaux R₂ et R₃ représentent un atome d'hydrogène;
- le radical Y représente un radical choisi dans le groupe **(G7)** tel que défini ci-dessus.

24. Composé selon l'une quelconque des revendications précédentes, **caractérisés par le fait qu'**ils sont choisis parmi :
- le chlorure de 3-[(1-hydroxy-naphthalèn-2-ylcarbamoyl)-méthyl]-1-méthyl-3H-imidazol-1-ium ;
- le dichlorure de 3-[(1-hydroxy-5-(2-(3-méthyl-1H-imidazol-3-ium-1-yl)acétyl)-amino-naphthalèn-2-ylcarbamoyl)-méthyl]-1-méthyl-3H-imidazol-1-ium ;
- le dichlorure de 3-[(1-hydroxy-8-(2-(3-méthyl-1H-imidazol-3-ium-1-yl)acétyl)-amino-naphthalèn-2-ylcarbamoyl)-méthyl]-1-méthyl-3H-imidazol-1-ium ;
- le chlorure de 3-[(1-hydroxy-5-amino-naphthalèn-2-ylcarbamoyl)-méthyl]-1-méthyl-3H-imidazol-1-ium ;
- le chlorure de 3-[(1-hydroxy-5-acétylamino-naphthalèn-2-ylcarbamoyl)-méthyl]-1-méthyl-3H-imidazol-1-ium ;
- le chlorure de 3-[(1-hydroxy-5-méthoxycarbonylamino-naphthalèn-2-yl carbamoyl)-méthyl]-1-méthyl-3H-imidazol-1-ium ;
- le chlorure de 3-[(1-hydroxy-2-acétylamino-naphthalèn-5-ylcarbamoyl)-méthyl]-1-méthyl-3H-imidazol-1-ium ;
- le chlorure de 3-[(1-hydroxy-2-méthoxycarbonylamino-naphthalèn-5-ylcarbamoyl)-méthyl]-1-méthyl-3H-imidazol-1-ium ;
- le chlorure de 3-[(1-hydroxy-5-méthanesulfonylamino-naphthalèn-2-ylcarbamoyl)-méthyl]-1-méthyl-3H-imidazol-1-ium ;
- le chlorure de 3-[(1-hydroxy-8-amino-naphthalèn-2-ylcarbamoyl)-méthyl]-1-méthyl-3H-imidazol-1-ium;
- le chlorure de 3-[(1-hydroxy-8-acétylamino-naphthalèn-2-ylcarbamoyl)-méthyl]-1-méthyl-3H-imidazol-1-ium ;
- le chlorure de 3-[(1-hydroxy-8-méthoxycarbonylamino-naphthalèn-2-ylcarbamoyl)-méthyl]-1-méthyl-3H-imidazol-1-ium ;
- le chlorure de 3-[(1-hydroxy-2-acétylamino-naphthalèn-8-ylcarbamoyl)-méthyl]-1-méthyl-3H-imidazol-1-ium ;
- le chlorure de 3-[(1-hydroxy-2-méthoxycarbonylamino-naphthalèn-8-ylcarbamoyl)-méthyl]-1-méthyl-3H-imidazol-1-ium ;
- le chlorure de 3-[(1-hydroxy-8-méthanesulfonylamino-naphthalèn-2-ylcarbamoyl)-méthyl]-1-méthyl-3H-imidazol-1-ium ;
- le chlorure de 1-[(1-hydroxy-naphthalèn-2-ylcarbamoyl)-méthyl]-pyridinium ;
- le dichlorure de 1-[(1-hydroxy-5-(2-(pyridinium-1-yl)acétyl)amino-naphthalèn-2-ylcarbamoyl)-méthyl]-pyridinium ;
- le dichlorure de 1-[(1-hydroxy-8-(2-(pyridinium-1-yl)acétyl)amino-naphthalèn-2-ylcarbamoyl)-méthyl]-pyridinium ;
- le chlorure de 1-[(1-hydroxy-5-amino-naphthalèn-2-ylcarbamoyl)-méthyl]-pyridinium ;
- le chlorure de 1-[(1-hydroxy-5-acétylamino-naphthalèn-2-ylcarbamoyl)-méthyl]-pyridinium ;
- le chlorure de 1-[(1-hydroxy-5-méthoxycarbonylamino-naphthalèn-2-ylcarbamoyl)-méthyl]-pyridinium ;
- le chlorure de 1-[(1-hydroxy-2-acétylamino-naphthalèn-5-ylcarbamoyl)-méthyl]-pyridinium ;
- le chlorure de 1-[(1-hydroxy-2-méthoxycarbonylamino-naphthalèn-5-ylcarbamoyl)-méthyl]-pyridinium ;
- le chlorure de 1-[(1-hydroxy-5-méthanesulfonylamino-naphthalèn-2-ylcarbamoyl)-méthyl]-pyridinium ;
- le chlorure de 1-[(1-hydroxy-8-amino-naphthalèn-2-ylcarbamoyl)-méthyl]-pyridinium ;
- le chlorure de 1-[(1-hydroxy-8-acétylamino-naphthalèn-2-ylcarbamoyl)-méthyl]-pyridinium ;
- le chlorure de 1-[(1-hydroxy-8-méthoxycarbonylamino-naphthalèn-2-ylcarbamoyl)-méthyl]-pyridinium ;
- le chlorure de 1-[(1-hydroxy-2-acétylamino-naphthalèn-8-ylcarbamoyl)-méthyl]-pyridinium ;
- le chlorure de 1-[(1-hydroxy-2-méthoxycarbonylamino-naphthalèn-8-ylcarbamoyl)-méthyl]-pyridinium ;
- le chlorure de 1-[(1-hydroxy-8-méthanesulfonylamino-naphthalèn-2-ylcarbamoyl)-méthyl]-pyridinium ;
- le chlorure de 1-[(1-hydroxy-naphthalèn-2-ylcarbamoyl)-méthyl]-1,4-diméthylpipérazin-1-ium ;
- le dichlorure de 1-[(1-hydroxy-5-(2-(1,4-diméthyl-pipérazin-1-ium-1-yl)acétyl)-amino-naphthalèn-2-ylcarbamoyl)-méthyl]-1,4-diméthyl-pipérazin-1-ium ;
- le dichlorure de 1-[(1-hydroxy-8-(2-(1,4-diméthyl-pipérazin-1-ium-1-yl)acétyl)-amino-naphthalèn-2-ylcarbamoyl)-méthyl]-1,4-diméthyl-pipérazin-1-ium ;
- le chlorure de 1-[(1-hydroxy-5-amino-naphthalèn-2-ylcarbamoyl)-méthyl]-1,4--diméthyl-pipérazin-1-ium ;
- le chlorure de 1-[(1-hydroxy-5-acétylamino-naphthalèn-2-ylcarbamoyl)-méthyl]-1,4-diméthyl-pipérazin-1-ium ;
- le chlorure de 1-[(1-hydroxy-5-méthoxycarbonylamino-naphthalèn-2-ylcarbamoyl)-méthyl]-1,4-diméthyl-pipérazin-1-ium ;
- le chlorure de 1-[(1-hydroxy-2-acétylamino-naphthalèn-5-ylcarbamoyl)-méthyl]-1,4-diméthyl-pipérazin-1-ium ;
- le chlorure de 1-[(1-hydroxy-2-méthoxycarbonylamino-naphthalèn-5-ylcarbamoyl)-méthyl]-1,4-diméthyl-pipérazin-1-ium ;
- le chlorure de 1-[(1-hydroxy-5-méthanesulfonylamino-naphthalèn-2-ylcarbamoyl)-méthyl]-1,4-diméthyl-pipérazin-1-ium ;
- le chlorure de 1-[(1-hydroxy-8-amino-naphthalèn-2-ylcarbamoyl)-méthyl]-1,4-diméthyl-pipérazin-1-ium ;
- le chlorure de 1-[(1-hydroxy-8-acétylamino-naphthalèn-2-ylcarbamoyl)-méthyl]-1,4-diméthyl-pipérazin-1-ium ;
- le chlorure de 1-[(1-hydroxy-8-méthoxycarbonylamino-naphthalèn-2-ylcarbamoyl)-méthyl]-1,4-diméthyl-pipérazin-1-ium ;
- le chlorure de 1-[(1-hydroxy-2-acétylamino-naphthalèn-8-ylcarbamoyl)-méthyl]-1,4-diméthyl-pipérazin-1-ium ;
- le chlorure de 1-[(1-hydroxy-2-méthoxycarbonylamino-naphthalèn-8-ylcarbamoyl)-méthyl]-1,4-diméthyl-pipérazin-1-ium ;
- le chlorure de 1-[(1-hydroxy-8-méthanesulfonylamino-naphthalèn-2-ylcarbamoyl)-méthyl]-1,4-diméthyl-pipérazin-1-ium ;
et leurs sels d'addition avec un acide

25. Composé selon l'une quelconque des revendications précédentes, **caractérisé par le fait que** les sels d'addition avec un acide sont choisis parmi les chlorhydrates, les bromhydrates, les sulfates, les citrates, les succinates, les tartrates, les lactates et les acétates.

26. Utilisation des composés de formule (I) tels que définis à l'une quelconque des revendications 1 à 24, à titre de coupleur pour la teinture d'oxydation des fibres kératiniques.

27. Composition pour la teinture d'oxydation des fibres kératiniques, **caractérisée par le fait qu'**elle contient, dans un milieu approprié pour la teinture :
- au moins une base d'oxydation, et
- au moins un coupleur choisi parmi les composés de formule (I) tels que définis à l'une quelconque des revendications 1 à 25.

28. Composition selon la revendication 27, **caractérisée par le fait que** le ou les composés de formule (I) et/ou le ou leurs sels d'addition avec un acide représentent de 0,0005 à 12 % en poids du poids total de la composition tinctoriale.

29. Composition selon la revendication 27 ou 28, **caractérisée par le fait que** la ou les bases d'oxydation sont choisies parmi les paraphénylènediamines, les bis-phénylalkylènediamines, les para-aminophénols, les ortho-aminophénols et les bases hétérocycliques, et leurs sels d'addition avec un acide.

30. Composition selon l'une quelconque des revendications 27 à 29, **caractérisée par le fait que** la ou les bases d'oxydation représentent de 0,0005 à 12 % en poids du poids total de la composition tinctoriale.

31. Composition selon l'une quelconque des revendications 27 à 30, **caractérisée par le fait qu'**elle renferme en plus du ou des composés de formule (I) ci-dessus, un ou plusieurs coupleurs additionnels choisis parmi les métaphénylènediamines, les méta-aminophénols, les métadiphénols et les coupleurs hétérocycliques, et leurs sels d'addition avec un acide, et/ou un ou plusieurs colorants directs.

32. Composition selon la revendication 31, **caractérisée par le fait que** le ou les coupleurs additionnels représentent de 0,0001 à 10 % en poids du poids total de la composition tinctoriale.

33. Composition selon l'une quelconque des revendications 27 à 32, **caractérisée par le fait que** les sels d'addition avec un acide sont choisis parmi les chlorhydrates, les bromhydrates, les sulfates, les citrates, les succinates, les tartrates, les lactates et les acétates.

34. Procédé de teinture d'oxydation des fibres kératiniques, **caractérisé par le fait que** l'on applique sur ces fibres au moins une composition tinctoriale telle que définie à l'une quelconque des revendications 27 à 33, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

35. Procédé selon la revendication 34, **caractérisé par le fait que** l'agent oxydant est choisi parmi le peroxyde d'hydrogène, le peroxyde d'urée, les bromates de métaux alcalins, les persels, et les enzymes.

36. Dispositif à plusieurs compartiments, ou "kit" de teinture à plusieurs compartiments, dont un premier compartiment renferme une composition tinctoriale telle que définie à l'une quelconque des revendications 27 à 33 et un second compartiment renferme une composition oxydante.

## Patentansprüche

1. Verbindung der folgenden Formel (I) und deren Additionssalze mit einer Säure: 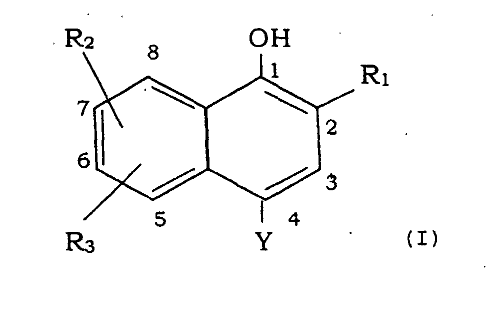 worin bedeuten:
· R₁ eine Gruppe, die unter -NR₄(C=O)R₅, -NR₆SO₂R₇ und -(C=O)NR₆R₈ ausgewählt ist, wobei die Gruppen R₄, R₅, R₆, R₇, und R₈ nachstehend definiert sind;
· R₂ und R₃, die gleich oder verschieden sind, Wasserstoff; Halogen; eine nachstehend definierte Gruppe Z; eine geradkettige oder verzweigte Gruppe mit 1 bis 20 Kohlenstoffatomen (wobei der Zweig oder die Zweige einen oder mehrere 3-bis 7-gliedrige kohlenstoffhaltige Ringe bilden können), die eine oder mehrere Doppelbindungen und/oder eine oder mehrere Dreifachbindungen enthalten kann (wobei die Doppelbindungen gegebenenfalls zu aromatischen Gruppen führen), in der ein oder mehrere Kohlenstoffatome durch ein Sauerstoffatom, Stickstoffatom, Schwefelatom oder eine Gruppe -SO₂ ersetzt sein können und in die Kohlenstoffatome unabhängig voneinander mit einem oder mehreren Halogenatomen substituiert sein können;
mit der Maßgabe, dass:
- die Gruppen R₂ und R₃ nicht über eine Binding -NH-NH- an den Benzolring der Formel (I) gebunden sein können; und die Gruppen R₂ und R₃ keine Peroxidbindung und keine Diazogruppe, Nitrogruppe oder Nitrosogruppe enthalten;
- die Gruppen R₂ und R₃ keine Hydroxygruppe oder Thiogruppe bedeuten können; und
- die Gruppen R₂ und R₃ nicht gleichzeitig Amino, Alkylamino, Acylamino oder Sulfonylamino bedeuten können;
Y Wasserstoff, Halogen; eine Gruppe -OR₉, -SR₉ oder -NH-SO₂R₉, worin R₉ eine geradkettige oder verzweigte C₁₋₆-Alkylgruppe bedeutet (wobei der Zweig oder die Zweige einen oder mehrere 3- bis 6-gliedrige Ringe bilden können), die unsubstituiert vorliegt oder mit einer oder mehreren Gruppen substituiert ist, die unter Halogen, Hydroxy, C₁₋₄-Alkoxy, Amino und C₁₋₄-Aminoalkyl ausgewählt sind; eine Phenylgruppe, die gegebenenfalls mit einer oder zwei Gruppen substituiert ist, die unter C₁₋₄-Alkyl, Trifluormethyl, Carboxy, C₁₋₄-Alkoxycarbonyl, Halogen, Hydroxy, C₁₋₄-Alkoxy, Amino und C₁₋₄-Aminoalkyl ausgewählt sind; oder eine Benzylgruppe; R₄, R₆ und R₈, die gleich oder verschieden sind, Wasserstoff; eine nachstehend definierte Gruppe Z; eine geradkettige oder verzweigte Gruppe mit 1 bis 15 Kohlenstoffatomen (wobei der Zweig oder die Zweige einen oder mehrere 3- bis 7-gliedrige kohlenstoffhaltige Ringe bilden können), die eine oder mehrere Doppelbindungen und/oder eine oder mehrere Dreifachbindungen enthalten kann (wobei die Doppelbindungen gegebenenfalls zu aromatischen Gruppen führen), in der ein oder mehrere Kohlenstoffatome durch ein Sauerstoffatom, Stickstoffatom, Schwefelatom oder eine Gruppe SO₂ ersetzt sein können und in der die Kohlenstoffatome unabhängig voneinander mit einem oder mehreren Halogenatomen substituiert sein können;
mit der Maßgabe, dass die Gruppe SO₂ nicht direkt an das Stickstoffatom gebunden sein kann, welches die Gruppe R₄ oder R₆ trägt;
mit der Maßgabe, dass die Gruppe -(C=O)- nicht direkt an das Stickstoffatom gebunden sein kann, welches die Gruppe R₆ trägt;
mit der Maßgabe, dass die Gruppen R_{4,} R₆ und R₈ keine Peroxidbindung und keine Diazogruppe, Nitrogruppe oder Nitrosogruppe enthalten;
mit der Maßgabe, dass die Gruppen R₄, R₆ und R₈ nicht Hydroxy, Thio, Amino, Alkoxy oder Alkylthio bedeuten können;
· R₅ und R₇, die gleich oder verschieden sind, Wasserstoff; eine nachstehend definierte Gruppe Z; eine geradkettige oder verzweigte Gruppe mit 1 bis 20 Kohlenstoffatomen (wobei der Zweig oder die Zweige einen oder mehrere 3- bis 7-gliedrige kohlenstoffhaltige Ringe bilden können), die eine oder mehrere Doppelbindungen und/oder eine oder mehrere Dreifachbindungen enthalten kann (wobei die Doppelbindungen gegebenenfalls zu aromatischen Gruppen führen), in der ein oder mehrere Kohlenstoffatome durch ein Sauerstoffatom, Stickstoffatom, Schwefelatom oder eine Gruppe SO₂ ersetzt sein können und in der die Kohlenstoffatome unabhängig voneinander mit einem oder mehreren Halogenatomen substituiert sein können;
mit der Maßgabe, dass die Gruppen R₅ und R₇ keine Peroxidbindung und keine Diazogruppe, Nitrogruppe oder Nitrosogruppe enthalten;
mit der Maßgabe, dass die Gruppe R₅ keine Hydroxygruppe und keine Thiogruppe bedeuten kann;
mit der Maßgabe, dass die Gruppe R₇ keine Thiogruppe bedeuten kann;
mit der Maßgabe, dass einerseits die Gruppen R₄ und R₅ und andererseits die Gruppen R₆ und R₈ ferner unabhängig voneinander einen 5- bis 7-gliedrigen, gesättigten oder ungesättigten Ring bilden können, der aus Kohlenstoff, Stickstoff und/oder Acyl besteht, wobei jedes Kettenglied gegebenenfalls mit einer oder zwei identischen oder voneinander verschiedenen Gruppen R substituiert sein kann, wobei R eine geradkettige oder verzweigte C₁₋₈-Alkylgruppe bedeutet (wobei der Zweig oder die Zweige einen oder mehrere 3- bis 7-gliedrige Ringe bilden können), die eine oder mehrere Doppelbindungen und/oder eine oder mehrere Dreifachbindungen enthalten kann (wobei die Doppelbindungen gegebenenfalls zu aromatischen Gruppen führen), in der ein oder mehrere Kohlenstoffatome durch ein Sauerstoffatom, Stickstoffatom, Schwefelatom oder eine Gruppe SO₂ ersetzt sein können und in der die Kohlenstoffatome unabhängig voneinander mit einem oder mehreren Halogenatomen substituiert sein können; wobei die Ringe keine Peroxidbindung und keine Diazogruppe, Nitrogruppe oder Nitrosogruppe enthalten;
· Z eine kationische Gruppe der folgenden Formel (II): 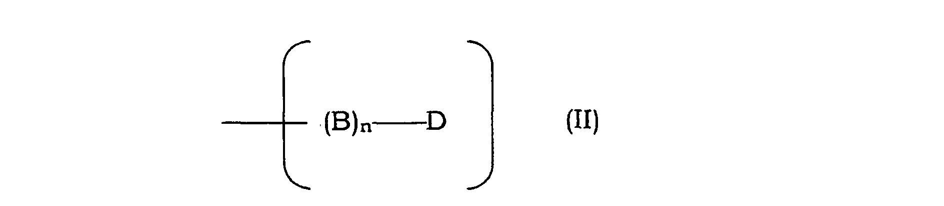 worin bedeuten:
- n 0 oder 1,
- B eine geradkettige oder verzweigte (wobei der Zweig oder die Zweige einen oder mehrere 3- bis 7-gliedrige Ringe bilden können) Alkylgruppe mit 1 bis 15 Kohlenstoffatomen, die eine oder mehrere Doppelbindungen und/oder eine oder mehrere Dreifachbindungen enthalten kann, wobei die Doppelbindungen gegebenenfalls zu aromatischen Gruppen führen, und in der ein oder mehrere Kohlenstoffatome durch ein Sauerstoffatom, Stickstoffatom, Schwefelatom oder eine Gruppe -SO₂ ersetzt sein können und in der ein oder mehrere Kohlenstoffatome unabhängig voneinander mit einem oder mehreren Halogenatomen oder einer oder mehreren Gruppen Z substituiert sein können; wobei die Gruppe B keine Peroxidbindung und keine Diazogruppe, Nitrogruppe oder Nitrosogruppe enthält;
- D eine Gruppe, die unter den kationischen Gruppen der folgenden Formeln (III) und (IV) ausgewählt ist: 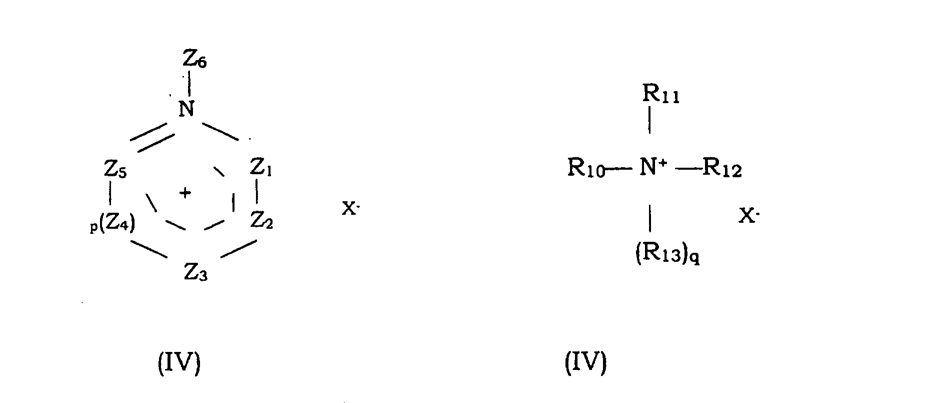
wobei in den Formeln:
- p und q unabhängig voneinander den Wert 0 oder 1 haben;
- die Gruppe B über ein beliebiges Atom der Gruppe D an die Gruppe D gebunden ist;
- die Gruppe der Formel (IV) direkt über das Stickstoffatom der quartären Ammoniumgruppe an die Verbindung der Formel (I) gebunden sein kann, wenn n = 0 und q = 0;
- Z₁, Z₂, Z₃ und Z₄ unabhängig voneinander ein Sauerstoffatom, Schwefelatom, ein unsubstituiertes oder mit einer Gruppe R₁₄ substituiertes Stickstoffatom oder ein unsubstituiertes oder mit einer oder zwei gleichen oder verschiedenen Gruppen R₁₄ substituiertes Kohlenstoffatom bedeuten;
- Z₅ ein Stickstoffatom oder ein unsubstituiertes oder mit einer Gruppe R₁₄ substituiertes Kohlenstoffatom bedeutet;
- Z₆ die nachstehend für die Gruppe R₁₄ angegebenen Bedeutungen annehmen kann, mit der Maßgabe, dass Z₆ von Wasserstoff verschieden ist;
- wobei die Gruppen Z₁ oder Z₅ ferner mit Z₆ einen 5- bis 7-gliedrigen, gesättigten oder ungesättigten Ring bilden können, wobei jedes Kettenglied gegebenenfalls mit einer oder zwei gleichen oder verschiedenen Gruppen R₁₄ substituiert ist;
- R₁₄ Wasserstoff; eine Gruppe Z; eine geradkettige oder verzweigte Gruppe mit 1 bis 10 Kohlenstoffatomen bedeutet, die eine oder mehrere Doppelbindungen und/oder eine oder mehrere Dreifachbindungen enthalten kann, wobei die Doppelbindungen gegebenenfalls zu aromatischen Gruppen führen, in der ein oder mehrere Kohlenstoffatome durch ein Sauerstoffatom, Stickstoffatom, Schwefelatom oder eine Gruppe SO₂ ersetzt sein können und in der ein oder mehrere Kohlenstoffatome unabhängig voneinander mit einem oder mehreren Halogenatomen substituiert sein können; wobei die Gruppe keine Peroxidbindung und keine Diazogruppe, Nitrogruppe oder Nitrosogruppe enthält;
- wobei zwei angrenzende Gruppen Z₁, Z₂, Z₃, Z₄ und Z₅ ferner einen 5- bis 7-gliedrigen Ring bilden können, wobei jedes Kettenglied unabhängig ein Kohlenstoffatom, das gegebenenfalls mit einer oder zwei gleichen oder verschiedenen Gruppen R₁₄ substituiert ist, ein unsubstituiertes oder mit einer Gruppe R₁₄ substituiertes Stickstoffatom, ein Sauerstoffatom oder ein Schwefelatom bedeuten kann;
- die Gruppen R₁₀, R₁₁, R₁₂ und R₁₃, die gleich oder verschieden sind, die oben für die Gruppe R₁₄ angegebenen Bedeutungen haben;
- wobei jeweils 2 der Gruppen R₁₀, R₁₁, R₁₂ und R₁₃ ferner mit dem quartären Stickstoffatom, an das sie gebunden sind, einen oder mehrere, gesättigte 5- bis 7-gliedrige Ringe bilden können, wobei jedes Kettenglied unabhängig ein Kohlenstoffatom, das gegebenenfalls mit einer oder zwei gleichen oder verschiedenen Gruppen R₁₄ substituiert ist, ein unsubstituiertes oder mit einer Gruppe R₁₄ substituiertes Stickstoffatom, ein Sauerstoffatom oder ein Schwefelatom bedeuten kann;
· X ein organisches oder anorganisches Anion bedeutet;
mit der Maßgabe, dass mindestens eine der Gruppen R₁ bis R₃ eine Gruppe Z bedeutet oder eine Gruppe Z enthält.

2. Verbindung nach Anspruch 1, **dadurch gekennzeichnet, dass** die Gruppen R₂ und R₃ unabhängig voneinander bedeuten:
Wasserstoff; Chlor; eine Gruppe Z; Methyl oder Methoxy; Amino, Methylamino, 2-Hydroxyethylamino; eine Gruppe -NH(CO)R₁₅, wobei R₁₅ eine Gruppe bedeutet, die aus der Gruppe (G1) ausgewählt ist, die aus den folgenden Gruppen besteht: Methyl, Ethyl, Propyl, Isopropyl, Butyl, Isobutyl, *t*-Butyl, Pentyl, Isopentyl, Neopentyl, Hexyl, Cyclopropyl, Cyclobutyl, Cyclopentyl, Cyclopentylmethyl, 3-Cyclopentylpropyl, Cyclohexyl, 2-Cyclohexylethyl, Norbornan-2-yl, Vinyl, 1-Methylvinyl, 2-Methylvinyl, 2,2-Dimethylvinyl, Allyl, 3-Butenyl, Phenyl, Methylphenyl, Dimethylphenyl, 2,4,6-Trimethylphenyl, 4-Ethylphenyl, (Trifluormethyl)-phenyl, Hydroxyphenyl, Methoxyphenyl, Ethoxyphenyl, Acetoxyphenyl, (Trifluormethoxy)phenyl, Aminophenyl, 4-Dimethylaminopheriyl, Fluorphenyl, Difluorphenyl, Fluor(trifluormethyl)-phenyl, Chlorphenyl, Dichlorphenyl, Bromphenyl, Naphth-1-yl, Naphth-2-yl, (2-Methoxy)naphth-1-yl, Benzyl, 4'-Methoxybenzyl, 2',5'-Dimethoxybenzyl, 3',4'-Dimethoxybenzyl, 4'-Fluorbenzyl, 4'-Chlorbenzyl, Phenethyl, 2-Phenylvinyl, (1-Naphthyl)methyl, (2-Naphthyl)methyl; Tetrahydrofuran-2-yl, Furan-2-yl, 5-Methyl-2-(trifluormethyl)furan-3-yl, 2-Methyl-5-phenylfuran-3-yl, Thiophen-2-yl, (Thiophen-2-yl)methyl, 3-Chlorthiophen-2-yl, 2,5-Dichlorthiophen-3-yl, Benzothiophen-2-yl, 3-Chlorbenzothiophen-2-yl, Isoxazol-5-yl, 5-Methylisoxazol-3-yl, 3,5-Dimethylisoxazol-4-yl, 1,3-Dimethylpyrazol-5-yl, 1-Ethyl-3-methylpyrazol-5-yl, 1-*t*-Butyl-3-methylpyrazol-5-yl, 3-*t*-Butyl-1-methylpyrazol-5-yl, 4-Brom-1-ethyl-3-methylpyrazol-5-yl, Pyridinyl, Chlorpyridinyl, Dichlorpyridinyl, 5-(Brom)pyridin-3-yl, Piperazin-2-yl, 2-Chlor-4-(trifluormethyl)pyrimidin-5-yl, Chinoxal-2-yl, Benzofurazan-5-yl, Fluormethyl, Difluormethyl, Trifluormethyl, 1,1,2,2-Tetrafluorethyl, Pentafluorethyl, Heptafluorpropyl, 1,1,2,2,3,3,4,4-Octafluorbutyl, Nonafluorbutyl, Chlormethyl, Chlorethyl, 1,1-Dimethyl-2-chlorethyl, 1,2-Dichlorethyl, 1-Chlorpropyl, 3-Chlorpropyl, 4-Chlorbutyl, Hydroxymethyl, Methoxymethyl, Phenoxymethyl, (4-Chlorphenoxy)methyl, Benzyloxymethyl, Acetoxymethyl, 1,2-Dihydroxyethyl, 1-Phenoxyethyl, 1-Acetoxyethyl, 2-(2-Carboxyethoxy)ethyl, 1-Phenoxyethyl, 1-Acetoxyethyl, (Methoxycarbonyl)methyl, 2-Carboxyethyl, 2-(Methoxycarbonyl)ethyl, 2-Carboxycyclopropyl, 2-Carboxycyclohexan, Methoxy, Ethoxy, Propoxy, Isopropoxy, Butoxy, Isobutoxy, Pentoxy, Neopentoxy, Hexyloxy, Cyclopentyloxy, Cyclohexyloxy, Vinyloxy, Allyloxy, Propargyloxy, Chlormethoxy, 1-Chlorethoxy, 2-Methoxyethoxy, 4-Chlorbutoxy, Phenoxy, 4-Methylphenoxy, 4-Fluorphenoxy, 4-Bromphenoxy, 4-Chlorphenoxy, 4-Methoxyphenoxy, Naphth-2-yloxy, Benzyloxy; Amino, Methylamino, Ethylamino, Propylamino, Isopropylamino, Butylamino, Cyclohexylamino, Allylamino, 2-Chlorethylamino, 3-Chlorpropylamino, Carboxymethylamino, Phenylamino, Fluorphenylamino, (Trifluormethyl)phenylaino, Chlorphenylamino, Bromphenylamino, 4-Acetylphenylamino, Methoxyphenylamino, (Trifluormethoxy)phenylamino, Naphth-1-ylamino, Benzylamino, Phenetylamino, Pyrid-3-ylamino, Dimethylamino, 1-Pyrolidinyl und 4-Morpholinyl; oder eine Gruppe -NHSO₂R₁₆, wobei R₁₆ eine Gruppe bedeutet, die aus der Gruppe (G2) ausgewählt ist, die aus Methyl, Trifluormethyl, Ethyl, 2-Chlorethyl, Propyl, 3-Chlorpropyl, Isopropyl, Butyl, Thiophen-2-yl, Hydroxy, Ethoxy, Amino und Dimethylamino besteht.

3. Verbindung nach Anspruch 2, **dadurch gekennzeichnet, dass** die Gruppen R₂ und R₃ unabhängig voneinander bedeuten:
Wasserstoff; eine Gruppe O-E-D', -NH-E-D', -NH(CO)-D', -NH(CO)-E-D', -NH(CO)O-E-D', -NH(CO)NH-E-D oder -NH(SO₂)-E-D', wobei -E- eine Kette -(CH₂)_{q}- bedeutet, worin q 1 oder 2 ist, und D' 3-Methylimidazolidinium-1-yl, 3-(2-Hydroxyethyl)-imidazolidinium-1-yl, 1,2,4-Triazolinium-1-yl, 1,2,4-Triazolinium-4-yl, N-Alkyl(C₁₋₄)pyridinium-2-yl, N-Alkyl(C₁₋₄)pyridinium-3-yl, N-Alkyl(C₁₋₄)pyridinium-4-yl, N-(2-Hydroxyethyl)-pyridinium-2-yl, N-(2-Hydroxyethyl)pyridinium-3-yl, N-(2-Hydroxyethyl)pyridinium-4-yl, Pyridinium-1-yl, Trialkyl(C₁₋₄)-ammonium-N-yl, 1-Methylpiperidinium-1-yl und 1,4-Dimethylpiperazinium-1-yl; eine Gruppe Methyl, Methoxy, Amino oder Methylamino; eine Gruppe-NHSO₂R₁₇, wobei R₁₇ aus der oben definierten Gruppe (G2) stammt; oder Methansulfonylamino, Ethansulfonylamino oder Dimethylaminosulfonylamino bedeutet.

4. Verbindung nach einem der vorhergehenden Ansprüche, **dadurch gekennzeichnet, dass** die Gruppe R₄ bedeutet: Wasserstoffatom; eine Gruppe A₁, die eine geradkettige oder verzweigte C₁₋₈-Alkylgruppe ist, die eine oder zwei Doppelbindungen oder eine Dreifachbindung enthalten kann und die unsubstituiert vorliegt oder gegebenenfalls mit einer Gruppe, die unter den nachfolgend definierten Gruppen A₂, A₄ und A₅ ausgewählt ist, gegebenenfalls mit einer oder zwei Gruppen, die identisch oder voneinander verschieden sind und unter den Gruppen N-Alkyl(C₁₋₃)amino, N-Alkyl(C₁₋₃)-N-alkyl(C₁₋₃)amino, Alkoxy(C₁₋₆), Oxo, Alkoxycarbonyl, Acyloxy, Amid, Acylamino, Ureyl, Sulfoxy, Sulfonyl, Sulfonamido, Sulfonylamino, Brom, Cyano und Carboxy ausgewählt sind, und gegebenenfalls mit einer oder mehreren Hydroxygruppen, Fluoratomen oder Chloratomen substituiert ist; eine Gruppe A₂, bei der es sich um eine aromatische Gruppe vom Typ Phenyl oder Naphthyl handelt, die unsubstituiert vorliegen oder gegebenenfalls mit 1 bis 3 Gruppen substituiert sein kann, die gleich oder verschieden sind und unter den Gruppen Methyl, Trifluormethyl, Ethyl, Isopropyl, Butyl, Pentyl, Fluor, Chlor, Brom, Methoxy, Trifluormethoxy, Ethoxy, Propyloxy, Acetyloxy, Acetyl und Cyano ausgewählt sind; eine Gruppe A₃, wobei es sich um heteroaromatische Gruppen handelt, die unter den folgenden Gruppen ausgewählt sind: Furanyl, Thiophenyl, Pyrrolyl, Imidazolyl, Thiazolyl, Oxazolyl, 1,2,3-Triazolyl, 1,2,4-Triazolyl, Isoxazolyl, Isothiazolyl, Pyrazolyl, Pyrazoltriazolyl, Pyrazoloimidazolyl, Pyrrolotriazolyl, Pyrazolopyrimidyl, Pyrazolopyridyl, Pyridyl, Pyrimidyl, Benzoimidazolyl, Benzoxazolyl, Benzothiazolyl, Indolyl, Indolidinyl, Isoindolyl, Indazolyl, Benzotriazolyl, Chinolinyl, Benzoimidazolyl und Benzopyrimidyl, wobei diese heteroaromatischen Gruppen gegebenenfalls mit 1 bis 3 Gruppen substituiert sein können, die unter den geradkettigen oder verzweigten C₁₋₄-Alkylgruppen, C₁₋₄-Monohydroxyalkyl, C₂₋₄-Polyhydroxyalkyl, Carboxy, Alkoxycarbonyl, Halogen, Amido, Amino und Hydroxy ausgewählt sind; eine Gruppe A₄, wobei des sich um C₃₋₇-Cycloalkyl oder eine Norbornanylgruppe handelt und wobei sie gegebenenfalls eine Doppelbindung aufweist und gegebenenfalls mit einer oder zwei Gruppen substituiert ist, die unter den geradkettigen oder verzweigten C₁₋₄-Alkylgruppen, C₁₋₄-Monohydroxyalkyl, C₂₋₄-Polyhydroxyalkyl, Carboxy, Alkoxycarbonyl, Halogen, Amido, Amino und Hydroxy ausgewählt sind, oder eine Gruppe A₅, die ein Heterocyclus ist, der unter Dihydrofuranyl, Tetrahydrofuranyl, Butyrolact-on-yl, Dihydrothiophenyl, Tetrahydrothiophenyl, Tetrahydrothiophen-onyl, Iminothiolan, Dihydropyrrolyl, Pyrrolidinyl, Pyrrolidin-on-yl, Imidazolidin-on-yl, Imidazolidinthionyl, Oxazolidinyl, Oxazolidinon-yl, Oxazolanthion, Thiazolidinyl, Isothiazol-on-yl, Mercaptothiazolinyl, Pyrazolidin-on-yl, Iminothiolan, Dioxolanyl, Pentalacton, Dioxanyl, Dihydropyridinyl, Piperdinyl, Pentalactam, Morpholinyl, Pyrazoli(di)nyl, Pyrimi(di)nyl, Pyrazinyl, Piperazinyl und Azepinyl ausgewählt ist, wobei die Gruppen A₁, A₂, A₃, A₄ und/oder As gegebenenfalls über eine Gruppe -(CO)- an das Stickstoffatom der Gruppe -NR₄(C=O)R₅ gebunden sind.

5. Verbindung nach Anspruch 4, **dadurch gekennzeichnet, dass** R₄ bedeutet: Wasserstoff, Methyl, Ethyl, Isopropyl, Allyl, Phenyl, Benzyl, Fluorbenzyl, Hydroxybenzyl, Difluorbenzyl, Trifluorbenzyl, Chlorbenzyl, Brombenzyl, Methoxybenzyl, Dimethoxybenzyl, (Trinuorm.ethoxy)benzyl, 3,4-Methylendioxybenzyl, 6-Chlorpiperonyl, 4-Methylthiobenzyl, 4-Methylsulfonylbenzyl, 4-Acetylaminobenzyl, 4-Carboxybenzyl, 1-Naphtomethyl, 2-Naphthomethyl; 2-Hydroxyethyl, 2-Methoxyethyl oder 2-Ethoxyethyl.

6. Verbindung nach einem der vorhergehenden Ansprüche, **dadurch gekennzeichnet, dass** die Gruppe R₅ bedeutet: Wasserstoff, Amino; eine Gruppe Z; oder eine in Anspruch 4 definierte Gruppe A₁, A₂, A₃, A₄ oder A₅, die gegebenenfalls über eine Gruppe -O-, -NH-, -Nalkyl(C₁₋₃)- oder -(CO)- an das Kohlenstoffatom (in 2-Stellung) der Amidfunktion der Verbindung der Formel (I) gebunden ist.

7. Verbindung nach Anspruch 6, **dadurch gekennzeichnet, dass** die Gruppe R₅ bedeutet: eine Gruppe Z; oder eine Gruppe, die zur Gruppe (G3) gehört, welche aus Methyl, Ethyl, Propyl, Allyl, Phenyl, Tetrahydrofuran-2-yl, Furan-2-yl, Thiophen-2-yl, Pyridinyl, Piperazin-2-yl, Fluormethyl, Chlormethyl, 2-Chlorethyl, Methoxymethyl, Acetoxymethyl, 1,2-Dihydroxyethyl, Methoxycarbonyl, 2-Carboxyethyl, Methoxy, Ethoxy, Propoxy, Allyloxy, 2-Chlorethoxy, 2-Methoxyethoxy, Amino, Ethylamino, Allylamino, 2-Chlorethylamino, Pyridylamino, Dimethylamino, 1-Pyrolidinyl und 4-Morpholinyl besteht.

8. Verbindung nach Anspruch 7, **dadurch gekennzeichnet, dass** die Gruppe R₅ bedeutet: -D', -E-D', -O-E-D' oder -NH-E-D', wobei -E- eine Kette -(CH₂)_{q}-, worin q 1 oder 2 ist, und D' eine in Anspruch 3 definierte Gruppe bedeutet; oder eine Gruppe, die zur Gruppe (G4) gehört, welche aus Methyl, Methoxymethyl, 2-Carboxyethyl, Methoxy, Amino, Ethylamino und 1-Pyrolidinyl besteht.

9. Verbindung nach einem der vorhergehenden Ansprüche, **dadurch gekennzeichnet, dass**, falls die Gruppen R₄ und R₅ einen Ring bilden, der Ring ausgewählt ist unter: 2-Pyrrolidinon-1-yl, Methyl-2-pyrrolidinon-1-yl, 5-Carboxy-2-pyrrolidinon-1-yl, 5-Methoxycarbonyl-2-pyrrolidinon-1-yl, Pyrazolinon-1-yl, Succinimd-1-yl, 3,5-Diketopyrazolidin-1-yl, Oxindolin-1-yl, Maleimid-1-yl, Isoindol-1,3-dion-2-yl, 2-Piperidinon-1-yl und Glutarimid-1-yl.

10. Verbindung nach einem der vorhergehenden Ansprüche, **dadurch gekennzeichnet, dass** die Gruppe R₆ bedeutet: Wasserstoff oder eine in Anspruch 4 definierte Gruppe A₁, A₂, A₃, A₄ oder A₅.

11. Verbindung nach Anspruch 10, **dadurch gekennzeichnet, dass** die Gruppe R₆ bedeutet: Wasserstoff; Methyl, Ethyl, Isopropyl, Allyl; Phenyl, Benzyl, Fluorbenzyl, Hydroxybenzyl, Difluorbenzyl, Trifluorbenzyl, Chlorbenzyl, Brombenzyl, Methoxybenzyl, Dimethoxybenzyl, (Trifluormethoxy)benzyl, 3,4-Methylendioxybenzyl, 6-Chlorpiperonyl, 4-Methylthiobenzyl, 4-Methylsulfonylbenzyl, 4-Acetylaminobenzyl, 4-Carboxybenzyl, 1-Naphtomethyl, 2-Naphthomethyl; 2-Hydroxyethyl, 2-Methoxyethyl oder 2-Ethoxyethyl.

12. Verbindung nach einem der vorhergehenden Ansprüche, **dadurch gekennzeichnet, dass** die Gruppe R₈ bedeutet: eine Gruppe Z; Wasserstoff oder eine in Anspruch 4 definierte Gruppe A₁, A₂, A₃, A₄ oder A₅.

13. Verbindung nach Anspruch 12, **dadurch gekennzeichnet, dass** die Gruppe R₈ bedeutet: eine Gruppe Z; Wasserstoff; Methyl, Ethyl, Isopropyl, Allyl; Phenyl, Benzyl, Fluorbenzyl, Hydroxybenzyl, Difluorbenzyl, Trifluorbenzyl, Chlorbenzyl, Brombenzyl, Methoxybenzyl, Dimethoxybenzyl, (Trifluormethoxy)benzyl, 3,4-Methylendioxybenzyl, 6-Chlorpiperonyl, 4-Methylthiobenzyl, 4-Methylsulfonylbenzyl, 4-Acetylaminobenzyl, 4-Carboxybenzyl, 1-Naphtomethyl, 2-Naphthomethyl; 2-Hydroxyethyl, 2-Methoxyethyl oder 2-Ethoxyethyl.

14. Verbindung nach Anspruch 13, **dadurch gekennzeichnet, dass** die Gruppe R₈ bedeutet: Wasserstoff, Methyl oder eine Gruppe -D' oder -E-D', worin -E- eine Kette -(CH₂)_{q} mit q = 1 oder 2 bedeutet und D' eine in Anspruch 3 definierte Gruppe ist.

15. Verbindung nach einem der vorhergehenden Ansprüche, **dadurch gekennzeichnet, dass**, falls die Gruppen R₆ und R₈ einen Ring bilden, der Ring ausgewählt ist unter: Pyrrolidinyl, Piperidinyl, Morpholinyl, Pyrazolyl, Isoxazolyl, Imidazolyl, Thiazolyl, Indolyl, Indolidinyl, Indazolyl, Pyrazolotriazolyl, Pyrrolotriazolyl, Pyrazoloimidazolyl, Pyrazolidinyl, Thiomorpholinyl, Thiazolyl und Pyrazinyl.

16. Verbindung nach einem der vorhergehenden Ansprüche, **dadurch gekennzeichnet, dass** die Gruppe R₇ bedeutet: Amino; eine Gruppe Z; oder eine in Anspruch 4 definierte Gruppe A₁, A₂, A₃, A₄ oder A₅, die gegebenenfalls über eine Gruppe-NH oder Nalkyl(C₁₋₃) an den Schwefel der Sulfonylgruppe der Verbindung der Formel (I) gebunden sein kann.

17. Verbindung nach Anspruch 16, **dadurch gekennzeichnet, dass** die Gruppe R₇ bedeutet: eine Gruppe Z oder eine Gruppe aus der Gruppe (G4), welche aus Methyl, Trifluormethyl, Ethyl, 2-Chlorethyl, Propyl, 3-Chlorpropyl, Isopropyl, Butyl, Thiophen-2-yl, Hydroxy, Ethoxy, Amino und Dimethylamino besteht.

18. Verbindung nach Anspruch 17, **dadurch gekennzeichnet, dass** die Gruppe R₇ bedeutet: eine Gruppe -D', E-D' oder -NH-E-D', worin -E- eine Kette -(CH₂)_{q} mit q = 1 oder 2 ist und D' eine oben definierte Gruppe bedeutet; Methyl, Ethyl oder Dimethylamino.

19. Verbindung nach einem der vorhergehenden Ansprüche, **dadurch gekennzeichnet, dass** Y vorzugsweise bedeutet: Wasserstoff, Chlor, Fluor oder Brom; Methoxy, Ethoxy, Propoxy, Benzyloxy, Phenoxy; -OCH₂CH₂OMe; -OCH₂CH₂OMe; -OCH₂CH₂NMe₂; -OCH₂(CO)OH, -OCH₂(CO)OMe, -OCH₂(CO)OEt, -SCH₂CH₂CO₂H oder -NHSO₂Me

20. Verbindung nach Anspruch 19, **dadurch gekennzeichnet, dass** die Gruppe Y bedeutet: Wasserstoff, Chlor, Methoxy, -OCH₂(CO)OH oder -OCH₂(CO)OMe.

21. Verbindung nach einem der vorhergehenden Ansprüche, **dadurch gekennzeichnet, dass** D ausgewählt ist unter: Imidazolinium, Thiazolinium, Oxazolinium, Pyrrolinium, 1,2,3-Triazolinium, 1,2,4-Triazolinium, Isoxazolinium, Isothiazolinium, Imidazolidinium, Thiazolidinium, Pyrazolinium, Pyrazolidinium, Oxazolidinium, Pyrazoltriazolinium, Pyrazoloimidazolinium, Pyrrolotriazolinium, Pyrazolopyrimidinium, Pyrazolopyridinium, Pyridinium, Pyrimidinium, Pyrazinium, Triazinium, Benzimidazolinium, Benzoxazolinium, Benzothiazolinium, Indolinium, Indolidinium, Isoindolinium, Indazolinium, Benzotriazolinium, Chinolinium, Tetrahydrochinolinium, Benzoimidazolidinium, Benzopyrimidinium, Tetra-alkyl(C₁₋₄)-ammonium, Dialkylpiperidinium, Dialkylpyrrolidinium, Dialkylmorpholinium, Dialkylthiomorpholinium, Dialkylpiperazinium, Azepinium und 1,4-Diazabicyclo-[2,2,2]octanium.

22. Verbindung nach Anspruch 21, **dadurch gekennzeichnet, dass** D ausgewählt ist unter: 3-Methylimidazolidinium-1-yl, 3-(2-Hydroxyethyl)imidazolidinium-1-yl, 1,2,4-Triazolinium-1-yl, 1,2,4-Triazolinium-4-yl, N-Alkyl(C₁₋₄)pyridinium-2-yl, N-Alkyl(C₁₋₆)pyridinium-3-yl, N-Alkyl(C₁₋₄)pyridinium-4-yl, N-(2-Hydroxyethyl)pyridinium-2-yl, N-(2-Hydroxyethyl)pyridinium-3-yl, N-(2-Hydroxyethyl)pyridinium-4-yl, Pyridinium-1-yl, Trialkyl(C₁₋₄)ammonium-N-yl, 1-Methylpiperidinium-1-yl und 1,4-Dimethylpiperazinium-1-yl.

23. Verbindung nach einem der vorhergehenden Ansprüche, **dadurch gekennzeichnet, dass** sie unter den Verbindungen ausgewählt ist, worin:
i) - R₁ eine Gruppe bedeutet, die aus der Gruppe (G5) ausgewählt ist, welche aus Acetylamino, Methansulfonylamino, Amido, Methylamido, -NH-C(O)-CH₂-D1, -NH-SO₂-CH₂-CH₂-D1 und -(CO)-NH-CH₂-CH₂-D1 besteht, wobei D1 3-Methylimidazolidinium-1-yl, Triazolinium-1-yl, Pyridinium-1-yl oder Trimethylammonium-N-yl bedeutet;
- R₂ sich in 5-Stellung befindet und eine Gruppe bedeutet, die aus der Gruppe (G6) ausgewählt ist, welche aus Amino, Methylamino, Acetylamino, Methansulfonylamino, -NH-[C(O)]ₘ-CH₂-D1 mit m = 0 oder 1 oder -NH-SO₂-CH₂-CH₂-D1 besteht;
- R₃ Wasserstoff bedeutet;
- Y aus (G7) ausgewählt ist, wobei die Gruppe (G7) aus Wasserstoff, Chlor und Methoxy besteht; oder
ii) - R₁ eine Gruppe aus der oben definierten Gruppe (G5) bedeutet;
- R₂ sich in 8-Stellung befindet und eine Gruppe bedeutet, die zur oben definierten Gruppe (G6) gehört;
- R₃ Wasserstoff bedeutet;
- Y eine Gruppe aus der oben definierten Gruppe (G7) ist; oder
iii) - R₁ eine Gruppe aus der oben definierten Gruppe (G5) bedeutet;
- R₂ und R₃ Wasserstoff bedeuten;
- Y eine Gruppe aus der oben definierten Gruppe (G7) ist.

24. Verbindung nach einem der vorhergehenden Ansprüche, **dadurch gekennzeichnet, dass** sie unter den folgenden Verbindungen ausgewählt ist:
- 3-[(1-Hydroxy-naphthalin-2-ylcarbamoyl)-methyl]-1-methyl-3H-imidazol-1-iumchlorid;
- 3-[(1-Hydroxy-5-(2-(3-methyl-1H-imidazol-3-ium-1-yl)-acetyl)-amino-naphthalin-2-ylcarbamoyl)-methyl]-1-methyl-3H-imidazol-1-iumdichlorid;
- 3-[(1-Hydroxy-8-(2-(3-methyl-1H-imidazol-3-ium-1-yl)-acetyl)-amino-naphthalin-2-ylcarbamoyl)-methyl]-1-methyl-3H-imidazol-1-iumdichlorid;
- 3-[(1-Hydroxy-5-amino-naphthalin-2-ylcarbamoyl)-methyl]-1-methyl-3H-imidazol-1-iumchlorid;
- 3-[(1-Hydroxy-5-acetylamino-naphthalin-2-ylcarbamoyl)-methyl]-1-methyl-3H-imidazol-1-iumchlorid;
- 3-[(1-Hydroxy-5-methoxycarbonylamino-naphthalin-2-ylcarbamoyl)-methyl]-1-methyl-3H-imidazol-1-iumchlorid;
- 3-[(1-Hydroxy-2-acetylamino-naphthalin-5-ylcarbamoyl)-methyl]-1-methyl-3H-imidazol-1-iumchlorid;
- 3-[(1-Hydroxy-2-methoxycarbonylamino-naphthalin-5-ylcarbamoyl)-methyl]-1-methyl-3H-imidazol-1-iumchlorid;
- 3-[(1-Hydroxy-5-methansulfonylamino-naphthalin-2-ylcarbamoyl)-methyl]-1-methyl-3H-imidazol-1-iumchlorid;
- 3-[(1-Hydroxy-8-amino-naphthalin-2-ylcarbamoyl)-methyl]-1-methyl-3H-imidazol-1-iumchlorid;
- 3-[(1-Hydroxy-8-acetylamino-naphthalin-2-ylcarbamoyl)-methyl]-1-methyl-3H-imidazol-1-iumchlorid;
- 3-[(1-Hydroxy-8-methoxycarbonylamino-naphthalin-2-ylcarbamoyl)-methyl]-1-methyl-3H-imidazol-1-iumchlorid;
- 3-[(1-Hydroxy-2-acetylamino-naphthalin-8-ylcarbamoyl)-methyl]-1-methyl-3H-imidazol-1-iumchlorid;
- 3-[(1-Hydroxy-2-methoxycarbonylamino-naphthalin-8-ylcarbamoyl)-methyl]-1-methyl-3H-imidazol-1-iumchlorid;
- 3-[(1-Hydroxy-8-methansulfonylamino-naphthalin-2-ylcarbamoyl)-methyl]-1-methyl-3H-imidazol-1-iumchlorid;
- 1-[(1-Hydroxy-naphthalin-2-ylcarbamoyl)-methyl]-pyridiniumchlorid;
- 1-[(1-Hydroxy-5-(2-(pyridinium-1-yl)-acetyl)amino-naphthalin-2-ylcarbamoyl)-methyl]-pyridiniumdichlorid;
- 1-[(1-Hydroxy-8-(2-(pyridinium-1-yl)-acetyl)amino-naphthalin-2-ylcarbamoyl)-methyl]-pyridiniumdichlorid;
- 1-[(1-Hydroxy-5-amino-naphthalin-2-ylcarbamoyl)-methyl]-pyridiniumchlorid;
- 1-[(1-Hydroxy-5-acetylamino-naphthalin-2-ylcarbamoyl)-methyl]-pyridiniumchlorid;
- 1-[(1-Hydroxy-5-methoxycarbonylamino-naphthalin-2-ylcarbamoyl)-methyl]-pyridiniumchlorid;
- 1-[(1-Hydroxy-2-acetylamino-naphthalin-5-ylcarbamoyl)-methyl]-pyridiniumchlorid;
- 1-[(1-Hydroxy-2-methoxycarbonylamino-naphthalin-5-ylcarbamoyl)-methyl]-pyridiniumchlorid;
- 1-[(1-Hydroxy-5-methansulfonylamino-naphthalin-2-ylcarbamoyl)-methyl]-pyridiniumchlorid;
- 1-[(1-Hydroxy-8-amino-naphthalin-2-ylcarbamoyl)-methyl]-pyridiniumchlorid;
- 1-[(1-Hydroxy-8-acetylamino-naphthalin-2-ylcarbamoyl)-methyl]-pyridiniumchlorid;
- 1-[(1-Hydroxy-8-methoxycarbonylamino-naphthalin-2-ylcarbamoyl)-methyl]-pyridiniumchlorid;
- 1-[(1-Hydroxy-2-acetylamino-naphthalin-8-ylcarbamoyl)-methyl]-pyridiniumchlorid;
- 1-[(1-Hydroxy-2-methoxycarbonylamino-naphthalin-8-ylcarbamoyl)-methyl]-pyridiniumchlorid;
- 1-[(1-Hydroxy-8-methansulfonylamino-naphthalin-2-ylcarbamoyl)-methyl]-pyridiniumchlorid;
- 1-[(1-Hydroxy-naphthalin-2-ylcarbamoyl)-methyl]-1,4-dimethyl-piperazin-1-iumchlorid;
- 1-[(1-Hydroxy-5-(2-(1,4-dimethyl-piperazin-1-ium-1-yl)-acetyl)amino-naphthalin-2-ylcarbamoyl)-methyl]-1,4-dimethyl-piperazin-1-iumdichlorid;
- 1-[(1-Hydroxy-8-(2-(1,4-dimethyl-piperazin-1-ium-1-yl)-acetyl)amino-naphthalin-2-ylcarbamoyl)-methyl]-1,4-dimethyl-piperazin-1-iumdichlorid;
- 1-[(1-Hydroxy-5-amino-naphthalin-2-ylcarbamoyl)-methyl]-1,4-dimethyl-piperazin-1-iumchlorid;
- 1-[(1-Hydroxy-5-acetylamino-naphthalin-2-ylcarbamoyl)-methyl]-1,4-dimethyl-piperazin-1-iumchlorid;
- 1-[(1-Hydroxy-5-methoxycarbonylamino-naphthalin-2-ylcarbamoyl)-methyl]-1,4-dimethyl-piperazin-1-iumchlorid;
- 1-[(1-Hydroxy-2-acetylamino-naphthalin-5-ylcarbamoyl)-methyl]-1,4-dimethyl-piperazin-1-iumchlorid;
- 1-[(1-Hydroxy-2-methoxycarbonylamino-naphthalin-5-ylcarbamoyl)-methyl]-1,4-dimethyl-piperazin-1-iumchlorid;
- 1-[(1-Hydroxy-5-methansulfonylamino-naphthalin-2-ylcarbamoyl)-methyl]-1,4-dimethyl-piperazin-1-iumchlorid;
- 1-[(1-Hydroxy-8-amino-naphthalin-2-ylcarbamoyl)-methyl]-1,4-dimethyl-piperazin-1-iumchlorid;
- 1-[(1-Hydroxy-8-acetylamino-naphthalin-2-ylcarbamoyl)-methyl]-1,4-dimethyl-piperazin-1-iumchlorid;
- 1-[(1-Hydroxy-8-methoxycarbonylamino-naphthalin-2-ylcarbamoyl)-methyl]-1,4-dimethyl-piperazin-1-iumchlorid;
- 1-[(1-Hydroxy-2-acetylamino-naphthalin-8-ylcarbamoyl)-methyl]-1,4-dimethyl-piperazin-1-iumchlorid;
- 1-[(1-Hydroxy-2-methoxycarbonylamino-naphthalin-8-ylcarbamoyl)-methyl]-1,4-dimethyl-piperazin-1-iumchlorid;
- 1-[(1-Hydroxy-8-methansulfonylamino-naphthalin-2-ylcarbamoyl)-methyl]-1,4-dimethyl-piperazin-1-iumchlorid; und deren Additionssalze mit einer Säure.

25. Verbindung nach einem der vorhergehenden Ansprüche, **dadurch gekennzeichnet, dass** die Additionssalze mit einer Säure unter den Hydrochloriden, Hydrobromiden, Sulfaten, Citraten, Succinaten, Tartraten, Lactaten und Acetaten ausgewählt sind.

26. Verwendung von Verbindungen der Formel (I) nach einem der Ansprüche 1 bis 24 als Kuppler zum oxidativen Färben von Keratinfasern.

27. Zusammensetzung zum oxidativen Färben von Keratinfasern, **dadurch gekennzeichnet, daß** sie in einem zum Färben geeigneten Medium enthält:
- mindestens eine Oxidationsbase, und
- mindestens einen Kuppler, der unten den Verbindungen der Formel (I) nach einem der Ansprüche 1 bis 25 ausgewählt ist.

28. Zusammensetzung nach Anspruch 27, **dadurch gekennzeichnet, dass** die Verbindung(en) der Formel (I) und/oder ihr(e) Additionssalz(e) mit einer Säure 0,0005 bis 12 Gew.-% des Gesamtgewichts der Farbmittelzusammensetzung ausmachen.

29. Zusammensetzung nach Anspruch 27 oder 28, **dadurch gekennzeichnet, dass** die die Oxidationsbase(n) unter den p-Phenylendiaminen, Bisphenylalkylendiaminen, p-Aminophenolen, o-Aminophenolen und den heterocyclischen Basen und ihren Additionssalzen mit einer Säure ausgewählt sind.

30. Zusammensetzung nach einem der Ansprüche 27 bis 29, **dadurch gekennzeichnet, dass** die Oxidationsbase(n) 0,0005 bis 12 Gew.-% des Gesamtgewichts der Farbmittelzusammensetzung ausmachen.

31. Zusammensetzung nach einem der Ansprüche 27 bis 30, **dadurch gekennzeichnet, dass** sie neben der oder den Verbindungen der Formel (I) ferner einen oder mehrere zusätzliche Kuppler, die unter den m-Phenylendiaminen, m-Aminophenolen, m-Dihydroxybenzolen und den heterocyclischen Kupplern ausgewählt sind, und ihre Additionssalze mit einer Säure und/oder einen oder mehrere Direktfarbstoffe enthält.

32. Zusammensetzung nach Anspruch 31, **dadurch gekennzeichnet, daß** der oder die zusätzlichen Kuppler 0,0001 bis 10 Gew.-% des Gesamtgewichts der Farbmittelzusammensetzung ausmachen.

33. Zusammensetzung nach einem der Ansprüche 27 bis 29, **dadurch gekennzeichnet, dass** die Additionssalze mit einer Säure unter den Hydrochloriden, Hydrobromiden, Sulfaten, Citraten, Succinaten, Tartraten, Lactaten und Acetaten ausgewählt sind.

34. Verfahren zum oxidativen Färben von Keratinfasern, **dadurch gekennzeichnet, dass** auf die Fasern mindestens eine Farbmittelzusammensetzung nach einem der Ansprüche 27 bis 33 aufgetragen wird und die Farbe bei einem sauren, neutralen oder alkalischen pH-Wert mit einem Oxidationsmittel entwickelt wird, das kurz vor der Anwendung zu der Farbmittelzusammensetzung gegeben wird oder das in einer oxidierenden Zusammensetzung vorliegt, die gleichzeitig oder getrennt davon anschließend aufgetragen wird.

35. Verfahren nach Anspruch 34, **dadurch gekennzeichnet, daß** das Oxidationsmittel unter Wasserstoffperoxid, Harnstoffperoxid, Alkalimetallbromaten, Salzen von Persäuren und Enzymen ausgewählt ist.

36. Vorrichtung mit mehreren Abteilungen oder "Kit" zum Färben mit mehreren Abteilungen, wobei eine erste Abteilung eine Farbmittelzusammensetzung nach einem der Ansprüche 27 bis 33 und eine zweite Abteilung eine oxidierende Zusammensetzung enthält.

## Claims

1. Compound of the following formula (I), as well as its addition salts with an acid: 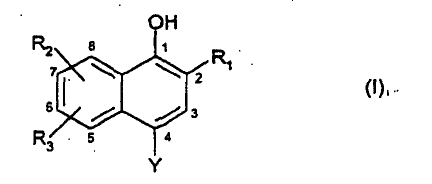 in which:
- R₁ represents a group chosen from -NR₄(C=O)R₅, -NR₆SO₂R₇ and -(C=O)NR₆R₈, the radicals R₄, R₅, R₆, R₇ and R₈ being as defined below;
- R₂ and R₃, which are identical or different, represent a hydrogen atom; a halogen atom; a group Z as defined below; a radical comprising from 1 to 20 carbon atoms, linear or branched (it being possible for the branch(es) to form one or more carbon-containing rings comprising from 3 to 7 members), which may contain one or more double bonds and/or one or more triple bonds (the said double bonds possibly leading to aromatic groups), in which one or more carbon atoms may be replaced with an oxygen, nitrogen or sulphur atom or with an SO₂ group, and in which the carbon atoms may, independently of each other, be substituted with one or more halogen atoms;
it being understood that:
- the said radicals R₂ and R₃ cannot be linked to the benzene ring of formula (I) by an -NH-NH-bond; and that R₂ and R₃ do not contain a peroxide bond or diazo, nitro and nitroso radicals;
- that R₂ and R₃ cannot represent a hydroxyl radical or a thio radical;
- that R₂ and R₃ cannot represent, simultaneously, an amino, alkylamino, acylamino or sulphonylamino radical;
- Y represents a hydrogen or halogen atom; a group -OR₉, -SR₉, or -NH-SO₂R₉ in which R₉ represents a C₁-C₆ alkyl radical, linear or branched (it being possible for the branch(es) to then form one or more rings comprising from 3 to 6 members), unsubstituted or substituted with one or more radicals chosen from the group consisting of a halogen atom, a hydroxyl, C₁-C₄ alkoxy, amino and amino(C₁-C₄ alkyl) radical; a phenyl radical, unsubstituted or substituted with one or two radicals chosen from the group consisting of a C₁-C₄ alkyl, trifluoromethyl, carboxyl, C₁-C₄ alkoxycarbonyl, halogen, hydroxyl, C₁-C₄ alkoxy, amino and amino (C₁-C₄ alkyl) radical; or a benzyl radical;
- R₄, R₆ and R₈, which are identical or different, represent a hydrogen atom; a group Z as defined below; a radical comprising from 1 to 15 carbon atoms, linear or branched (it being possible for the branch(es) to form one or more carbon-containing rings comprising from 3 to 7 members), which may contain one or more double bonds and/or one or more triple bonds (the said double bonds possibly leading to aromatic groups), in which one or more carbon atoms may be replaced with an oxygen, nitrogen or sulphur atom or, with an SO₂ group, and in which the carbon atoms may, independently of each other, be substituted with one or more halogen atoms;
it being understood that the said SO₂ group is not directly linked to the nitrogen atom carrying the radical R₄ or R₆;
it being understood that the group -(C=O)- is not directly linked to the nitrogen atom carrying the radical R₆;
it being understood that the said radicals R₄, R₆ and R₈ do not contain a peroxide bond or diazo, nitro and nitroso radicals;
it being understood that the radicals R₄, R₆ and R₈ cannot represent a hydroxyl radical, a thio radical, an amino radical, an alkoxy radical, an alkylthio radical;
- R₅ and R₇, which are identical or different, represent a hydrogen atom; a group Z as defined below; a radical comprising from 1 to 20 carbon atoms, linear or branched (it being possible for the branch(es) to form one or more carbon-containing rings comprising from 3 to 7 members), which may contain one or more double bonds and/or one or more triple bonds (the said double bonds possibly leading to aromatic groups), in which one or more carbon atoms may be replaced with an oxygen, nitrogen or sulphur atom or with an SO₂ group, and in which the carbon atoms may, independently of each other, be substituted with one or more halogen atoms;
it being understood that the said radicals R₅ and R₇ do not contain peroxide bonds or diazo, nitro and nitroso radicals;
it being understood that R₅ cannot represent a hydroxyl radical or a thio radical;
it being understood that R₇ cannot represent a thio radical;
it being understood that the radicals R₄ and R₅ on the one hand, and the radicals R₆ and R₈ on the other hand, can, in addition, be linked to form, independently of each other, a saturated or unsaturated ring comprising from 5 to 7 members, consisting of carbon, nitrogen and/or acyl, each member being unsubstituted or substituted with 1 or 2 radicals R, which are identical or different, R being a C₁-C₈ alkyl radical, linear or branched (it being possible for the branch(es) to then form one or more rings comprising from 3 to 7 members), which may contain one or more double bonds and/or one or more triple bonds (the said double bonds possibly leading to aromatic groups), and in which one or more carbon atoms may be replaced with an oxygen, nitrogen or sulphur atom or with an SO₂ group, and in which the carbon atoms may, independently of each other, be substituted with one or more halogen atoms; the said ring containing no peroxide bonds or diazo, nitro and nitroso radicals;
- Z is a cationic group represented by the following formula (II): 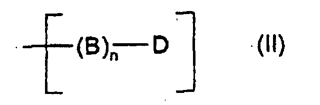
in which:
- n can take the value 0 or 1;
- B represents an alkyl radical comprising from 1 to 15 carbon atoms, linear or branched (it being possible for the branch(es) to then form one or more rings comprising from 3 to 7 members), which may contain one or more double bonds and/or one or more triple bonds, the said double bonds possibly leading to aromatic groups, and in which one or more carbon atoms may be replaced with an oxygen, nitrogen or sulphur atom or with an -SO₂ radical; and in which one or more carbon atoms may, independently of each other, be substituted with one or more halogen atoms or with one or more groups Z; the said radical B containing no peroxide bond or diazo, nitro or nitroso radicals;
- D is chosen from the cationic groups of the following formulae (III) and (IV): 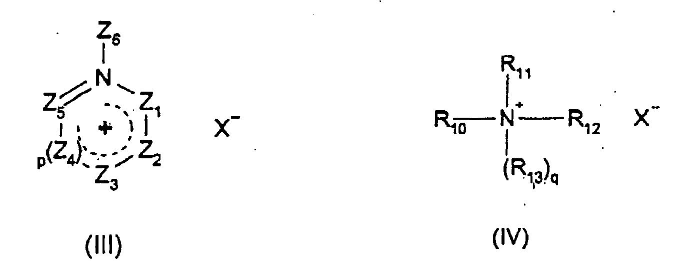
in which:
- p and q can, independently of each other, take the value 0 or 1;
- the radical B is linked to the group D by any one of the atoms of the radical D;
- when n = 0 and q = 0, then the group of formula (IV) can be linked to the compound of formula (I) directly by the nitrogen atom of the quaternary ammonium;
- Z₁, Z₂, Z₃ and Z₄, independently of each other, represent an oxygen atom; a sulphur atom; a nitrogen atom which is unsubstituted or substituted with a radical R₁₄; a carbon atom which is unsubstituted or substituted with one or two radicals R₁₄, which are identical or different;
- Z₅ represents a nitrogen atom or a carbon atom which is unsubstituted or substituted with a radical R₁₄;
- Z₆ can have the same meanings as those indicated below for the radical R₁₄; it being understood that Z₆ is different from a hydrogen atom;
the radicals Z₁ or Z₅ can, in addition, form with Z₆ a saturated or unsaturated ring comprising from 5 to 7 members, each member being unsubstituted or substituted with one or two radicals R₁₄ which are identical or different;
- R₁₄ represents a hydrogen atom; a group Z; a radical comprising from 1 to 10 carbon atoms, linear or branched, which may contain one or more double bonds and/or one or more triple bonds, it being possible for the said double bonds to then possibly lead to aromatic .groups, and in which one or more carbon atoms may be replaced with an oxygen, nitrogen or sulphur atom, or with an SO₂ group, and in which one or more carbon atoms may, independently of each other, be substituted with one or more halogen atoms; the said radical containing no peroxide bond or diazo, nitro and nitroso radicals;
it being possible, in addition, for two of the adjacent radicals Z₁, Z₂, Z₃, Z₄ and Z₅ to form a ring comprising from 5 to 7 members, each member being independently represented by a carbon atom which is unsubstituted or substituted with one or two radicals R₁₄ which are identical or different; a nitrogen atom which is unsubstituted or substituted with a radical R₁₄; an oxygen atom; or a sulphur atom;
- R₁₀, R₁₁, R₁₂ and R₁₃, which are identical or different, have the same meanings as those indicated above for the radical R₁₄;
it being possible for the radicals R₁₀, R₁₁, R₁₂ and R₁₃ to also form, in pairs with the quaternary nitrogen atom to which they are attached, one or more saturated rings comprising from 5 to 7 members, each member being independently represented by a carbon atom which is unsubstituted or substituted with one or two radicals R₁₄ which are identical or different; a nitrogen atom which is unsubstituted or substituted with a radical R₁₄; an oxygen atom; or a sulphur atom;
- X⁻ represents an organic or inorganic anion;
it being understood that at least one of the groups R₁ to R₃ represents or contains a group Z.

2. Compound according to Claim 1, **characterized in that** R₂ and R₃ represent, independently of each other, a hydrogen or chlorine atom; a group Z; a methyl or methoxy radical; an amino, methylamino, 2-hydroxyethylamino radical; a group -NH(CO)R₁₅ in which R₁₅ represents one of the radicals listed in the group **(G1)** consisting of a methyl, ethyl, propyl, isopropyl, butyl, isobutyl, tert-butyl, pentyl, isopentyl, neopentyl, hexyl, cyclopropyl, cyclobutyl, cyclopentyl, cyclopentylmethyl, 3-cyclopentylpropyl, cyclohexyl, 2-cyclohexylethyl, norborn-2-yl, vinyl, 1-methylvinyl, 2-methylvinyl, 2,2-dimethylvinyl, allyl, 3-butenyl, phenyl, methylphenyl, dimethylphenyl, 2,4,6-trimethylphenyl, 4-ethylphenyl, (trifluoromethyl)phenyl, hydroxyphenyl, methoxyphenyl, ethoxyphenyl, acetoxyphenyl, (trifluoromethoxy)phenyl, aminophenyl, 4-dimethylaminophenyl, fluorophenyl, difluorophenyl, fluoro(trifluoromethyl)phenyl, chlorophenyl, dichlorophenyl, bromophenyl, naphth-1-yl, naphth-2-yl, (2-methoxy)naphth-1-yl, benzyl, 4'-methoxybenzyl, 2',5'-dimethoxybenzyl, 3',4'-dimethoxybenzyl, 4'-fluorobenzyl, 4'-chlorobenzyl, phenethyl, 2-phenylvinyl, (1-naphthyl)methyl, (2-naphthyl)methyl; tetrahydrofuran-2-yl, furan-2-yl, 5-methyl-2-(trifluoromethyl)furan-3-yl, 2-methyl-5-phenylfuran-3-yl, thiophen-2-yl, (thiophen-2-yl)methyl, 3-chlorothiophen-2-yl, 2,5-dichlorothiophen-3-yl, benzothiophen-2-yl, 3-chlorobenzothiophen-2-yl, isoxazol-5-yl, 5-methylisoxazol-3-yl, 3,5-dimethylisoxazol-4-yl, 1,3-dimethylpyrazol-5-yl, 1-ethyl-3-methylpyrazol-5-yl, 1-tert-butyl-3-methylpyrazol-5-yl, 3-tert-butyl-1-methylpyrazol-5-yl, 4-bromo-1-ethyl-3-methylpyrazol-5-yl, pyridinyl, chloropyridinyl, dichloropyridinyl, 5-(bromo)pyridin-3-yl, piperazin-2-yl, 2-chloro-4-(trifluoromethyl)pyperimidin-5-yl, quinoxal-2-yl, benzofurazan-5-yl; fluoromethyl, difluoromethyl, trifluoromethyl, 1,1,2,2-tetrafluoroethyl, pentafluoroethyl, heptafluoropropyl, 1,1,2,2,3,3,4,4-octafluorobutyl, nonafluorobutyl, chloromethyl, chloroethyl, 1,1-dimethyl-2-chloroethyl., 1,2-dichloroethyl, 1-chloropropyl, 3-chloropropyl, 4-chlorobutyl, hydroxymethyl, methoxymethyl, phenoxymethyl, (4-chlorophenoxy)methyl, benzyloxymethyl, acetoxymethyl, 1,2-dihydroxyethyl, 1-phenoxyethyl, 1-acetoxyethyl, 2-(2-carboxyethoxy)ethyl, 1-phenoxyethyl, 1-acetoxyethyl, (methoxycarbonyl)methyl, 2-carboxyethyl, 2-(methoxycarbonyl)ethyl, 2-carboxycyclopropyl, 2-carboxycyclohexane, methoxy, ethoxy, propoxy, isopropoxy, butoxy, isobutoxy, pentoxy, neopentoxy, hexyloxy, cyclopentyloxy, cyclohexyloxy, vinyloxy, allyloxy, propargyloxy, chloromethoxy, 1-chloroethoxy, 2-methoxyethoxy, 4-chlorobutoxy, phenoxy, 4-methylphenoxy, 4-fluorophenoxy, 4-bromophenoxy, 4-chlorophenoxy, 4-methoxyphenoxy, naphth-2-yloxy, benzyloxy; amino, methylamino, ethylamino, propylamino, isopropylamino, butylamino, cyclohexylamino, allylamino, 2-chloroethylamino, 3-chloropropylamino, carboxymethylamino, phenylamino, fluorophenylamino, (trifluoromethyl)phenylamino, chlorophenylamino, bromophenylamino, 4-acetylphenylamino, methoxyphenylamino, (trifluoromethoxy)phenylamino, naphth-1-ylamino, benzylamino, phenethylamino, pyrid-3-ylamino, dimethylamino, 1-pyrrolidinyl and 4-morpholinyl radical; or a group -NHSO₂R₁₆ in which R₁₆ represents one of the radicals listed in the group **(G2)** consisting of the methyl, trifluoromethyl, ethyl, 2-chloroethyl, propyl, 3-chloropropyl, isopropyl, butyl, thiophen-2-yl, hydroxyl, ethoxy, amino and dimethylamino radicals.

3. Compound according to Claim 2, **characterized in that** R₂ and R₃ represent, independently of each other, a hydrogen atom; a group -O-E-D', -NH-E-D', -NH(CO)-D', -NH(CO)-E-D', -NH(CO)O-E-D', -NH(CO)NH-E-D' or -NH(SO₂)-E-D', in which -E- represents a -(CH₂)_{q}- arm, q being an integer equal to 1 or 2, and in which D' represents the 3-methylimidazolidinium-1-yl, 3-(2-hydroxyethyl)imidazolidinium-1-yl, 1,2,4-triazolinium-1-yl, 1,2,4-triazolinium-4-yl, N-(C₁-C₄)alkylpyridinium-2-yl, N-(C₁-C₄)alkylpyridinium-3-yl, N-(C₁-C₄)alkylpyridinium-4-yl, N-(2-hydroxyethyl)pyridinium-2-yl, N-(2-hydroxyethyl)pyridinium-3-yl, N-(2-hydroxyethyl)pyridinium-4-yl, pyridinium-1-yl, tri(C₁-C₄ alkyl)ammonium-N-yl, 1-methylpiperidinium-1-yl and 1,4-dimethylpiperazinium-1-yl groups; a methyl, methoxy, amino or methylamino radical; or a group -NH(CO)R₁₇ in which R₁₇ represents one of the radicals listed in the group (G2) as defined in Claim 2; or a methanesulphonylamino, ethanesulphonylamino or dimethylaminosulphonylamino group.

4. Compound according to any one of the preceding claims, **characterized in that** R₄ represents a hydrogen atom or a group A₁ consisting of a linear or branched C₁-C₈ alkyl radical which may carry one or two double bonds or one triple bond, which may be unsubstituted or substituted with a group chosen from a group A₂, a group A₄, a group A₅, as defined below which may be unsubstituted or substituted with one or two groups, which are identical or different, chosen from the N-(C₁-C₃)alkylamino, N-(C₁-C₃)alkyl-N-(C₁-C₃)alkylamino, (C₁-C₆)alkoxy, oxo, alkoxycarbonyl, acyloxy, amide, acylamino, ureyl, sulphoxy, sulphonyl, sulphonamido, sulphonylamino, bromo, cyano and carboxyl groups, and which may be unsubstituted or substituted with one or more hydroxyl, fluoro or chloro groups; a group A₂ consisting of an aromatic group of the phenyl or naphthyl type, which may be unsubstituted or substituted with one to three groups, which are identical or different, chosen from the methyl, trifluoromethyl, ethyl, isopropyl, butyl, pentyl, fluoro, chloro, bromo, methoxy, trifluoromethoxy, ethoxy, propyloxy, acetyloxy, acetyl and cyano groups; a group A₃ consisting of a heteroaromatic group chosen from the furanyl, thiophenyl, pyrrolyl, imidazolyl, thiazolyl, oxazolyl, 1,2,3-triazolyl, 1,2,4-triazolyl, isoxazolyl, isothiazolyl, pyrazolyl, pyrazolotriazolyl, pyrazoloimidazolyl, pyrrolotriazolyl, pyrazolopyrimidyl, pyrazolopyridyl, pyridyl, pyrimidyl, benzoimidazolyl, benzoxazolyl, benzothiazolyl, indolyl, indolidinyl, isoindolyl, indazolyl, benzotriazolyl, quinolinyl, benzoimidazolyl and benzopyrimidyl groups, the said heteroaromatic groups being unsubstituted or substituted with 1 to 3 radicals chosen from a linear or branched C₁-C₄ alkyl radical, a C₁-C₄ monohydroxyalkyl radical, a C₂-C₄ polyhydroxyalkyl radical, a carboxyl radical, an alkoxycarbonyl radical, a halogen atom, an amido radical, an amino radical and a hydroxyl radical; a group A₄ consisting of a C₃-C₇ cycloalkyl radical, a norbornyl radical, carrying or otherwise a double bond and unsubstituted or substituted with 1 or 2 radicals chosen from a linear or branched C₁-C₄ alkyl radical, a C₁-C₄ monohydroxyalkyl radical, a C₂-C₄ polyhydroxyalkyl radical, a carboxyl radical, an alkoxycarbonyl radical, a halogen atom, an amido radical, an amino radical and a hydroxyl radical; or a group A₅ consisting of a heterocycle chosen from the dihydrofuranyl, tetrahydrofuranyl, butyrolactonyl, dihydrothiophenyl, tetrahydrothiophenyl, tetrahydrothiophenonyl, iminothiolane, dihydropyrrolyl, pyrrolidinyl, pyrrolidinonyl, imidazolidinonyl, imidazolidinethionyl, oxazolidinyl, oxazolidinonyl, oxazolanethione, thiazolidinyl, isothiazolonyl, mercaptothiazolinyl, pyrazolidinonyl, iminothiolane, dioxolanyl, pentalactone, dioxanyl, dihydropyridinyl, piperidinyl, pentalactam, morpholinyl, pyrazoli(di)nyl, pyrimi(di)nyl, pyrazinyl, piperazinyl and azepinyl rings; the said groups A₁, A₂, A₃, A₄ and/or A₅ optionally separated from the nitrogen of the group -NR₄(C=O)R₅ by a group -(CO)-.

5. Compound according to Claim 4, **characterized in that** R₄ represents a hydrogen atom; a methyl, ethyl, isopropyl, allyl, phenyl, benzyl, fluorobenzyl, hydroxybenzyl, difluorobenzyl, trifluorobenzyl, chlorobenzyl, bromobenzyl, methoxybenzyl, dimethoxybenzyl, (trifluoromethoxy)benzyl, 3,4-methylenedioxybenzyl, 6-chloropiperonyl, 4-methylthiobenzyl, 4-methylsulphonylbenzyl, 4-acetylaminobenzyl, 4-carboxybenzyl, 1-naphthomethyl or 2-naphthomethyl radical; or a 2-hydroxyethyl, 2-methoxyethyl or 2-ethoxyethyl group.

6. Compound according to any one of the preceding claims, **characterized in that** R₅ denotes a hydrogen atom, an amino group; a group Z; a group A₁, A₂, A₃, A₄ or A₅ as defined in Claim 4, optionally separated from the carbon (at the 2-position) with respect to the amide function of the compound of formula (I) by a group -O-, -NH, -N(C₁-C₃)alkyl- or -(CO)-.

7. Compound according to Claim 6, **characterized in that** R₅ represents a group Z; a radical chosen from the group **(G3)** consisting of a methyl, ethyl, propyl, allyl, phenyl, tetrahydrofuran-2-yl, furan-2-yl, thiophen-2-yl, pyridinyl, piperazin-2-yl, fluoromethyl, chloromethyl, 2-chloroethyl, methoxymethyl, acetoxymethyl, 1,2-dihydroxyethyl, methoxycarbonyl, 2-carboxyethyl, methoxy, ethoxy, propoxy, allyloxy, 2-chloroethoxy, 2-methoxyethoxy, amino, ethylamino, allylamino, 2-chloroethylamino, pyridylamino, dimethylamino, 1-pyrrolidinyl and 4-morpholinyl radical.

8. Compound according to Claim 7, **characterized in that** R₅ represents a group -D', -E-D', -O-E-D' or -NH-E-D' in which -E- represents a -(CH₂)_{q}- arm, q being an integer equal to 1 or 2 and D' a group as defined in Claim 3; or a radical chosen from the group **(G4)** consisting of a methyl, methoxymethyl, 2-carboxyethyl, methoxy, amino, ethylamino and 1-pyrrolidinyl radical.

9. Compound according to any one of the preceding claims, **characterized in that** R₄ and R₅ form a ring, the said ring being chosen from the 2-pyrrolidinon-1-yl, methyl-2-pyrrolidinon-1-yl, 5-carboxy-2-pyrrolidinon-1-yl, 5-methoxycarbonyl-2-pyrrolidinon-1-yl, pyrazolinon-1-yl, succinimid-1-yl, 3,5-diketopyrazolidin-1-yl, oxindolin-1-yl, maleimid-1-yl, isoindole-1,3-dion-2-yl, 2-piperidinon-1-yl and glutarimid-1-yl groups.

10. Compound according to any one of the preceding claims, **characterized in that** R₆ represents a hydrogen atom or a group A₁, A₂, A₃, A₄ or A₅ as defined in Claim 4.

11. Compound according to Claim 10, **characterized in that** R₆ represents a hydrogen atom; a methyl, ethyl, isopropyl, allyl; phenyl, benzyl, fluorobenzyl, hydroxybenzyl, difluorobenzyl, trifluorobenzyl, chlorobenzyl, bromobenzyl, methoxybenzyl, dimethoxybenzyl, (trifluoromethoxy)benzyl, 3,4-methylenedioxybenzyl, 6-chloropiperonyl, 4-methylthiobenzyl; 4-methylsulphonylbenzyl, 4-acetylaminobenzyl, 4-carboxybenzyl, 1-naphthomethyl or 2-naphthomethyl radical; a 2-hydroxyethyl, 2-methoxyethyl or 2-ethoxyethyl group.

12. Compound according to any one of the preceding claims, **characterized in that** R₈ denotes a group Z; a. hydrogen atom or a group A₁, A₂, A₃, A₄ or A₅ as defined in Claim 4.

13. Compound according to Claim 12, **characterized in that** R₈ represents a group Z; a hydrogen atom; a methyl, ethyl, isopropyl, allyl; phenyl, benzyl, fluorobenzyl, hydroxybenzyl, difluorobenzyl, trifluorobenzyl, chlorobenzyl, bromobenzyl, methoxybenzyl, dimethoxybenzyl, (trifluoromethoxy)benzyl, 3,4-methylenedioxybenzyl, 6-chloropiperonyl, 4-methylthiobenzyl, 4-methylsulphonylbenzyl, 4-acetylaminobenzyl, 4-carboxybenzyl, 1-naphthomethyl or 2-naphthomethyl radical; a 2-hydroxyethyl, 2-methoxyethyl or 2-ethoxyethyl group.

14. Compound according to Claim 13, **characterized in that** R₈ represents a hydrogen atom or a methyl radical; a group -D', -E-D', in which -E- represents a -(CH₂)_{q}- arm, q being an integer equal to 1 or 2, and D' a group as defined in Claim 3.

15. Compound according to any one of the preceding claims, **characterized in that** R₆ and R₈ form a ring chosen from the pyrrolidinyl, piperidinyl, morpholinyl, pyrazolyl, isoxazolyl, imidazolyl, thiazolyl, indolyl, indolidinyl, indazolyl, pyrazolotriazolyl, pyrrolotriazolyl, pyrazoloimidazolyl, pyrazolidinyl, thiomorpholinyl, thiazolyl and pyrazinyl groups.

16. Compound according to any one of the preceding claims, **characterized in that** R₇ denotes an amino group; a group Z; a group A₁, A₂, A₃, A₄ or A₅ as defined in Claim 4, optionally separated from the sulphur of the sulphonyl group of the compound of formula (I) by a group -NH or N(C₁-C₃)alkyl-.

17. Compound according to Claim 16, **characterized in that** R₇ represents a group Z, one of the radicals listed in the group **(G4)** consisting of the methyl, trifluoromethyl, ethyl, 2-chloroethyl, propyl, 3-chloropropyl; isopropyl; butyl, thiophen-2-yl, hydroxyl, ethoxy, amino and dimethylamino radicals.

18. Compound according to Claim 17, **characterized in that** R₇ represents a group -D', -E-D' or -NH-E-D', in which -E- represents a -(CH₂)_{q}- arm, q being an integer equal to 1 or 2, and D' a group as defined in Claim 3; or a methyl, ethyl or dimethylamino radical.

19. Compound according to any one of the preceding claims, **characterized in that** Y preferably represents a radical chosen from the group comprising: a hydrogen, chlorine, fluorine or bromine atom; the methoxy, ethoxy, propoxy, benzyloxy and phenoxy groups; -OCH₂CH₂OMe; -OCH₂CH₂OMe; -OCH₂CH₂NMe₂; -OCH₂(CO)OH, -OCH₂(CO)OMe, -OCH₂(CO)OEt, -SCH₂CH₂CO₂H and NHSO₂Me groups.

20. Compound according to Claim 19, **characterized in that** Y represents a radical chosen from the group consisting of a hydrogen or chlorine atom or the methoxy, -OCH₂(CO)OH or -OCH₂(CO)OMe group.

21. Compound according to any one of the preceding claims, **characterized in that** D is chosen from the imidazolinium, thiazolinium, oxazolinium, pyrrolinium, 1,2,3-triazolinium, 1,2,4-triazolinium, isoxazolinium, isothiazolinium, imidazolidinium, thiazolidinium, pyrazolinium, pyrazolidinium, oxazolidinium, pyrazolotriazolinium, pyrazoloimidazolinium, pyrrolotriazolinium, pyrazolopyrimidinium, pyrazolopyridinium, pyridinium, pyrimidinium, pyrazinium, triazinium, benzoimidazolinium, benzoxazolinium, benzothiazolinium, indolinium, indolidinium, isoindolinium, indazolinium, benzotriazolinium, quinolinium, tetrahydroquinolinium, benzoimidazolidinium, benzopyrimidinium, tetra(C₁-C₄)alkylammonium, dialkylpiperidinium, dialkylpyrrolidinium, dialkylmorpholinium, dialkylthiomorpholinium, dialkylpiperazinium, azepinium and 1,4-diazabicyclo[2.2.2]octanium groups.

22. Compound according to Claim 21, **characterized in that** D is chosen from the 3-methylimidazolidinium-1-yl, 3-(2-hydroxyethyl)imidazolidinium-1-yl, 1,2,4-triazolinium-1-yl, 1,2,4-triazolinium-4-yl, N-(C₁-C₄)alkylpyridinium-2-yl, N-(C₁-C₄)alkylpyridinium-3-yl, N-(C₁-C₄)alkylpyridinium-4-yl, N-(2-hydroxyethyl)pyridinium-2-yl, N-(2-hydroxyethyl)pyridinium-3-yl, N-(2-hydroxyethyl)-pyridinium-4-yl, pyridinium-1-yl, tri(C₁-C₄)alkylammonium-N-yl, 1-methylpiperidinium-1-yl and 1,4-dimethylpiperazinium-1-yl groups.

23. Compound according to any one of the preceding claims, **characterized in that** it is chosen from those in which:
i) - the radical R₁ represents a radical chosen from the group **(G5)** consisting of an acetylamino, methanesulphonylamino, amido or methylamido radical, a group -NH-C(O)-CH₂-D1, a group -NH-SO₂-CH₂-CH₂-D1, a group -(CO)-NH-CH₂-CH₂-D1 in which D1 represents a 3-methylimidazblidinium-1-yl, triazolinium-1-yl, pyridinium-1-yl or trimethylammonium-N-yl group;
- the radical R₂ is at the 5-position and represents a radical chosen from the group **(G6)** consisting of an amino, methylamino, acetylamino or methanesulphonylamino radical, a group -NH-[C(O)]ₘ-CH₂-D1 in which m represents 0 or 1, or a group -NH-SO₂-CH₂-CH₂-D1,
- the radical R₃ represents a hydrogen atom;
- the radical Y represents a radical chosen from the group **(G7)** consisting of a hydrogen atom, a chlorine radical and a methoxy radical; or
ii) - the radical R₁ represents a radical chosen from the group **(G5)** as defined above;
- the radical R₂ is situated at the 8-position and represents a radical chosen from the group **(G6)** as defined above;
- the radical R₃ represents a hydrogen atom;
- the radical Y represents a radical chosen from the group **(G7)** as defined above;
or
iii) - the radical R₁ represents a radical chosen from the group **(G5)** as defined above;
- the radicals R₂ and R₃ represent a hydrogen atom;
- the radical Y represents a radical chosen from the group **(G7)** as defined above.

24. Compound according to any one of the preceding claims, **characterized in that** they are chosen from:
- 3-[(1-Hydroxynaphthalen-2-ylcarbamoyl)methyl]-1-methyl-3H-imidazol-1-ium chloride;
- 3-[(1-Hydroxy-5-(2-(3-methyl-lH-imidazol-3-ium-1-yl)acetyl)aminonaphthalen-2-ylcarbamoyl)methyl]-1-methyl-3H-imidazol-1-ium dichloride;
- 3-[(1-Hydroxy-8- (2-(3-methyl-lH-imidazol-3-ium-1-yl)acetyl)aminonaphthalen-2-ylcarbamoyl)methyl]-1-methyl-3H-imidazol-1-ium dichloride;
- 3-[(1-Hydroxy-5-aminonaphthalen-2-ylcarbamoyl)-methyl]-1-methyl-3H-imidazol-1-ium chloride;
- 3-[(1-Hydroxy-5-acetylaminonaphthalen-2-ylcarbamoyl)methyl]-1-methyl-3H-imidazol-1-ium chloride;
- 3-[(1-Hydroxy-5-methoxycarbonylaminonaphthalen-2-ylcarbamoyl)methyl]-1-methyl-3H-imidazol-1-ium chloride;
- 3-[(1-Hydroxy-2-acetylaminonaphthalen-5-ylcarbamoyl)methyl]-1-methyl-3H-imidazol-1-ium chloride;
- 3-[(1-Hydroxy-2-methoxycarbonylaminonaphthalen-5-ylcarbamoyl)methyl]-1-methyl-3H-imidazol-1-ium chloride;
- 3-[(1-Hydroxy-5-methanesulphonylaminonaphthalen-2-ylcarbamoyl)methyl]-1-methyl-3H-imidazol-1-ium chloride;
- 3-[(1-Hydroxy-8-aminonaphthalen-2-ylcarbamoyl)-methyl]-1-methyl-3H-imidazol-1-ium chloride;
- 3-[(1-Hydroxy-8-acetylaminonaphthalen-2-ylcarbamoyl)methyl]-1-methyl-3H-imidazol-1-ium chloride;
- 3-[(1-Hydroxy-8-methoxycarbonylaminonaphthalen-2-ylcarbamoyl)methyl]-1-methyl-3H-imidazol-1-ium chloride;
- 3-[(1-Hydroxy-2-acetylaminonaphthalen-8-ylcarbamoyl)methyl]-1-methyl-3H-imidazol-1-ium chloride;
- 3-[(1-Hydroxy-2-methoxycarbonylaminonaphthalen-8-ylcarbamoyl)methyl]-1-methyl-3H-imidazol-1-ium chloride;
- 3-[(1-Hydroxy-8-methanesulphonylaminonaphthalen-2-ylcarbamoyl)methyl]-1-methyl-3H-imidazol-1-ium chloride;
- 1-[(1-Hydroxynaphthalen-2-ylcarbamoyl)methyl]-pyridinium chloride;
- 1-[(1-Hydroxy-5-(2-(pyridinium-1-yl)acetyl)-aminonaphthalen-2-ylcarbamoyl)methyl]pyridinium dichloride;
- 1-[(1-Hydroxy-8-(2-(pyridinium-1-yl)acetyl)-aminonaphthalen-2-ylcarbamoyl)methyl]pyridinium dichloride;
- 1-[(1-Hydroxy-5-aminonaphthalen-2-ylcarbamoyl)-methyl]pyridinium chloride;
- 1-[(1-Hydroxy-5-acetylaminonaphthalen-2-ylcarbamoyl)methyl]pyridinium chloride;
- 1-[(1-Hydroxy-5-methoxycarbonylaminonaphthalen-2-ylcarbamoyl)methyl]pyridinium chloride;
- 1-[(1-Hydroxy-2-acetylaminonaphthalen-5-ylcarbamoyl)methyl]pyridinium chloride;
- 1-[(1-Hydroxy-2-methoxycarbonylaminonaphthalen-5-ylcarbamoyl)methyl]pyridinium chloride;
- 1-[(1-Hydroxy-5-methanesulphonylaminonaphthalen-2-ylcarbamoyl)methyl]pyridinium chloride;
- 1-[(1-Hydroxy-8-aminonaphthalen-2-ylcarbamoyl)-methyl]pyridinium chloride;
- 1-[(1-Hydroxy-8-acetylaminonaphthalen-2-ylcarbamoyl)methyl]pyridinium chloride;
- 1-[(1-Hydroxy-8-methoxycarbonylaminonaphthalen-2-ylcarbamoyl)methyl]pyridinium chloride;
- 1-[(1-Hydroxy-2-acetylaminonaphthalen-8-ylcarbamoyl)methyl]pyridinium chloride;
- 1-[(1-Hydroxy-2-methoxycarbonylaminonaphthalen-8-ylcarbamoyl)methyl]pyridinium chloride;
- 1-[(1-Hydroxy-8-methanesulphonylaminonaphthalen-2-ylcarbamoyl)methyl]pyridinium chloride;
- 1-[(1-Hydroxynaphthalen-2-ylcarbamoyl)methyl]-1,4-dimethylpiperazin-1-ium chloride;
- 1-[(1-Hydroxy-5-(2-(1,4-dimethylpiperazin-1-ium-1-yl)acetyl)aminonaphthalen-2-ylcarbamoyl)methyl]-1,4-dimethylpiperazin-1-ium dichloride;
- 1-[(1-Hydroxy-8-(2-(1,4-dimethylpiperazin-1-ium-1-yl)acetyl)aminonaphthalen-2-ylcarbamoyl)methyl]-1,4-dimethylpiperazin-1-ium dichloride;
- 1-[(1-Hydroxy-5-aminonaphthalen-2-ylcarbamoyl)-methyl]-1,4-dimethylpiperazin-1-ium chloride;
- 1-[(1-Hydroxy-5-acetylaminonaphthalen-2-ylcarbamoyl)methyl]-1,4-dimethylpiperazin-1-ium chloride;
- 1-[(1-Hydroxy-5-methoxycarbonylaminonaphthalen-2-ylcarbamoyl)methyl]-1,4-dimethylpiperazin-1-ium chloride;
- 1-[(1-Hydroxy-2-acetylaminonaphthalen-5-ylcarbamoyl)methyl]-1,4-dimethylpiperazin-1-ium chloride;
- 1-[(1-Hydroxy-2-methoxycarbonylaminonaphthalen-5-ylcarbamoyl)methyl]-1,4-dimethylpiperazin-1-ium chloride;
- 1-[(1-Hydroxy-5-methanesulphonylaminonaphthalen-2-ylcarbamoyl)methyl]-1,4-dimethylpiperazin-1-ium chloride;
- 1-[(1-Hydroxy-8-aminonaphthalen-2-ylcarbamoyl)-methyl]-1,4-dimethylpiperazin-1-ium chloride;
- 1-[(1-Hydroxy-8-acetylaminonaphthalen-2-ylcarbamoyl)methyl]-1,4-dimethylpiperazin-1-ium chloride;
- 1-[(1-Hydroxy-8-methoxycarbonylaminonaphthalen-2-ylcarbamoyl)methyl]-1,4-dimethylpiperazin-1-ium chloride;
- 1-[(1-Hydroxy-2-acetylaminonaphthalen-8-ylcarbamoyl)methyl]-1,4-dimethylpiperazin-1-ium chloride;
- 1-[(1-Hydroxy-2-methoxycarbonylaminonaphthalen-8-ylcarbamoyl)methyl]-1,4-dimethylpiperazin-1-ium chloride;
- 1-[(1-Hydroxy-8-methanesulphonylaminonaphthalen-2-ylcarbamoyl)methyl]-1,4-dimethylpiperazin-1-ium chloride;
and their addition salts with an acid.

25. Compound according to any one of the preceding claims, **characterized in that** the addition salts with an acid are chosen from hydrochlorides, hydrobromides, sulphates, citrates, succinates, tartrates, lactates and acetates.

26. Use of the compounds of formula (I) as defined in any one of Claims 1 to 24, as coupler for the oxidation dyeing of keratinous fibres.

27. Composition for the oxidation dyeing of keratinous fibres, **characterized in that** it contains, in a medium appropriate for dyeing:
- at least one oxidation base, and
- at least one coupler chosen from the compounds of formula (I) as defined in any one of Claims 1 to 25.

28. Composition according to Claim 27, **characterized in that** the compound(s) of formula (I) and/or the or their addition salt(s) with an acid represent from 0.0005 to 12% by weight of the total weight of the dyeing composition.

29. Composition according to Claim 27 or 28, **characterized in that** the oxidation base(s) are chosen from para-phenylenediamines, bisphenylalkylenediamines, para-aminophenols, ortho-aminophenols and heterocyclic bases, and their addition salts with an acid.

30. Composition according to any one of Claims 27 to 29, **characterized in that** the oxidation base(s) represent from 0.0005 to 12% by weight of the total weight of the dyeing composition.

31. Composition according to any one of Claims 27 to 30, **characterized in that** it contains, in addition to the compound(s) of formula (I) above, one or more additional couplers chosen from metaphenylenediamines, meta-aminophenols, metadiphenols and heterocyclic couplers, and their addition salts with an acid, and/or one or more direct dyes.

32. Composition according to Claim 31, **characterized in that** the additional coupler(s) represent from 0.0001 to 10% by weight of the total weight of the dyeing composition.

33. Composition according to any one of Claims 27 to 32, **characterized in that** the addition salts with an acid are chosen from hydrochlorides, hydrobromides, sulphates, citrates, succinates, tartrates, lactates and acetates.

34. Method of oxidation dyeing of keratinous fibres, **characterized in that** at least one dyeing composition as defined in any one of Claims 27 to 33 is applied to these fibres, and **in that** the colour is developed at acidic, neutral or alkaline pH with the aid of an oxidizing agent which is added just at the time of use to the dyeing composition and which is present in an oxidizing composition applied simultaneously or sequentially in a separate manner.

35. Method according to Claim 34, **characterized in that** the oxidizing agent is chosen from hydrogen peroxide, urea peroxide, alkali metal bromates, persalts and enzymes.

36. Multicompartment device, or multicompartment dyeing "kit", in which a first compartment contains a dyeing composition as defined in any one of Claims 27 to 33 and a second compartment contains an oxidizing composition.
